(19) Europäisches Patentamt
European Patent Office
Office européen des brevets

(11) EP 1 747 774 B1

(12) FASCICULE DE BREVET EUROPEEN

(45) Date de publication et mention de la délivrance du brevet:
**05.05.2010 Bulletin 2010/18**

(21) Numéro de dépôt: **06117400.9**

(22) Date de dépôt: **18.07.2006**

(51) Int Cl.:
***A61K 8/49*** *(2006.01)* ***A61Q 5/10*** *(2006.01)*
***A61Q 5/06*** *(2006.01)* ***C07D 498/04*** *(2006.01)*
***C07D 263/04*** *(2006.01)* ***C07D 209/14*** *(2006.01)*

# (54) Utilisation pour la coloration des fibres kératiniques d'une composition comprenant un colorant de type styrylique ou iminique

Verwendung einer Zusammensetzung enthaltend ein Farbstoff vom Styryl- oder Imintyp zur Färbung keratinischer Fasern

Use of a composition comprising a colouring agent of the styrylic or imino type for dyeing keratinuous fibres

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **22.07.2005 FR 0552277**

(43) Date de publication de la demande:
**31.01.2007 Bulletin 2007/05**

(73) Titulaire: **L'Oréal**
**75008 Paris (FR)**

(72) Inventeur: **Plos, Grégory**
**Tokyo**
**158-0097 (JP)**

(74) Mandataire: **Prevel, Estelle Nicole**
**L'Oréal**
**D.I.P.I.**
**25-29, Quai Aulagnier**
**92665 Asnières-sur-Seine Cedex (FR)**

(56) Documents cités:
**US-A- 5 474 578**

- **PATENT ABSTRACTS OF JAPAN vol. 2000, no. 20, 10 juillet 2001 (2001-07-10) -& JP 2001 081342 A (FUJI PHOTO FILM CO LTD), 27 mars 2001 (2001-03-27)**
- **PATENT ABSTRACTS OF JAPAN vol. 004, no. 173 (C-032), 29 novembre 1980 (1980-11-29) -& JP 55 113710 A (KANEBO LTD), 2 septembre 1980 (1980-09-02)**
- **PATENT ABSTRACTS OF JAPAN vol. 004, no. 065 (C-010), 16 mai 1980 (1980-05-16) -& JP 55 031057 A (KANEBO LTD; others: 01), 5 mars 1980 (1980-03-05)**
- **PATENT ABSTRACTS OF JAPAN vol. 006, no. 078 (C-102), 15 mai 1982 (1982-05-15) -& JP 57 014652 A (NIPPON KANKO SHIKISO KENKYUSHO:KK), 25 janvier 1982 (1982-01-25)**
- **PATENT ABSTRACTS OF JAPAN vol. 005, no. 075 (C-055), 19 mai 1981 (1981-05-19) -& JP 56 025106 A (KANEBO KESHOHIN KK), 10 mars 1981 (1981-03-10)**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 747 774 B1

Printed by Jouve, 75001 PARIS (FR)

## Description

La présente invention a pour objet l'utilisation pour la coloration des fibres kératiniques, et en particulier des fibres kératiniques humaines telles que les cheveux, d'une composition comprenant au moins un colorant de type styrylique ou iminique.

Il est connu de teindre les fibres kératiniques, et en particulier les fibres kératiniques humaines telles que les cheveux, avec des compositions tinctoriales contenant des précurseurs de colorants d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques, et en particulier les fibres kératiniques humaines telles que les cheveux, avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, azoïque cationique, xanthénique, acridinique azinique, triarylméthane, benzénique nitré ou des colorants naturels.

L'utilisation des colorants directs est très répandue car ils présentent certains avantages par rapport aux précurseurs de colorants d'oxydation et, notamment, une diminution des risques potentiels d'allergie, l'absence de sensibilisation du cheveu due au processus oxydatif et des temps de pose moins longs. Les colorations obtenues sont de plus chromatiques.

Les colorations obtenues sont cependant temporaires ou semi-permanentes, car la nature des interactions qui lient les colorants directs à la fibre kératinique, et leur désorption de la surface et / ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Par ailleurs, les compositions de coloration directe sont colorées et en coloration d'oxydation, la coloration se développe aussi bien dans la composition que sur les cheveux. Par conséquent, la coloration directe et la coloration d'oxydation présentent l'inconvénient de rendre l'application salissante.

Des composés de type styrylique ou iminique existant sous une forme colorée et sous une forme incolore et leur utilisation dans le domaine de la cosmétique, et en particulier dans le domaine du soin de la peau et des vernis à ongles, ont été décrits dans les documents JP 56-150006, JP 56-081522, JP 56-025106, JP 55-113710 et JP 55-031057.

Le but de la présente invention est de fournir de nouvelles compositions pour la coloration des fibres kératiniques qui ne présentent pas les inconvénients de celles de l'art antérieur. En particulier, le but de la présente invention est de fournir de nouveaux colorants pour la coloration des fibres kératiniques permettant d'obtenir rapidement des colorations chromatiques et tenaces, notamment vis-à-vis des shampooings, et qui ne tachent pas.

Ce but est atteint avec la présente invention qui a pour objet l'utilisation pour la coloration des fibres kératiniques d'une composition tinctoriale comprenant, dans un milieu approprié pour la teinture, au moins un composé choisi parmi les composés de formule (I), les composés de formule (II) et leurs sels d'addition :

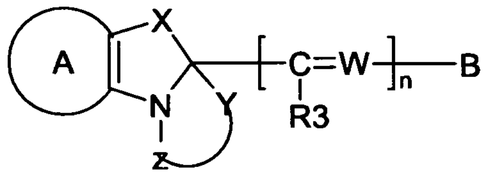

(I)

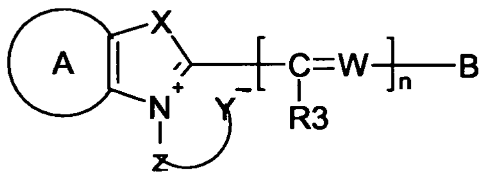

(II)

dans lesquelles :

- A est un noyau aromatique ou hétéroaromatique, condensé ou non, comportant de 5 à 16 chaînons, non substitué ou substitué ;
- X représente un atome d'oxygène, un atome de soufre ou un groupement $CR_1R_2$ ;
- $R_1$ et $R_2$ représentent, indépendamment l'un de l'autre, un atome d'hydrogène, un radical alkyle en $C_1$-$C_6$, un radical hydroxyalkyle en Ci-$C_6$, un radical alcoxyalkyle en $C_1$-$C_6$, une chaîne alkylène pouvant contenir un atome d'oxygène ou de soufre ; $R_1$ et $R_2$ peuvent former ensemble un cycle à 5 ou 6 chaînons aromatiques ou non aromatiques, contenant éventuellement un ou plusieurs hétéroatomes tels qu'un atome d'azote, d'oxygène ou de soufre ;
- $R_3$ représente un atome d'hydrogène, un atome d'halogène, un radical alkyle en $C_1$-$C_6$, un radical cyano, un groupement aromatique, un groupement phénoxy, un radical nitro ;
- W représente un groupement $CR_4$ ou un atome d'azote ;
- $R_4$ représente un atome d'hydrogène, un atome d'halogène, un radical alkyle en $C_1$-$C_6$, un radical cyano, un groupement aromatique, un radical phénoxy, un radical nitro ;
- Y représente un atome d'oxygène, un atome de soufre, un groupement $NR_5$ ;
- $R_5$ représente un atome d'hydrogène, un radical alkyle en $C_1$-$C_6$ ;
- Z représente un groupement $—CpH_{2p}$-, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement $—C_qH_{2q}CO$-, avec q un entier compris entre 1 et 3, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ;
- n représente un entier de 1 à 4 ;
- B représente un noyau aromatique ou hétéroaromatique, condensé ou non, comportant de 5 à 16 chaînons non substitué ou substitué.

La présente invention a également pour objet un procédé de coloration des fibres kératiniques mettant en oeuvre la composition tinctoriale utile dans le cadre de l'invention.

La présente invention a aussi pour objet un dispositif à plusieurs compartiments pour la mise en oeuvre du procédé de coloration conforme à l'invention.

La présente invention a également pour objet de nouveaux composés choisis parmi les composés de formule (I), les composés de formule (II) et leurs sels d'addition et comportant au moins un groupement cationique du type ammonium quaternaire, ainsi qu'une composition tinctoriale comprenant au moins un tel composé.

La présente invention permet en particulier d'obtenir rapidement une coloration des fibres kératiniques qui est chromatique et tenace, notamment vis-à-vis des shampooings.

Un autre avantage de l'invention est de fournir un produit de coloration propre, c'est-à-dire qui ne tache pas : le produit de coloration appliqué sur les fibres kératiniques est incolore, la coloration se révèle dans les fibres kératiniques et les eaux de rinçage sont claires. Il en résulte que l'application de ce produit de coloration n'est pas salissante.

Les composés à cycle ouvert de formule (II) sont par exemple obtenus par l'ouverture de l'hétérocycle formé par N, Y et Z dans les composés de formule (I) sous l'effet d'un stimulus tel que la lumière, un courant électrique, la chaleur, l'ajout d'un agent acidifiant et / ou un agent alcalinisant, l'ajout de solvant ou une radiation électromagnétique. Les composés de formule (I) sont incolores ou faiblement colorés et les composés de formule (II) correspondant dont

l'hétérocycle est ouvert sont des espèces colorées et colorantes. Dans le milieu approprié pour la teinture, il s'établit un équilibre entre les espèces colorées et les espèces non colorées qui dépend de divers facteurs tels que le solvant, les agents alcalinisants et / ou les agents acidifiants présents dans le milieu et la température et qui peut être représenté par le schéma suivant :

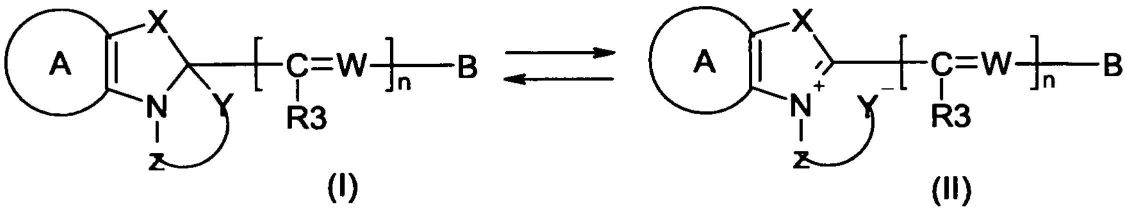

(I) (II)

Lorsque les composés utiles dans le cadre de la présente invention sont appliqués sur les fibres kératiniques sous leur forme incolore, la coloration est révélée sur les fibres kératiniques.

Dans le cadre de la présente invention, on entend par noyau hétéroaromatique un noyau aromatique comprenant un ou plusieurs hétéroatomes tels que les atomes d'azote, de soufre, d'oxygène ou de phosphore.

Dans le cadre de la présente invention, le terme "condensé" signifie au moins deux cycles accolés présentant au moins deux atomes communs.

Un radical halogéno désigne un atome d'halogène choisi parmi le chlore, le brome, l'iode et le fluor.

On entend par radical alkyle (alk) un radical linéaire ou ramifié, par exemple un radical méthyle, éthyle, n-propyle, iso-propyle, n-butyle ou ter-butyle. Un radical alcoxy est un radical alk-O-, un radical mono ou dialkylamino est un radical —N(alk)$_n$ avec n = 1 ou 2, un radical alkylcarbonyle est un radical alk-CO-, un radical alcoxycarbonyle est un radical alk-O-CO-, un radical alkylcarbonylalkyle est un radical alk-CO-alk-, dans chacune de ces définitions le radical alkyle étant tel que défini précédemment.

Un radical alkyle substitué est un alkyle mono ou polysubstitué. Par exemple, un hydroxyalkyle ou un aminoalkyle est un alkyle qui peut être substitué par un ou plusieurs groupes hydroxy ou amino.

On entend par radical aryle (ar) un radical carboné dérivé des composés benzéniques condensés ou non condensés, par exemple phényle, anthracényle ou naphtyle.

A titre d'exemples de cycles à 5 ou 6 chaînons aromatiques ou non aromatiques, on peut citer le cyclo-1,3-pentadiène, le benzène, le cyclopentane et le cyclobutane.

Les composés utiles dans le cadre de l'invention peuvent être neutralisées par un contre-ion négatif ou positif quand elles sont chargées. Les contre-ions négatifs peuvent par exemple être choisis parmi un halogénure tel qu'un chlorure, un bromure, un iodure ou un fluorure, un perchlorate, un p-méthylbenzène sulfonate, un tétrafluoroborate, un sulfate, un alkylsulfate, un toluènesulfonate, un sulfonate. Les contre-ions positifs peuvent être choisis parmi les sels de métaux alcalins ou alcalino-terreux tels que les ions sodium ou potassium.

Selon un mode de réalisation particulier de l'invention, A est un noyau benzénique, anthracénique, naphtalénique ou quinolinique.

Selon un mode de réalisation particulier de l'invention, A est non substitué ou substitué par un ou plusieurs groupements pouvant être choisis parmi un radical halogéno, un radical alkyle en $C_1$-$C_6$, un radical alcoxy en $C_1$-$C_6$, un radical alkylsulfonyle en $C_1$-$C_6$ (-$SO_2$-alkyl), un radical alkylsulfonate en $C_1$-$C_6$ (-$SO_3$-alkyl) un radical cyano, un radical trifluorométhyle, un radical alkylcarbonyle en $C_1$-$C_6$, un radical trifluorométhylsulfonyle (-$SO_2$-$CF_3$), un radical trifluorométhylcarbonyle, un radical phénylsulfonyle (-$SO_2$-Ph), un radical phénylsulfonate (-$SO_3$-Ph), un radical phénylcarbonyle, un radical nitro, un radical alcoxycarbonyle en $C_1$-$C_6$, un radical phosphonyle (-PO(OH)$_2$), un radical phosphonylalkyle($C_1$-$C_6$) (-alkyl-PO(OH)$_2$), un radical hydroxyle, un radical amino, un radical dialkyl($C_1$-$C_6$)amino, un radical (hydroxyalkyl($C_1$-$C_6$))amino, un radical di(hydroxyalkyl($C_1$-$C_6$))amino, un radical (aminoalkyl($C_1$-$C_6$))amino, un radical di(aminoalkyl($C_1$-$C_6$))amino, un radical (hydroxyalkyl($C_1$-C-$_6$))(alkyl($C_1$-$C_6$))amino, un radical (aminoalkyl ($C_1$-$C_6$))(alkyl($C_1$-$C_6$))amino, un radical (aminoalkyl($C_1$-$C_6$))(hydroxyalkyl($C_1$-$C_6$)amino, un radical hydroxyalkyle ($C_1$-$C_6$), un radical aminoalkyle($C_1$-$C_6$), un radical (alkyl($C_1$-$C_6$))aminoalkyle($C_1$-$C_6$), un radical di(alkyl($C_1$-$C_6$))aminoalkyle($C_1$-$C_6$), un radical (hydroxyalkyl($C_1$-$C_6$))aminoalkyle($C_1$-$C_6$), un radical di(hydroxyalkyl($C_1$-$C_6$))aminoalkyle($C_1$-$C_6$), un radical (aminoalkyl($C_1$-$C_6$))aminoalkyle($C_1$-$C_6$), un radical di(aminoalkyl($C_1$-$C_6$))aminoalkyle($C_1$-$C_6$), un radical (alkyl ($C_1$-$C_6$))(hydoxyalkyl($C_1$-$C_6$))aminoalkyle($C_1$-$C_6$), un radical (aminoalkyl($C_1$-$C_6$))(alkyl($C_1$-$C_6$))amino, un radical (hydroxyalkyl($C_1$-$C_6$))(alkyl($C_1$-$C_6$))aminoalkyle($C_1$-$C_6$), un radical phénylalkyle($C_1$-$C_6$) éventuellement substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un groupement cationique du type ammonium quaternaire, un radical alkyle en $C_1$-$C_6$ substitué par un groupement cationique du type ammonium quaternaire, un radical carboxyle, un radical alkyle($C_1$-$C_6$) substitué par un radical carboxyle, un radical thio, un radical thioalkyle($C_1$-$C_6$), un radical

sulfonate ($-SO_3^-$), un radical alkyle($C_1$-$C_6$) substitué par un radical sulfonate, un radical alkyl($C_1$-$C_6$)carbonylalkyle ($C_1$-$C_6$), un radical di(halogénoalkyl($C_1$-$C_6$))amino, un radical acétamido, un radical aryloxy, un radical aryloxyalkyle ($C_1$-$C_6$), un radical éthényle ($-CH=CH_2$), un radical éthénylcarbonyle ($-CO-CH=CH_2$); deux groupements adjacents pouvant former un cycle aromatique ou hétéroaromatique, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, ou un cycle de type $-O-C_mH_{2m}-O-$ où m est un entier égal à 1 ou 2. De préférence, A est non substitué ou substitué par un ou plusieurs groupements choisis parmi un radical alkyl($C_1$-$C_6$)sulfonyle ; un groupement pyridinium ou imidazolium, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement trialkyl($C_1$-$C_6$)ammonium ; un radical sulfonate. A titre d'exemple, A peut être substitué par un radical méthylsulfonyle ; un groupement 1-méthyl pyridin-2-ium ; un groupement imidazolium ; un groupement triméthylammonium ; un radical sulfonate.

Selon un mode de réalisation particulier de l'invention, X est choisi parmi un groupement $CR_1R_2$.

Selon un mode de réalisation particulier de l'invention, $R_1$ et $R_2$ sont choisis parmi un radical alkyle en $C_1$-$C_6$. A titre d'exemple, $R_1$ et $R_2$ peuvent être un radical méthyle ; un radical éthyle.

Selon un mode de réalisation particulier de l'invention, $R_3$ est choisi parmi un atome d'hydrogène.

Selon un mode de réalisation particulier de l'invention, W est choisi parmi un groupement $CR_4$.

Selon un mode de réalisation particulier de l'invention, $R_4$ est choisi parmi un atome d'hydrogène.

Selon un mode de réalisation particulier de l'invention, Y est choisi parmi un atome d'oxygène.

Selon un mode de réalisation particulier de l'invention, Z est choisi parmi un groupement $—C_pH_{2p}-$, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle. A titre d'exemple, Z peut être un groupement $-C_2H_4-$.

Selon un mode de réalisation particulier de l'invention, n est égal à 1 ou 2.

Selon un mode de réalisation particulier de l'invention, B est un noyau benzénique, carbazolique ou indolique.

Selon un mode de réalisation particulier de l'invention, B est non substitué ou substitué par un ou plusieurs groupements pouvant être choisis parmi un radical halogéno, un radical alkyle en $C_1$-$C_6$, un radical alcoxy en $C_1$-$C_6$, un radical cyano, un radical trifluorométhyle, un radical alkylcarbonyle en $C_1$-$C_6$, un radical trifluorométhylsulfonyle, un radical trifluorométhylcarbonyle, un radical phénylsulfonyle, un radical phénylcarbonyle, un radical phényle non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un radical acylamino, un radical hydroxyle, un radical amino, un radical di(alkyl($C_1$-$C_6$))amino, un radical hydroxyalkyl($C_1$-$C_6$)amino, un radical di(hydroxyalkyl($C_1$-$C_6$))amino, un radical (aminoalkyl($C_1$-$C_6$))amino, un radical di(aminoalkyl($C_1$-$C_6$))amino, un radical (alkyl ($C_1$-$C_6$))(hydroxyalkyl($C_1$-$C$-$_6$))amino, un radical (aminoalkyl($C_1$-$C_6$))(alkyl($C_1$-$C$-$_6$))amino, un radical (aminoalkyl ($C_1$-$C_6$))(hydroxyalkyl($C_1$-$C_6$))amino, un radical hydroxyalkyle($C_1$-$C_6$), un radical aminoalkyle($C_1$-$C_6$), un radical alkyl ($C_1$-$C_6$)aminoalkyle($C_1$-$C_6$), un radical di(alkyl($C_1$-$C_6$))aminoalkyle($C_1$-$C_6$), un radical (hydroxyalkyl($C_1$-$C_6$))aminoalkyle ($C_1$-$C_6$), un radical di(hydroxyalkyl($C_1$-$C_6$))aminoalkyle($C_1$-$C_6$), un radical aminoalkyl($C_1$-$C_6$)aminoalkyle($C_1$-$C_6$), un radical di(aminoalkyl($C_1$-$C_6$))aminoalkyle($C_1$-$C_6$), un radical (alkyl($C_1$-$C_6$))(hydroxyalkyl($C_1$-$C_6$))aminoalkyle($C_1$-$C_6$), un radical (alkyl($C_1$-$C_6$))(aminoalkyl($C_1$-$C_6$))amino, un radical (hydroxyalkyl($C_1$-$C_6$))(alkyl($C_1$-$C_6$))aminoalkyle($C_1$-$C_6$), un radical phénylalkyle($C_1$-$C_6$) non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un groupement cationique du type ammonium quaternaire, un radical alkyle($C_1$-$C_6$) substitué par un groupement cationique du type ammonium quaternaire, un radical carboxyle, un radical alkyle($C_1$-$C_6$) substitué par un radical carboxyle, un radical thio, un radical thioalkyle($C_1$-$C_6$), un radical sulfonate, un radical alkyle($C_1$-$C_6$) substitué par un radical sulfonate, un radical alkyl($C_1$-$C_6$)carbonylalkyle($C_1$-$C_6$), un radical di(halogénoalkyl($C_1$-$C_6$))amino, un radical acétamido, un radical aryloxy, un radical aryloxyalkyle($C_1$-$C_6$), un radical éthényle, un radical éthénylcarbonyle, un groupement $NR_6R_7$, $R_6$ et $R_7$ pouvant former ensemble avec l'atome d'azote auquel ils sont attachés un cycle en $C_5$, $C_6$ ou $C_7$ non aromatique, éventuellement interrompu par un ou plusieurs hétéroatomes tels qu'un atome d'azote, d'oxygène ou de soufre, une chaîne alkylène pouvant contenir un atome d'oxygène ou de soufre et pouvant être terminée par un groupement cyano, alkylsulfonyle en $C_1$-$C_6$ ou alkylcarbonyle en $C_1$-$C_6$ ; deux groupements adjacents de B pouvant former un cycle aromatique ou hétéroaromatique, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, ou un cycle de type $-O-C_rH_{2r}-O-$ où r représente un entier égal à 1 ou 2. De préférence, B est non substitué ou substitué par un ou plusieurs groupements choisis parmi un radical hydroxyle ; un radical amino ; un radical di(alkyl ($C_1$-$C_6$))amino ; un radical alkyle en $C_1$-$C_6$ ; un radical acétamido ; un groupement pyridinium ; un groupement trialkyl ($C_1$-$C_6$)ammonium. A titre d'exemple, B peut être substitué par un radical hydroxyle ; un radical amino ; un radical diméthylamino ; un radical éthyle ; un radical acétamido ; un groupement pyridinium ; un groupement triméthylammonium.

Les groupements cationiques du type ammonium quaternaire peuvent par exemple être choisis parmi les groupements trialkylammonium, oxazolium, thiazolium, imidazolium, pyrazolium, pyridinium, pyrrolium, triazolium, isooxazolium, isothiazolium, pyrimidinium, pyrazinium, triazinium, pyridazinium, indolium, quinolinium ou isoquinolimiun, substitué ou non substitué, et peuvent être reliés au noyau A ou au noyau B par n'importe lequel de leurs atomes de carbone non substitués.

A titre d'exemples de composés de formule (I), on peut citer l'acide 9a-[2-[4-(diméthylamino)phényl]-1,3-butadiényl]-2,3,9,9a-tétrahydro-9,9-diméthyl oxazolo[3,2-a]indole-7-carboxylique ; l'acide [9a-[2-[4-(diméthylamino)phényl] éthènyl]-2,3,9,9a-tétrahydro-9,9-diméthyloxazolo[3,2-a]indol-7-yl] phosphonique ; la 4-[2-(9,9-diéthyl-2,3-dihydro-7-méthoxyoxazolo[3,2-a]indol-9a(9H)-yl)éthènyl]-N,N-diéthyl benzenamine ; l'acide [3-[9a-[2-[4-(diméthylamino)phényl] éthènyl]-2,3,9,9a-tétrahydro-9,9-diméthyloxazolo[3,2-a]indol-7-yl]propyl] phosphonique ; la 4-[2-(2,3-dihydro-9,9-diméthyloxazolo[3,2-a]indol-9a(9H)-yl)éthènyl]-N-méthyl-N-phényl benzènamine ; la 4-[2-(2,3-dihydro-9,9-diméthyloxazolo [3,2-a]indol-9a(9H)-yl)éthènyl]-3-éthoxy-N,N-diéthyl benzènamine ; la 4-[2-(2,3-dihydro-9,9-diméthyloxazolo[3,2-a]indol-9a(9H)-yl)éthènyl]-N-éthyl-N-(2-méthylpropyl) benzènamine; la 4-[2-(2,3-dihydrooxazolo[3,2-a]indol-9a(9H)-yl) éthènyl]-N,N-diméthyl benzènamine ; la 4-[4-(2,3-dihydro-7,9,9-triméthyloxazolo[3,2-a]indol-9a(9H)-yl)-1,3-butadiènyl]-N,N-diméthyl benzènamine ; la 4-[4-(2,3-dihydro-9,9-diméthyl-7-nitrooxazolo[3,2-a]indol-9a(9H)-yl)-1,3-butadiènyl]-N, N-diméthyl benzenamine ; le 9a-[4-[4-(diméthylamino)phényl]-1,3-butadiènyl]-2,3,9,9a-tétrahydro-9,9-diméthyl oxazolo [3,2-a]indole-7-carbonitrile ; le 9a-[2-[4-(diméthylamino)phényl]éthènyl]-2,3,9,9a-tétrahydro-9,9-diméthyl oxazolo[3,2-a] indole-7-carbonitrile ; la 4-[2-(2,3-dihydro-9,9-diméthyloxazolo[3,2-a]indol-9a(9H)-yl)éthènyl]-N,N-diméthyl benzenamine ; l'acide 9a-[2-[4-(diméthylamino)phényl]éthènyl]-2,3,9,9a-tétrahydro-9,9-diméthyl-méthylester oxazolo[3,2-a]indole-7-sulfonique ; la N,N-bis(2-chloroéthyl)-4-[2-(2,3-dihydro-9,9-diméthyloxazolo[3,2-a]indol-9a(9H)-yl)éthènyl] benzènamine ; la 9a-[2-[4-(diméthylamino)phényl]éthènyl]-9,9a-dihydro-9,9-diméthyl-7-(octylsulfonyl) oxazolo[3,2-a]indol-2(3H)-one ; la 9a-[2-[4-(diméthylamino)phényl]éthènyl]-9,9a-dihydro-9,9-diméthyl-7-(phénylsulfonyl) oxazolo[3,2-a] indol-2(3H)-one ; la 9a-[2-[4-(diméthylamino)phényl]-1-méthyléthènyl]-9,9a-dihydro-9,9-diméthyl-7-(méthylsulfonyl) oxazolo[3,2-a]indol-2(3H)-one ; la 9a-[2-[4-(diméthylamino)phényl]éthènyl]-9,9a-dihydro-9,9-diméthyl-7-(méthylsulfonyl) oxazolo[3,2-a]indol-2(3H)-one ; la 4-[2-[2,3-dihydro-9,9-diméthyl-7-(méthylsulfonyl)oxazolo[3,2-a]indol-9a(9H)-yl] éthènyl]-N,N-diméthyl benzènamine ; la 4-[2-[9-(éthoxyméthyl)-2,3-dihydro-9-méthyl-7-(méthylsulfonyl)oxazolo[3,2-a] indol-9a(9H)-yl]éthènyl]-N,N-diméthyl benzenamine ; la 4-[2-[2,3-dihydro-2,9,9-triméthyl-7-(méthylsulfonyl)oxazolo[3,2-a]indol-9a(9H)-yl]éthènyl]-N,N-diméthyl benzènamine ; la 4-[2-[2,3-dihydro-9,9-diméthyl-7-(méthylsulfonyl)oxazolo[3,2-a]indol-9a(9H)-yl]-1-propényl]-N,N-diméthylbenzènamine ; la N,N-dibutyl-4-[2-[2,3-dihydro-9,9-diméthyl-7-(méthylsulfonyl)oxazolo[3,2-a]indol-9a(9H)-yl]éthènyl] benzènamine ; 4-[2-[2,3-dihydro-9,9-diméthyl-7-(phénylsulfonyl)oxazolo[3,2-a]indol-9a(9H)-yl]éthènyl]-N,N-diméthyle ; la 4-[2-[2,3-dihydro-9,9-diméthyl-7-(octylsulfonyl)oxazolo[3,2-a]indol-9a (9H)-yl]éthènyl]-N,N-diméthyl benzènamine ; le N-[4-[2-[7-(butylsulfonyl)-2,3-dihydro-9,9-diméthyloxazolo[3,2-a]indol-9a(9H)-yl]éthènyl]phényl] acétamide ; la 4-[2-[7-(butylsulfonyl)-2,3-dihydro-9,9-diméthyloxazolo[3,2-a]indol-9a(9H)-yl] éthènyl]-N,N-diméthyl benzènamine ; la 4-[4-[2,3-dihydro-9,9-diméthyl-7-(méthylsulfonyl)oxazolo[3,2-a]indol-9a(9H)-yl]-1,3-butadiènyl]-N,N-diméthyl benzènamine ; le 9a-[2-[4-(diméthylamino)phényl]éthènyl]-2,3,9,9a-tétrahydro-9-méthyl oxazolo[3,2-a]indole-9-éthanol ; la 4-[2-(9,9-diéthyl-2,3-dihydrooxazolo[3,2-a]indol-9a(9H)-yl)éthènyl]-N,N-diméthyl benzènamine ; la 4-[2-[2,3-dihydro-9-méthyl-9-(2-phénoxyéthyl)oxazolo[3,2-a]indol-9a(9H)-yl]éthènyl]-N,N-diméthyl benzènamine ; la 4-[2-[9-(éthoxyméthyl)-2,3-dihydro-9-méthyloxazolo[3,2-a]indol-9a(9H)-yl]éthènyl]-N,N-diméthyl benzènamine ; la 4-[2-(11,11-diméthylbenz[e]oxazolo[3,2-a]indol-10a(11H)-yl)éthènyl]-N,N-diméthyl benzenamine ; la 4-[2-(7-méthoxy-9,9-diméthyloxazolo[3,2-a]indol-9a(9H)-yl)éthènyl]-N,N-diméthyl benzènamine ; la 4-[2-(9,9-diméthyloxazolo[3,2-a]indol-9a(9H)-yl)éthènyl]-N,N-diméthyl benzènamine ; la 9a-[2-[4-(diméthylamino)phényl]éthènyl]-3-éthyl-9,9a-dihydro-9,9-diméthyl oxazolo[3,2-a]indol-2(3H)-one ; la 7-chloro-9a-[2-[4-(diméthylamino)phényl]éthènyl]-9,9a-dihydro-3,9,9-triméthyl oxazolo[3,2-a]indol-2(3H)-one ; la 9a-[2-[4-(dibutylamino)phényl]éthènyl]-9,9a-dihydro-9,9-diméthyl oxazolo[3,2-a]indol-2(3H)-one ; la 9a-[2-[4-(diméthylamino)phényl]éthènyl]-9,9a-dihydro-9-(2-hydroxyéthyl)-9-méthyl oxazolo[3,2-a]indol-2(3H)-one ; la 9a-[2-[4-(diméthylamino)phényl]-1-propényl]-9,9a-dihydro-9,9-diméthyl oxazolo[3,2-a]indol-2(3H)-one ; la 9,9a-dihydro-7,9,9-triméthyl-9a-[2-(4-nitrophényl)éthènyl] oxazolo[3,2-a]indol-2 (3H)- one ; le N-[4-[2-(2,3-dihydro-9,9-diméthyl-2-oxooxazolo[3,2-a]indol-9a(9H)-yl)éthènyl]phényl] acetamide ; la 9a-[2- [4-(diméthylamino)phényl]éthènyl]-9,9a-dihydro-6-méthoxy-9,9-diméthyl oxazolo[3,2-a]indol-2(3H)-one ; l'acide 9a-[2- [4-(diméthylamino)phényl]éthènyl]-2,3,9,9a-tétrahydro-9,9-diméthyl-2-oxo-éthyl ester oxazolo[3,2-a]indole-7-carboxylique ; la 9a-[2-[4-(diméthylamino)phényl]éthènyl]-9,9a-dihydro-7,9,9-triméthyl oxazolo[3,2-a]indol-2(3H)-one ; la 10a- [2-[4-(diméthylamino)phényl]éthènyl]-10a,11-dihydro-11,11-diméthyl benz[e]oxazolo[3,2-a]indol-9(8H)-one; la 9,9a-dihydro-9,9-diméthyl-9a-[2-(4-nitrophényl)éthènyl] oxazolo[3,2-a]indol-2(3H)-one ; la 7-chloro-9a-[2-[4-(diméthylamino)phényl]éthènyl]-9,9a-dihydro-9,9-diméthyl oxazolo[3,2-a]indol-2(3H)-one; la 9a-[2-[4-(diméthylamino)phényl] éthènyl]-9,9a-dihydro-9,9-diméthyl oxazolo[3,2-a]indol-2(3H)-one; la 4-[2-(7-chloro-2,3-dihydro-9,9-diméthyloxazolo [3,2-a]indol-9a(9H)-yl)éthènyl]-N,N-diméthyl benzènamine ; la 4-[2-(2,3-dihydro-9,9-diméthyloxazolo[3,2-a]indol-9a (9H)-yl)éthènyl]-N,N-diméthyl benzènamine ; la 4-[2-(7-chloro-2,3-dihydro-2,9,9-triméthyloxazolo[3,2-a]indol-9a(9H)-yl) éthènyl]-N,N-diméthyl benzènamine ; la 4-[2-(2,3-dihydro-9,9-diméthyloxazolo[3,2-a]indol-9a(9H)-yl)-1-méthyléthènyl]-N,N-diméthyl benzènamine ; la 4-[2-(2,3-dihydro-9,9-diméthyloxazolo[3,2-a]indol-9a(9H)-yl)éthènyl]-N,N-diéthyl

benzènamine ; le 2,3,9,9a-tétrahydro-7-méthoxy-9,9-diméthyl-9a-[2-(4-nitrophényl)éthènyl] oxazolo[3,2-a]indole ; la 4-[2-(2,3-dihydro-9,9-diméthyl-7-nitrooxazolo[3,2-a]indol-9a(9H)-yl)éthènyl]-N,N-diméthyl benzènamine ; la N,N-dibutyl-4-[2-(2,3-dihydro-9,9-diméthyloxazolo[3,2-a]indol-9a(9H)-yl)éthènyl] benzènamine ; le 2,3,9,9a-tétrahydro-9,9-diméthyl-7-nitro-9a-[2-(4-nitrophényl)éthènyl] oxazolo[3,2-a]indole ; le 7-chloro-2,3,9,9a-tétrahydro-9,9-diméthyl-9a-[2-(4-nitrophényl)éthènyl] oxazolo[3,2-a]indole ; la 4-[2-(2,3-dihydro-7-iodo-9,9-diméthyloxazolo[3,2-a]indol-9a(9H)-yl)éthènyl]-N,N-diméthyl benzènamine ; la 4-[2-(2,3-dihydro-5-méthoxy-9,9-diméthyloxazolo[3,2-a]indol-9a(9H)-yl)éthènyl]-N,N-diméthyl benzènamine ; la 4-[2-(2,3-dihydro-7-méthoxy-9,9-diméthyloxazolo[3,2-a]indol-9a(9H)-yl)éthènyl]-N,N-diméthyl benzènamine ; la 4-[4-(2,3-dihydro-9,9-diméthyloxazolo[3,2-a]indol-9a(9H)-yl)-1,3-butadiènyl]-N,N-diéthyl benzènamine ; la 4-[4-(7-chloro-2,3-dihydro-9,9-diméthyloxazolo[3,2-a]indol-9a(9H)-yl)-1,3-butadiènyl]-N,N-diméthyl benzènamine ; la 4-[4-(2,3-dihydro-9,9-diméthyloxazolo[3,2-a]indol-9a(9H)-yl)-1,3-butadiènyl]-N,N-diméthyl benzènamine ; la 4-[4-(2,3-dihydro-7-méthoxy-9,9-diméthyloxazolo[3,2-a]indol-9a(9H)-yl)-1,3-butadiènyl]-N,N-diméthyl benzènamine ; le 2,3,9,9a-tétrahydro-7,9,9-triméthyl-9a-[2-(4-nitrophényl)éthènyl] oxazolo[3,2-a]indole ; le N-[4-[2-(2,3-dihydro-9,9-diméthyloxazolo[3,2-a]indol-9a(9H)-yl)éthènyl]phényl] acétamide ; la 4-[2-(2,3-dihydro-6-méthoxy-9,9-diméthyloxazolo[3,2-a]indol-9a(9H)-yl)éthènyl]-N,N-diméthyl benzènamine; la 4-[2-(8,9-dihydro-11,11-diméthylbenz[e]oxazolo[3,2-a]indol-10a(11H)-yl)éthènyl]-N,N-diméthyl benzènamine ; la 4-[2-(2,3-dihydro-7,9,9-triméthyloxazolo[3,2-a]indol-9a(9H)-yl)éthènyl]-N,N-diméthyl benzènamine ; le 2,3,9,9a-tétrahydro-9,9-diméthyl-9a-[2-(4-nitrophényl)éthènyl] oxazolo[3,2-a]indole ; la 9,9a-dihydro-9,9-diméthyl-9a-[2-(9-méthyl-9H-carbazol-3-yl)éthènyl]-7-(méthylsulfonyl) oxazolo[3,2-a]indol-2(3H)-one; le 9a-[2-(9-hexyl-9H-carbazol-3-yl)éthènyl]-2,3,9,9a-tétrahydro-9,9-diméthyl-7-(phénylsulfonyl) oxazolo[3,2-a]indole ; le 2,3,9,9a-tétrahydro-9,9-diméthyl-7-(méthylsulfonyl)-9a-[2-(9-octyl-9H-carbazol-3-yl)éthènyl] oxazolo[3,2-a]indole ; l'acide 9a-[2-(9-butyl-6-méthoxy-9H-carbazol-3-yl)éthènyl]-2,3,9,9a-tétrahydro-9,9-diméthyl-, éthyl ester oxazolo[3,2-a]indole-7-carboxylique ; l'acide 9a-[2-(9-éthyl-9H-carbazol-3-yl)éthènyl]-2,3,9,9a-tétrahydro-9,9-diméthyl-, méthyl ester oxazolo[3,2-a]indole-7-sulfonique ; le 3-chloro-6-[2-[2,3-dihydro-9,9-diméthyl-7-(méthylsulfonyl)oxazolo[3,2-a]indol-9a(9H)-yl]éthènyl]-9-octyl 9H-carbazole ; le 3-[2-[2,3-dihydro-9,9-diméthyl-7-(méthylsulfonyl)oxazolo[3,2-a]indol-9a(9H)-yl]éthènyl]-9-méthyl 9H-carbazole ; le 3-[2-[9-(2-éthoxyéthyl)-2,3-dihydro-9-méthyl-7-(méthylsulfonyl)oxazolo[3,2-a]indol-9a(9H)-yl]éthènyl] 9H-carbazole ; l'acide 9a-[2-(9-hexyl-9H-carbazol-3-yl)éthènyl]-2,3,9,9a-tétrahydro-9-(2-hydroxyéthyl)-9-méthyl-, éthyl ester oxazolo[3,2-a]indole-7-carboxylique ; l'acide 2,3,9,9a-tétrahydro-9,9-diméthyl-9a-[2-(9-octyl-9H-carbazol-3-yl)éthènyl]-, éthyl ester oxazolo[3,2-a]indole-7-carboxylique ; l'acide 9a-[2-(9-butyl-9H-carbazol-3-yl)éthènyl]-2,3,9,9a-tétrahydro-9,9-diméthyl-, éthyl ester oxazolo[3,2-a]indole-7-carboxylique ; l'acide 2,3,9,9a-tétrahydro-9,9-diméthyl-9a-[2-(9-méthyl-9H-carbazol-3-yl)éthènyl]-, éthyl ester oxazolo[3,2-a]indole-7-carboxylique ; la 9a-[2-(9-butyl-6-éthoxy-9H-carbazol-3-yl)éthènyl]-2,3,9,9a-tétrahydro-N,N,9,9-tétraméthyl oxazolo[3,2-a]indol-7-amine ; le 2,3,9,9a-tétrahydro-9-méthyl-9a-[2-(9-méthyl-9H-carbazol-3-yl)éthènyl] oxazolo[3,2-a]indole-9-éthanol ; la 9a-[2-(9-butyl-9H-carbazol-3-yl)éthènyl]-2,3,9,9a-tétrahydro-N,N,9,9-tétraméthyl oxazolo[3,2-a]indol-7-amine ; le 3-[2-(2,3-dihydro-6-méthoxy-9,9-diméthyloxazolo[3,2-a]indol-9a(9H)-yl)éthènyl]-9-méthyl 9H-carbazole ; le 3-[2-(2,3-dihydro-9,9-diméthyloxazolo[3,2-a]indol-9a(9H)-yl)-1-propényl]-9-méthyl 9H-carbazole ; le 3-[2-(7-chloro-2,3-dihydro-9,9-diméthyloxazolo[3,2-a]indol-9a(9H)-yl)éthènyl]-9-méthyl 9H-carbazole ; le 3-bromo-6-[2-(2,3-dihydro-9,9-diméthyloxazolo[3,2-a]indol-9a(9H)-yl)éthènyl]-9-éthyl 9H-carbazole ; le 3-[2-(2,3-dihydro-9,9-diméthyloxazolo[3,2-a]indol-9a(9H)-yl)éthènyl]-9-méthyl 9H-carbazole.

De préférence, le ou les composés de formule (I) sont choisis parmi les composés présentés dans le tableau ci-dessous :

| | |
|---|---|
| 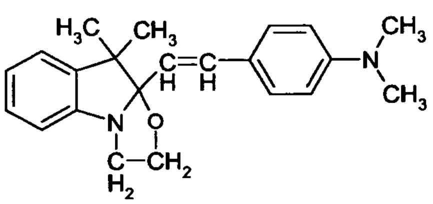 | Colorant 1 |
| 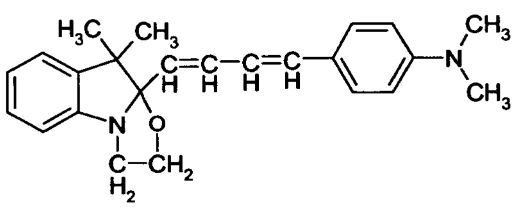 | Colorant 2 |

(suite)

| | |
|---|---|
| 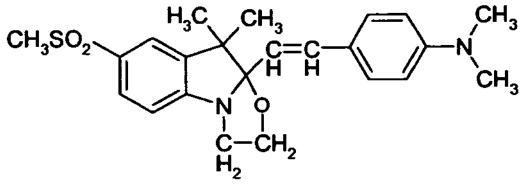 | Colorant 3 |
| 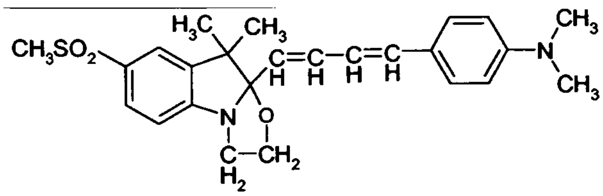 | Colorant 4 |
| 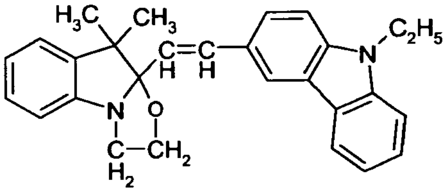 | Colorant 5 |
| 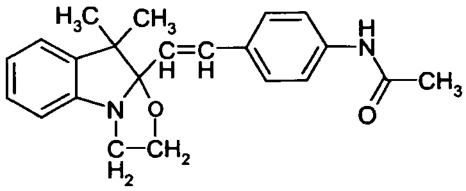 | Colorant 6 |
| 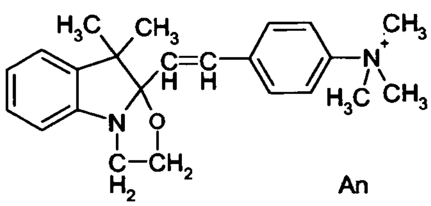 | Colorant 7 |
| 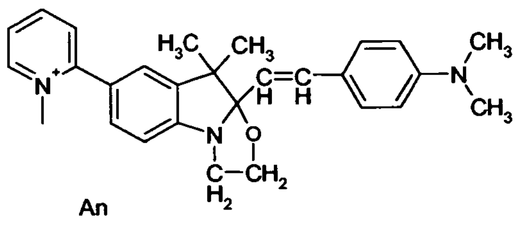 | Colorant 8 |

(suite)

| | |
|---|---|
| 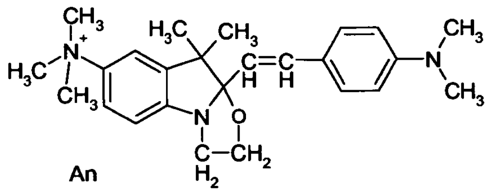 | Colorant 9 |
| 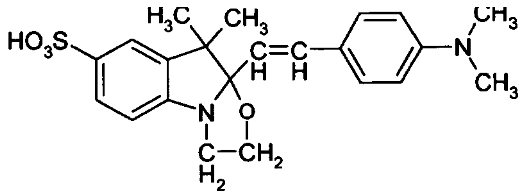 | Colorant 10 |
| 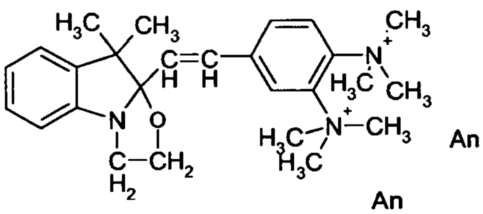 | Colorant 11 |

dans lesquels An, identiques ou différents, représentent un contre-ion négatif pouvant par exemple être choisis parmi un halogénure tel qu'un chlorure, un bromure, un iodure ou un fluorure, un perchlorate, un p-méthylbenzène sulfonate, un tétrafluoroborate, un sulfate, un alkylsulfate, un toluènesulfonate, un sulfonate.

De préférence, le ou les composés de formule (I) sont choisis parmi les composés présentés dans le tableau ci-dessous :

| | |
|---|---|
| 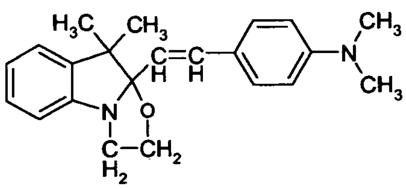 | Colorant 1 |
| 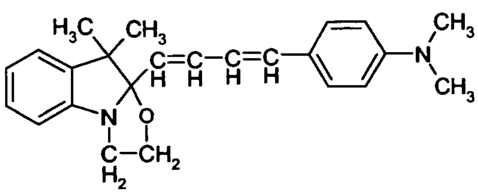 | Colorant 2 |
| 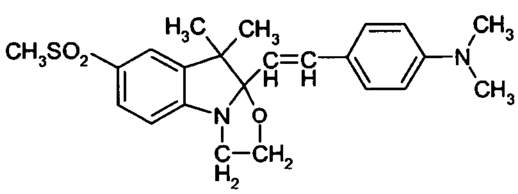 | Colorant 3 |

(suite)

| | |
|---|---|
| 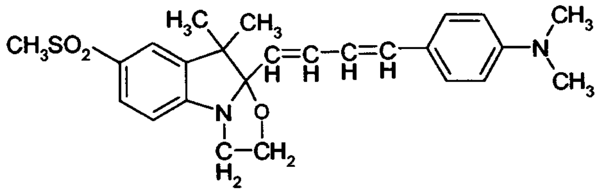 | Colorant 4 |
| 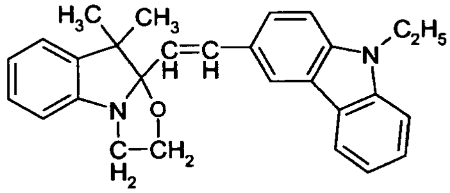 | Colorant 5 |
| 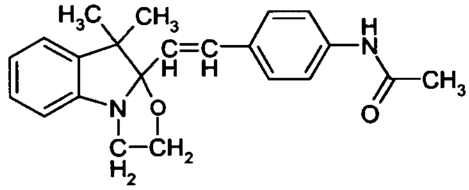 | Colorant 6 |
| 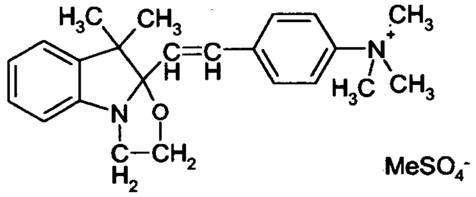 | Colorant 7' |
| 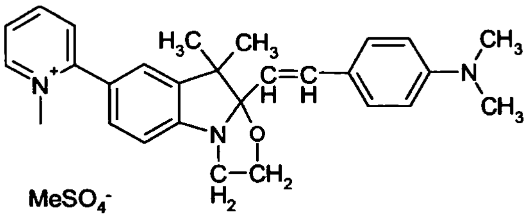 | Colorant 8' |
| 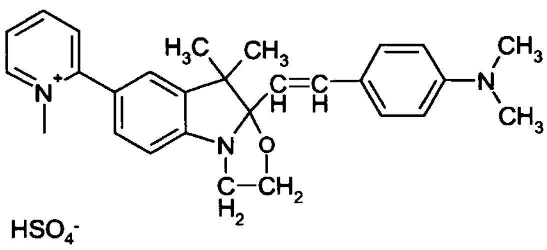 | Colorant 8" |

(suite)

| | |
|---|---|
| 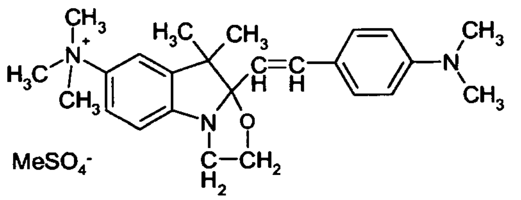 | Colorant 9' |
| 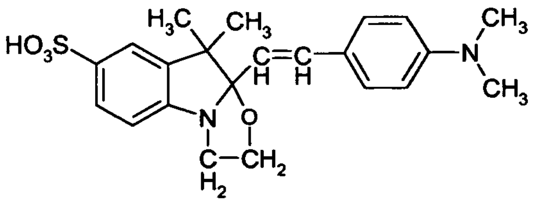 | Colorant 10 |
| 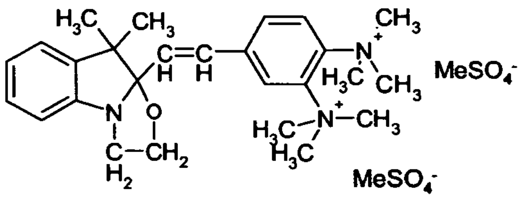 | Colorant 11' |

Les composés de formule (I) utiles dans le cadre de la présente invention peuvent par exemple être préparés selon les modes de préparation tels que décrits dans les brevets FR 2 285 439 et US 4 380 629. Ces modes de préparation peuvent être adaptés aux composés de formule (I) cationiques en ajoutant une étape de quaternisation. A titre d'exemple, on peut réaliser la synthèse du colorant 8 selon le schéma réactionnel suivant :

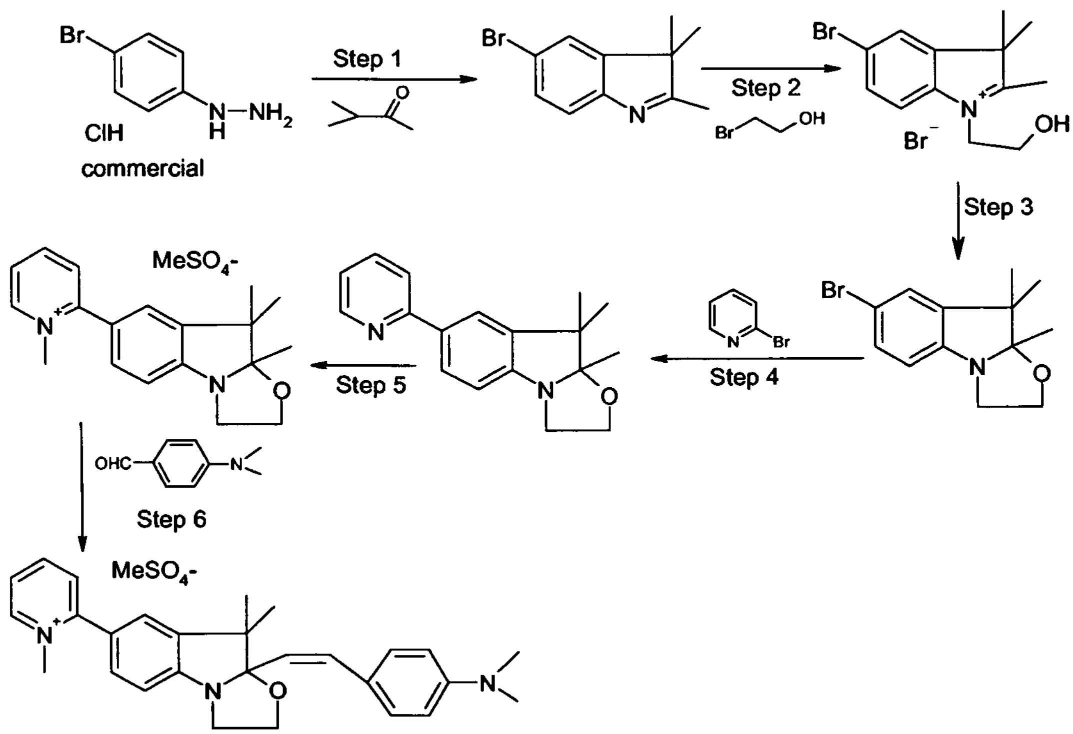


Le ou les composés choisis parmi les composés de formule (I), les composés de formule (II) et leurs sels d'addition représentent en général de 0,0001 à 10 % en poids par rapport au poids total de la composition tinctoriale utile dans le cadre de l'invention.

D'une manière générale, les sels d'addition des composés de formule (I) et des composés de formule (II) utilisables dans le cadre de l'invention sont notamment choisis parmi les sels d'addition avec un acide tels que les chlorhydrates, les bromhydrates, les sulfates, les méthosulfates, les gluconates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition utile dans le cadre de l'invention peut en outre contenir un ou plusieurs colorants directs pouvant notamment être choisis parmi les colorants nitrés de la série benzénique, les colorants directs azoïques, les colorants directs méthiniques et les colorants naturels. Ces colorants directs peuvent être de nature non ionique, anionique ou cationique.

En présence de colorants directs additionnels dans la composition utile dans le cadre de l'invention, ceux-ci représentent en général de 0,001 à 20 % en poids par rapport au poids total de la composition tinctoriale utile dans le cadre de l'invention, de préférence de 0,01 à 10 %.

La composition utile dans le cadre de l'invention peut en outre comprendre un ou plusieurs colorants d'oxydation choisis parmi les bases d'oxydation et les coupleurs classiquement utilisées en coloration d'oxydation.

Les bases d'oxydation peuvent être choisies parmi les para-phénylènediamines, les bis-phénylalkylènediamines, les para-aminophénols, les bis-para-aminophénols, les ortho-aminophénols, les ortho-phénylènediamines, les bases hétérocycliques et leurs sels d'addition.

Les coupleurs peuvent être choisis parmi les méta-phénylènediamines, les méta-aminophénols, les méta-diphénols, les coupleurs naphtaléniques, les coupleurs hétérocycliques et leurs sels d'addition.

En présence de colorants d'oxydation dans la composition conforme à l'invention, ceux-ci représentent en général de 0,001 à 20 % en poids par rapport au poids total de la composition tinctoriale utile dans le cadre de l'invention, de préférence de 0,01 à 10 %.

La composition utile dans le cadre de l'invention peut également comprendre un ou plusieurs agents acidifiants et / ou un ou plusieurs agents alcalinisants habituellement utilisés en teinture des fibres kératiniques.

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, une fonction acide sulfonique, une fonction acide phosphonique ou une fonction acide phosphorique.

Parmi les agents alcalinisants on peut citer, à titre d'exemple :

- les acides aminés basiques ;
- les carbonates ou les bicarbonates de métaux alcalins ou alcalino-terreux ;
- les silicates ou les métasilicates ;
- les composés de formule (III) suivante :

$$X(OH)_n \qquad (III)$$

dans laquelle :

- X représente un ion potassium, lithium, sodium, ammonium $N^+R_8R_9R_{10}R_{11}$ avec $R_8$, $R_9$, $R_{10}$ et $R_{11}$, identiques ou différents, désignant un radical alkyle en $C_2$-$C_4$ lorsque n est égal à 1 ;
- X représente un atome de magnésium ou de calcium lorsque n est égal à 2 ;

et en particulier les hydroxyde de sodium ou de potassium ;

- les composés de formule (IV) suivante :

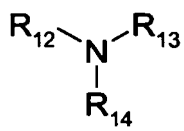

(IV)

dans laquelle :

- $R_{12}$ représente un atome d'hydrogène ; un radical alkyle en $C_1$-$C_6$, un radical monohydroxyalkyle en $C_1$-$C_6$ ; un radical polyhydroxyalkyle en $C_2$-$C_6$ ;
- $R_{13}$ et $R_{14}$, identiques ou différents, représentent un atome d'hydrogène, un radical alkyle en $C_1$-$C_6$, un radical monohydroxyalkyle en $C_1$-$C_6$ ; un radical polyhydroxyalkyle en $C_2$-$C_6$ ;

et en particulier l'ammoniaque et les alcanolamines telles que les mono-, di- et triéthanolamines ainsi que leurs dérivés ;

- les composés de formule (V) suivante :

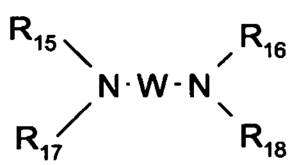

(V)

dans laquelle :

- W est un reste propylène éventuellement substitué par un groupement hydroxyle ou un radical alkyle en $C_1$-$C_4$ ;
- $R_{15}$, $R_{16}$, $R_{17}$ et $R_{18}$, identiques ou différents, représentent un atome d'hydrogène, un radical alkyle en $C_1$-$C_4$ ou hydroxyalkyle en $C_1$-$C_4$.

Au sens de la présente invention, on entend par "acide aminé basique", soit (i) un acide aminé présentant en plus de la fonction amine positionnée en $\alpha$ du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22, A. LEHNINGER (1993) pages 115-116, DE BOECK-WESMAEL (1994) pages 57-59.

Les acides aminés basiques conformes à l'invention sont choisis de préférence parmi ceux répondant à la formule (D) suivante :

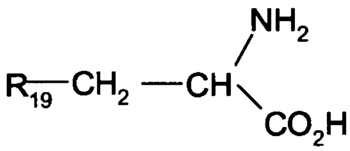

**(D)**

où $R_{19}$ désigne un groupe choisi parmi :

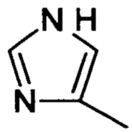

;

$-(CH_2)_3NH_2$ ;

$-(CH_2)_2NH_2$ ;

$-(CH_2)_2NHCONH_2$ ;

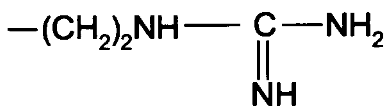

;

Parmi les composés de formule (D), on peut citer à titre d'exemple l'histidine, la lysine, l'ornithine, la citrulline, l'arginine.

Le milieu approprié pour la teinture, appelé aussi support de teinture, est généralement constitué par de l'eau ou par au moins un solvant organique ou par un mélange d'eau et d'au moins un solvant organique. A titre de solvant organique, on peut par exemple citer les cétones tels que l'acétone ; les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les polyols ou éthers de polyol tels que, par exemple, les éthers monométhylique, monoéthylique et monobutylique d'éthylèneglycol, le 2-butoxyéthanol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en $C_1$-$C_4$, comme par exemple, le monoéthyléther ou le monobutyléther du diéthylèneglycol, seuls ou en mélange.

Les solvants sont généralement présents dans des proportions comprises entre 1 et 40 % en poids environ par rapport au poids total de la composition tinctoriale, et encore plus préférentiellement entre 5 et 30 % en poids environ.

Le ou les composés choisis parmi les composés de formule (I), les composés de formule (II) et leurs sels d'addition sont soit solubles, soit en dispersion, dans le support de teinture.

La composition utile dans le cadre de l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les

épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des polymères cationiques ou amphotères, des cations, des silicones volatiles ou non volatiles, modifiées ou non modifiées, des chitosanes ou des dérivés de chitosane, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture utile dans le cadre de l'invention ne soient pas, ou substantiellement pas, altérées par la ou les adjonctions envisagées.

Lorsque le milieu de teinture est constitué par de l'eau ou par un mélange d'eau et d'au moins un solvant organique, le pH de la composition tinctoriale utile dans le cadre de l'invention est généralement compris entre 2 et 12, et de préférence entre 3 et 11. Il peut être ajusté à la valeur désirée au moyen d'agents acidifiants et / ou d'agents alcalinisants tels que définis précédemment ou bien encore à l'aide de systèmes tampons classiques.

La composition utile dans le cadre de l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de coloration des fibres kératiniques conforme à l'invention est un procédé dans lequel la composition tinctoriale utile dans le cadre de l'invention est appliquée sur les fibres kératiniques pendant un temps de pose suffisant, cette application étant éventuellement suivie d'un rinçage.

Lorsque les composés utiles dans le cadre de la présente invention sont appliqués sur les fibres kératiniques sous leur forme incolore, la coloration peut être révélée sur les fibres kératiniques par action d'un agent extérieur tel que la lumière, un courant électrique, la chaleur, un agent acidifiant et / ou un agent alcalinisant et / ou un solvant, une radiation électromagnétique.

Lorsque la coloration est révélée par action de la chaleur, les fibres kératiniques peuvent être chauffées à l'aide d'un casque, d'un sèche cheveux ou d'un fer à friser, avant ou après l'application de la composition tinctoriale.

Lorsque la coloration est révélée par action d'un agent acidifiant, d'un agent alcalinisant ou d'un solvant, celui-ci peut être ajouté à la composition tinctoriale utile dans le cadre de l'invention juste au moment de l'emploi ou il peut être mis en oeuvre à partir d'une composition le contenant, appliquée simultanément ou séquentiellement à la composition tinctoriale utile dans le cadre de l'invention.

Selon un mode de réalisation particulier de l'invention, le ou les composés utiles dans le cadre de l'invention sont appliqués sur les fibres kératiniques sous leur forme incolore et la coloration est révélée sur les fibres kératiniques lorsque la composition entre en contact avec les fibres kératiniques ou grâce à un pré- ou post-traitement consistant à traiter les fibres kératiniques par action d'un agent extérieur tel que la lumière, un courant électrique, la chaleur, un agent acidifiant et / ou un agent alcalinisant et / ou un solvant ou une radiation électromagnétique.

Selon un autre mode de réalisation particulier de l'invention, la composition tinctoriale utile dans le cadre de l'invention est appliquée en présence d'un agent oxydant. Dans ce cas-là, on réalise une coloration et une décoloration simultanées des fibres kératiniques.

L'agent oxydant peut être ajouté à la composition tinctoriale juste au moment de l'emploi ou il peut être mis en oeuvre à partir d'une composition oxydante le contenant, appliquée simultanément ou séquentiellement à la composition tinctoriale.

L'agent oxydant peut être choisi parmi le peroxyde d'hydrogène, le peroxyde d'urée, les bromates de métaux alcalins, les persels, les peracides et les enzymes de type oxydase.

Le temps de pose de la composition utile dans le cadre de l'invention est généralement compris entre 5 minutes et 1 heure, de préférence entre 15 minutes et 1 heure.

La température d'application est généralement fixée entre la température ambiante et 80˚C, de préférence entre la température ambiante et 60 ˚C.

La présente invention a également pour objet un dispositif à plusieurs compartiments permettant de mettre en oeuvre le procédé de coloration des fibres kératiniques conforme à l'invention.

Le dispositif à plusieurs compartiments de l'invention contient dans un premier compartiment une composition tinctoriale comprenant au moins un composé choisi parmi les composés de formule (I), les composés de formule (II) et leurs sels d'addition tels que définis précédemment et dans un deuxième compartiment au moins un agent acidifiant, au moins un agent alcalinisant et /ou au moins un solvant tels que définis précédemment capables d'ouvrir l'hétérocycle présent dans les composés de formule (I).

Selon un mode de réalisation particulier de l'invention, le dispositif à plusieurs compartiments de l'invention contient dans un troisième compartiment au moins un agent oxydant tel que défini précédemment.

Les composés utiles dans le cadre de l'invention tels que définis précédemment qui comportent au moins un groupement cationique du type ammonium quaternaire tel que les groupements trialkyl($C_1$-$C_6$)ammonium, oxazolium,

thiazolium, imidazolium, pyrazolium, pyridinium, pyrrolium, triazolium, isooxazolium, isothiazolium, pyrimidinium, pyrazinium, triazinium, pyridazinium, indolium, quinolinium ou isoquinolimiun, substitué ou non substitué, sont nouveaux et constituent un autre objet de la présente invention.

La présente invention a également pour objet une composition tinctoriale comprenant, dans un milieu approprié pour la teinture, au moins un composé utile dans le cadre de l'invention tel que défini précédemment comportant au moins un groupement cationique du type ammonium quaternaire.

Les exemples qui suivent servent à illustrer l'invention sans toutefois présenter un caractère limitatif.

## EXEMPLES

### Exemple de synthèse

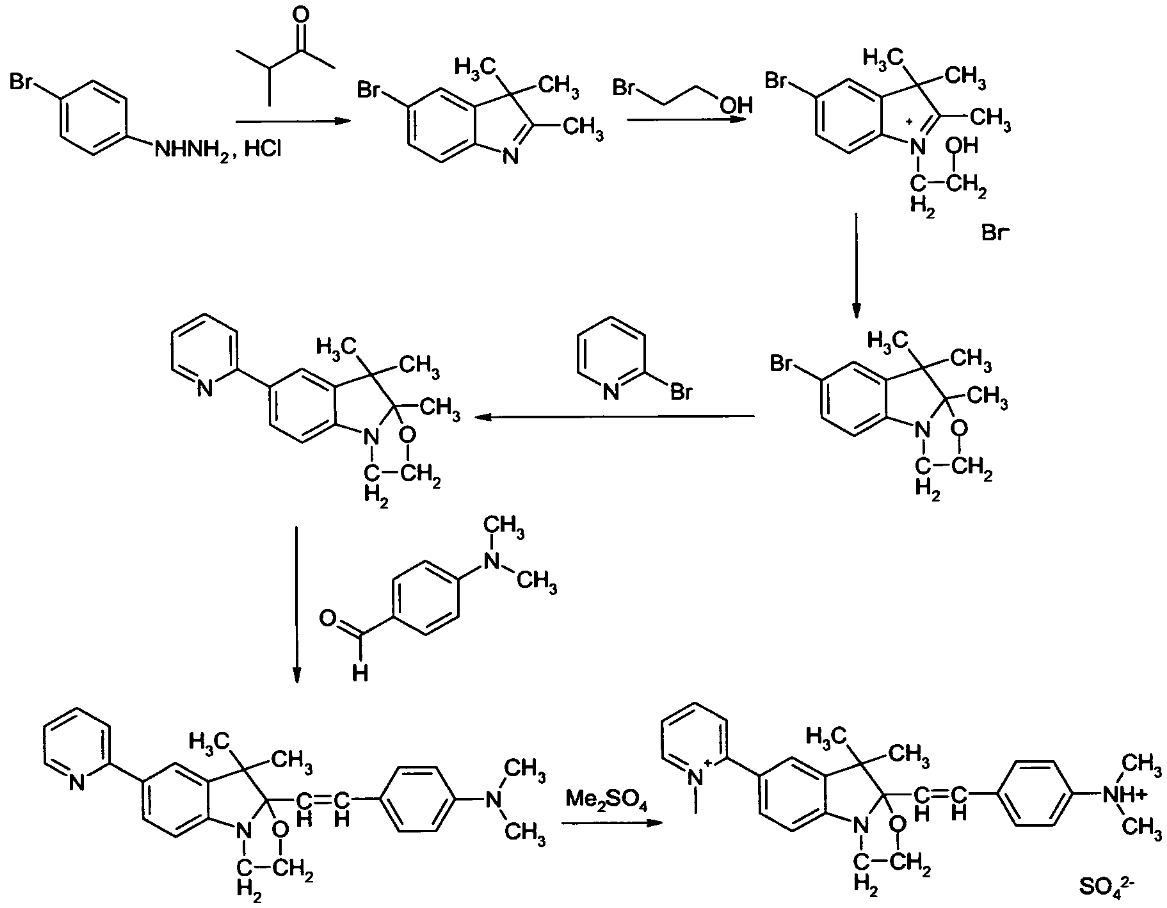


Etape 1 :

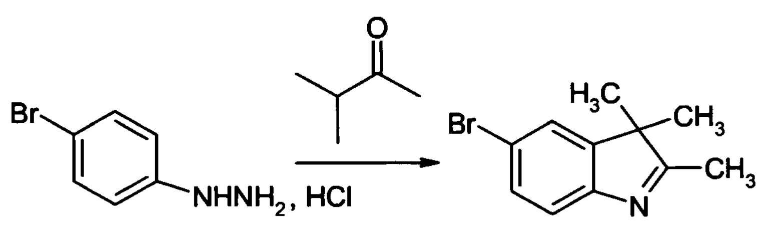

164,5 g de chlorhydrate de (4-bromophényl)hydrazine (0,7 mole), 72 g de 3-méthylbutan-2-one (0,84 mole) et 600 mL d'éthanol sont placés dans un réacteur, puis 70 mL d'acide sulfurique à 95 % sont ajoutés lentement. Le milieu réactionnel est porté au reflux, et le reflux est maintenu pendant 5 heures. Le milieu réactionnel est ensuite refroidi à température ambiante, puis 470 mL d'une solution de carbonate de sodium à 10 % sont ajoutés afin d'obtenir un pH égal à 8. Le mélange ainsi obtenu est extrait à l'acétate d'éthyle. Après évaporation de l'acétate d'éthyle, 164 g d'un liquide rouge-brun sont obtenus, ce qui correspond à un rendement de 98 %.

Etape 2 :

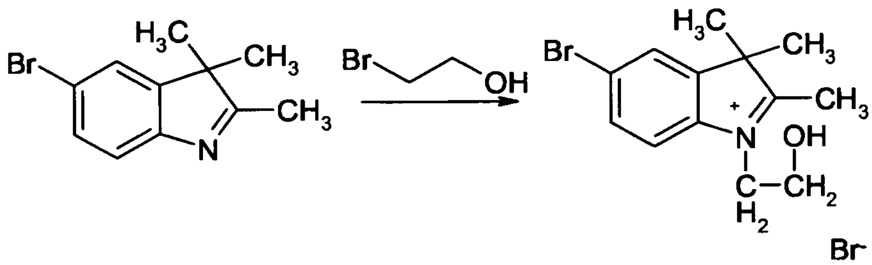

164 g de produit obtenu à l'étape 1 (0,68 mole) et 83,5 g de 2-bromoéthanol (0,68 mole) sont placés dans un réacteur. Le milieu réactionnel est porté à 80 °C et agité à cette température pendant 24 heures. Le milieu réactionnel est ensuite refroidi à température ambiante, filtré, puis le solide obtenu est lavé à l'acétone et séché sous vide. 167 g d'un solide blanc est obtenu, soit un rendement de 68 %.

Etape 3 :

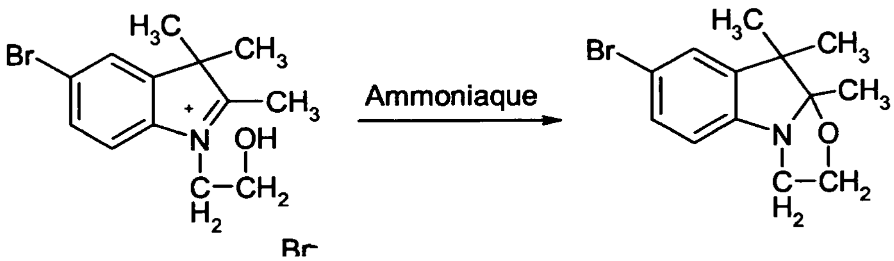


167 g de produit obtenu à l'étape 2 (0,462 mole), 300 mL d'eau et 300 mL d'ammoniaque sont placés dans un réacteur et le milieu réactionnel est agité pendant 3 minutes. Le milieu réactionnel est ensuite extrait à l'éther. Après séchage de la phase organique sur sulfate de sodium, filtration et évaporation sous vide, 117 g d'un solide jaune est obtenu, ce qui correspond à un rendement de 90,1 %.

Etape 4 :

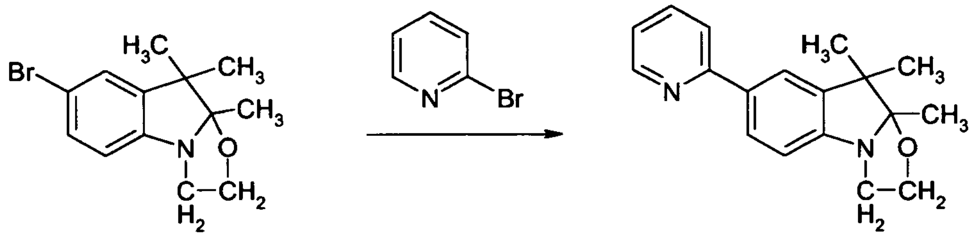

30 g de produit obtenu à l'étape 3 (107 mmoles) sont placés dans un réacteur sous atmosphère inerte. 100 mL de THF anhydre sont ajoutés puis le milieu réactionnel est porté à - 90 °C. 76 mL de n-butyl lithium (181 mmoles) sont ajoutés goutte à goutte et le milieu réactionnel est agité pendant une heure entre - 80 et - 90 °C. 107 mL d'une solution de $ZnCl_2$ à 1 M dans le THF anhydre (107 mmoles) sont ajoutés et la température du milieu réactionnel est ramenée à la température ambiante. L'agitation est alors maintenue pendant 30 minutes. 3,7 g de $Pd(PPh_3)_4$ (3,2 mmoles) et 10,4 mL de 2-bromopyridine (123 mmoles) sont ajoutés lentement, puis l'agitation est maintenue pendant 2 heures à température ambiante. 100 mL d'eau sont ajoutés, et le mélange est filtré. La phase aqueuse est extraite à l'acétate d'éthyle. Les phase organiques sont réunies, puis séchées sur gel de silice. Après filtration et évaporation sous vide, le produit obtenu est purifié sur colonne chromatographique avec le système éther de pétrôle / acétate d'éthyle (4 / 1) comme éluant. 18,6 g de produit sont obtenus, soit un rendement de 62 %.

Etape 5 :

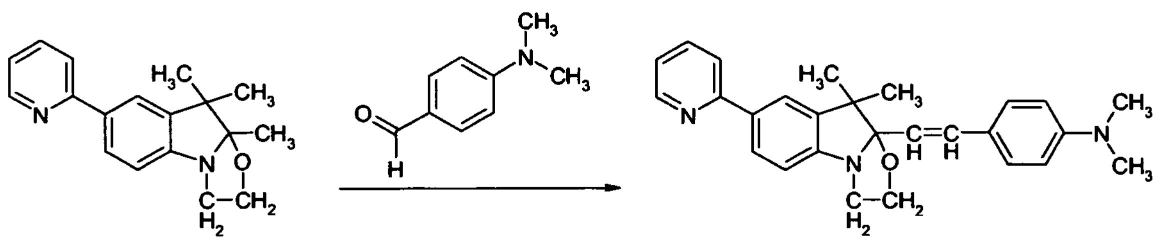

24 g de produit obtenu à l'étape 4 (0,086 mole), 80 mL de toluène, 14 g de 4-diméthylaminobenzaldéhyde (0,093 mole) et 9 mL de pipéridine sont placés dans un réacteur, puis le milieu réactionnel est porté au reflux. Après 5 heures de reflux, le milieu est ramené à la température ambiante. Le produit est purifié par colonne chromatographique sur gel de silice avec comme éluant le système éther de pétrôle / acétate d'éthyle (4 / 1). 16,5 g de solide blanc sont obtenus, soit un rendement de 46,7 %.

Etape 6 :

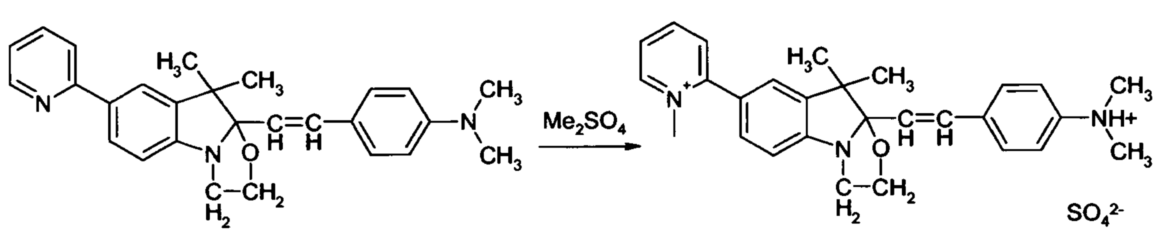


2 g de produit obtenu à l'étape 5 (4,8 mmoles), 20 mL de DMF, 0,24 g d'acide sulfurique à 98 % (2,4 mmoles) et 1,2 g de diméthyl sulfate (9,5 mmoles) sont placés dans un réacteur. Le milieu réactionnel est porté à 80 °C et agité pendant 4 heures. Le milieu réactionnel est ensuite ramené à température ambiante, 30 mL d'ammoniaque sont ajoutés, l'agitation est encore maintenue à température ambiante pendant 30 minutes, puis 200 mL d'eau sont ajoutés. Le produit obtenu est extrait au dichlorométhane, purifié par chromatographie sur gel de silice avec comme éluant le système acétate

d'éthyle / triéthylamine (40 / 1) et le système dichlorométhane / éthanol / triéthylamine (20 / 1 / 1). Après évaporation sous vide, le produit est retravaillé dans l'acétone à température ambiante. L'insoluble est filtré puis séché sous vide. 0,5 g de solide bleu-vert est obtenu, soit un rendement de 19,6 %.

Les résultats obtenus par RMN $^1H$ sont les suivants :

$^1H$ (($(CD_3)_2SO$, $\delta$) : 1,81 (6H, s), 3,16 (6H, s), 3,46 (3H, s), 4,22 (2H, t), 4,73 (2H, t), 6,87 (1H, d), 7,41 (2H, m), 7,80 (1H, d), 7,92 (1H, t), 8,09 (2H, m), 8,22 (1H, dd), 8,34 (1H, d), 8,41 (1H, d), 8,69 (1H, d).

L'analyse élémentaire est la suivante :

| | C % | H % | N % |
|---|---|---|---|
| Calculé : | 64,22 | 6,35 | 8,02 |
| Trouvé : | 64,32 | 6,34 | 8,34 |

**Exemples de teinture**

Exemples 1 à 6

Les compositions tinctoriales suivantes sont préparées, i étant un entier compris entre 1 et 6 :

| Constituant | Composition i |
|---|---|
| Colorant i | $3.10^{-3}$ mole % |
| Ethanol | 15 g |
| Alcool benzylique | 5 g |
| Acide benzoïque | 0,2 g |
| Eau distillée | q.s.p. 100 g |

Les colorants sont donnés ci-dessous :

| | |
|---|---|
| 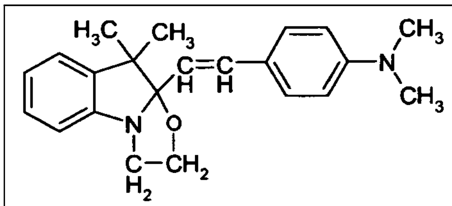 | 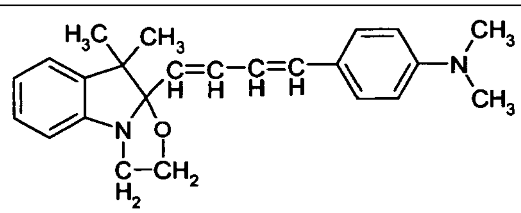 |
| Colorant 1 | Colorant 2 |
| 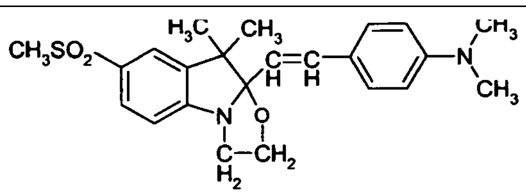 | 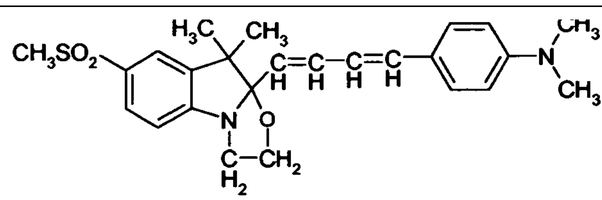 |
| Colorant 3 | Colorant 4 |
| 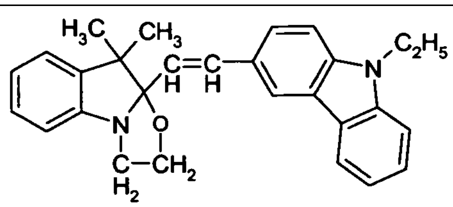 | 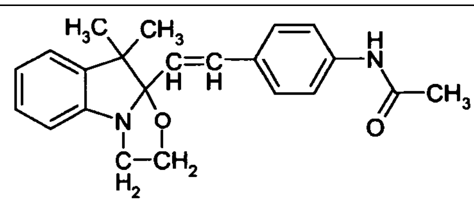 |

(suite)

| | |
|---|---|
| Colorant 5 | Colorant 6 |

Ces compositions sont appliquées sur des mèches de cheveux gris à 90 % de cheveux blancs naturels et permanentés, à raison de 5 g pour 1 g de cheveux, à température ambiante pendant 30 minutes. Les mèches sont ensuite simplement rincées à l'eau claire.

A l'issue de la coloration, la couleur des mèches est mesurée au spectrocolorimètre CM3600d (composantes spéculaires inclues, angle 10°, illuminant D65) dans le système CIEL*a*b*. Dans ce système, L* représente l'intensité de la couleur, a* indique l'axe de couleur vert / rouge et b* l'axe de couleur bleu / jaune.

DEa*b* représente la variation de couleur entre une mèche de cheveux non colorée et une mèche de cheveux colorée et est déterminé à partir de la formule suivante :

$$\Delta E = \sqrt{(L^* - L_o^*)^2 + (a^* - a_o^*)^2 + (b^* - b_o^*)^2}$$

dans laquelle L*, a* et b* représentent les valeurs mesurées sur la mèche colorée et $L_0$*, $a_0$* et $b_0$* représentent les valeurs mesurées sur la mèche non colorée.

Les résultats colorimétriques obtenus sont donnés dans le tableau ci-après.

| Mèche à 90 % de cheveux blancs naturels | | |
|---|---|---|
| | **DEa*b*** | **Couleur** |
| Colorant 1 | 57 | Fushia |
| Colorant 2 | 54 | Bleu |
| Colorant 3 | 65 | Violet |
| Colorant 4 | 41 | Bleu |
| Colorant 5 | 42 | Rouge |
| Colorant 6 | 42 | Jaune |

| Mèche à 90 % de cheveux blancs permanentés | | |
|---|---|---|
| | **DEa*b*** | **Couleur** |
| Colorant 1 | 57 | Fushia |
| Colorant 2 | 57 | Bleu |
| Colorant 3 | 71 | Violet |
| Colorant 4 | 36 | Bleu |
| Colorant 5 | 43 | Rouge |
| Colorant 6 | 47 | Jaune |

Un test de ténacité aux shampooings a été réalisé sur les mèches de cheveux colorés avec les colorants 1 et 2 obtenues dans les conditions décrites ci-dessus. Les mèches colorées ont été soumises à 20 shampooings selon un cycle qui comprend le mouillage des mèches à l'eau, le lavage aux shampooings, un rinçage à l'eau suivi d'un séchage.

La dégradation de la couleur après x shampooings est estimée selon la formule suivante :

$$\%dégradation = 100 * \frac{DE\ xShamp}{DE\ Col}$$

avec :

$$DE\ Col = \sqrt{(a*col - a*tém)^2 + (b*col - b*tém)^2 + (L*col - L*tém)^2}$$

et

$$DE\ xShamp = \sqrt{(a*col - a*xSh)^2 + (b*col - b*xSh)^2 + (L*col - L*xSh)^2}$$

dans lesquelles $a^*_{tém}$, $b^*_{tém}$ et $L^*_{tém}$ sont les valeurs de a*, b* et L* de la mèche non colorée, $a^*_{col}$, $b^*_{col}$ et $L^*_{col}$ les valeurs de a*, b* et L* de la mèche colorée avant shampooing, tandis que $a^*_{xSh}$, $b^*_{xSh}$ et $L^*_{xSh}$ les valeurs de a*, b* et L* de la mèche colorée après x shampooings.

| Mèche à 90 % de cheveux blancs permanentés | | | | |
|---|---|---|---|---|
| | L* | a* | b* | %dégradation |
| Colorant 1 | 23,5 | 34,6 | - 5,0 | - |
| Colorant 1 après 5 shampooings | 24,8 | 40,1 | - 5,5 | 10 |
| Colorant 1 après 12 shampooings | 28,3 | 42,8 | - 4,5 | 17 |
| Colorant 1 après 18 shampooings | 26,6 | 44,0 | - 2,6 | 18 |
| Colorant 1 après 20 shampooings | 28,7 | 47,4 | - 4,3 | 24 |

| Mèche à 90 % de cheveux blancs naturels | | | | |
|---|---|---|---|---|
| | L* | a* | b* | %dégradation |
| Colorant 2 | 18,1 | 11,2 | - 23,2 | - |
| Colorant 2 après 5 shampooings | 18,4 | 9,2 | - 23,6 | 4 |
| Colorant 2 après 12 shampooings | 23,4 | 8,6 | - 28,5 | 14 |
| Colorant 2 après 18 shampooings | 24,4 | 6,3 | - 29,6 | 18 |
| Colorant 2 après 20 shampooings | 25,8 | 6,2 | - 30,5 | 20 |

| Mèche à 90 % de cheveux blancs permanentés | | | | |
|---|---|---|---|---|
| | L* | a* | b* | %dégradation |
| Colorant 2 | 20,0 | 8,8 | - 27,3 | - |
| Colorant 2 après 5 shampooings | 19,3 | 7,9 | - 24,0 | 6 |
| Colorant 2 après 12 shampooings | 19,8 | 7,8 | - 24,1 | 6 |
| Colorant 2 après 18 shampooings | 20,6 | 7,4 | - 23,7 | 7 |
| Colorant 2 après 20 shampooings | 24,8 | 4,2 | - 27,0 | 11 |

Ces résultats montrent qu'après 20 shampooings, les colorants utiles dans le cadre de l'invention présentent une bonne ténacité.

Exemple 7

La composition tinctoriale suivante est préparée :

| Constituant | Composition 7 |
|---|---|
| Colorant | $3.10^{-3}$ mole % |
| Ethanol | 15 g |
| Alcool benzylique | 5 g |
| Acide benzoïque | 0,2 g |
| Eau distillée | q.s.p. 100 g |

Le colorant utilisé est le suivant :

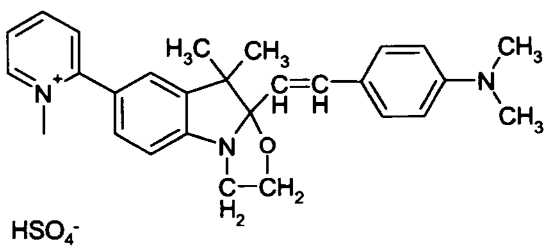

Cette composition est appliquée sur des mèches de cheveux gris à 90 % de cheveux blancs naturels et permanentés, à raison de 5 g pour 1 g de cheveux, à température ambiante pendant 30 minutes. Les mèches sont ensuite simplement rincées à l'eau claire.

A l'issu de la coloration, la couleur des mèches est violette chromatique.

## Revendications

1. Utilisation pour la coloration des fibres kératiniques d'une composition tinctoriale comprenant, dans un milieu approprié pour la teinture, au moins un composé choisi parmi les composés de formule (I), les composés de formule (II) et leurs sels d'addition :

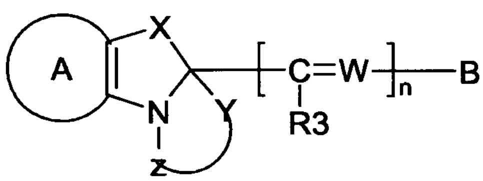

(I)

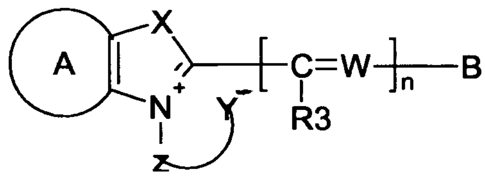

(II)

dans lesquelles :

- A est un noyau aromatique ou hétéroaromatique, condensé ou non, comportant de 5 à 16 chaînons, non substitué ou substitué ;
- X représente un atome d'oxygène, un atome de soufre ou un groupement $CR_1$ $R_2$ ;
- $R_1$ et $R_2$ représentent, indépendamment l'un de l'autre, un atome d'hydrogène, un radical alkyle en $C_1$-$C_6$, un radical hydroxyalkyle en $C_1$-$C_6$, un radical alcoxyalkyle en $C_1$-$C_6$, une chaîne alkylène pouvant contenir un atome d'oxygène ou de soufre ; $R_1$ et $R_2$ peuvent former ensemble un cycle à 5 ou 6 chaînons aromatiques ou non aromatiques, contenant éventuellement un ou plusieurs hétéroatomes tels qu'un atome d'azote, d'oxygène ou de soufre ;
- $R_3$ représente un atome d'hydrogène, un atome d'halogène, un radical alkyle en $C_1$-$C_6$, un radical cyano, un groupement aromatique, un groupement phénoxy, un radical nitro ;
- W représente un groupement $CR_4$ ou un atome d'azote ;
- $R_4$ représente un atome d'hydrogène, un atome d'halogène, un radical alkyle en $C_1$-$C_6$, un radical cyano, un groupement aromatique, un radical phénoxy, un radical nitro ;
- Y représente un atome d'oxygène, un atome de soufre, un groupement $NR_5$ ;
- $R_5$ représente un atome d'hydrogène, un radical alkyle en $C_1$-$C_6$ ;
- Z représente un groupement $-C_pH_{2p}-$, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement $-C_qH_{2q}CO-$, avec q un entier compris entre 1 et 3, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ;
- n représente un entier de 1 à 4 ;
- B représente un noyau aromatique ou hétéroaromatique, condensé ou non, comportant de 5 à 16 chaînons non substitué ou substitué.

**2.** Utilisation selon la revendication 1, dans laquelle A est un noyau benzénique, anthracénique, naphtalénique ou quinolinique.

**3.** Utilisation selon la revendication 1 ou 2, dans laquelle A est non substitué ou substitué par un ou plusieurs groupements choisis parmi un radical halogéno, un radical alkyle en $C_1$-$C_6$, un radical alcoxy en $C_1$-$C_6$, un radical alkylsulfonyle en $C_1$-$C_6$ ($-SO_2$-alkyl), un radical alkylsulfonate en $C_1$-$C_6$ ($-SO_3$-alkyl), un radical cyano, un radical trifluorométhyle, un radical alkylcarbonyle en $C_1$-$C_6$, un radical trifluorométhylsulfonyle ($-SO_2-CF_3$), un radical trifluorométhylcarbonyle, un radical phénylsulfonyle ($-SO_2$-Ph), un radical phénylsulfonate ($-SO_3$-Ph), un radical phénylcarbonyle, un radical nitro, un radical alcoxycarbonyle en $C_1$-$C_6$, un radical phosphonyle ($-PO(OH)_2$), un radical phosphonylalkyle($C_1$-$C_6$) (-alkyl-$PO(OH)_2$), un radical hydroxyle, un radical amino, un radical dialkyl($C_1$-$C_6$)amino, un radical (hydroxyalkyl($C_1$-$C_6$))amino, un radical di(hydroxyalkyl($C_1$-$C_6$))amino, un radical (aminoalkyl($C_1$-$C_6$))amino, un radical di(aminoalkyl($C_1$-$C_6$))amino, un radical (hydroxyalkyl($C_1$-$C_{-6}$))(alkyl($C_1$-$C_6$))amino, un radical (aminoalkyl($C_1$-$C_6$))(alkyl($C_1$-$C_6$))amino, un radical (aminoalkyl($C_1$-$C_6$))(hydroxyalkyl($C_1$-$C_6$)amino, un radical hydroxyalkyle($C_1$-$C_6$), un radical aminoalkyle($C_1$-$C_6$), un radical (alkyl($C_1$-$C_6$))aminoalkyle($C_1$-$C_6$), un radical di(alkyl ($C_1$-$C_6$))aminoalkyle($C_1$-$C_6$), un radical (hydroxyalkyl($C_1$-$C_6$))aminoalkyle($C_1$-$C_6$), un radical di(hydroxyalkyl ($C_1$-$C_6$))aminoalkyle($C_1$-$C_6$), un radical (aminoalkyl($C_1$-$C_6$))aminoalkyle($C_1$-$C_6$), un radical di(aminoalkyl($C_1$-$C_6$)) aminoalkyle($C_1$-$C_6$), un radical (alkyl($C_1$-$C_6$))(hydoxyalkyl($C_1$-$C_6$))aminoalkyle($C_1$-$C_6$), un radical (aminoalkyl ($C_1$-$C_6$))(alkyl($C_1$-$C_6$))amino, un radical (hydroxyalkyl($C_1$-$C_6$))(alkyl($C_1$-$C_6$))aminoalkyle($C_1$-$C_6$), un radical phénylalkyle($C_1$-$C_6$) éventuellement substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un groupement cationique du type ammonium quaternaire, un radical alkyle en $C_1$-$C_6$ substitué par un groupement

cationique du type ammonium quaternaire, un radical carboxyle, un radical alkyle($C_1$-$C_6$) substitué par un radical carboxyle, un radical thio, un radical thioalkyle($C_1$-$C_6$), un radical sulfonate ($-SO_3-$), un radical alkyle($C_1$-$C_6$) substitué par un radical sulfonate, un radical alkyl($C_1$-$C_6$)carbonylalkyle($C_1$-$C_6$), un radical di(halogénoalkyl($C_1$-$C_6$))amino, un radical acétamido, un radical aryloxy, un radical aryloxyalkyle($C_1$-$C_6$), un radical éthényle ($-CH=CH_2$), un radical éthénylcarbonyle ($-CO-CH=CH_2$) ; deux groupements adjacents pouvant former un cycle aromatique ou hétéroaromatique, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, ou un cycle de type $-O-C_mH_{2m}-O-$ où m est un entier égal à 1 ou 2.

**4.** Utilisation selon la revendication 3, dans laquelle A est non substitué ou substitué par un ou plusieurs groupements choisis parmi un radical alkyl($C_1$-$C_6$)sulfonyle ; un groupement pyridinium ou imidazolium non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle; un groupement trialkyl($C_1$-$C_6$)ammonium ; un radical sulfonate.

**5.** Utilisation selon l'une quelconque des revendications précédentes, dans laquelle X est choisi parmi un groupement $CR_1R_2$.

**6.** Utilisation selon l'une quelconque des revendications précédentes, dans laquelle $R_1$ et $R_2$ sont choisis parmi un radical alkyle en $C_1$-$C_6$.

**7.** Utilisation selon l'une quelconque des revendications précédentes, dans laquelle $R_3$ est choisi parmi un atome d'hydrogène.

**8.** Utilisation selon l'une quelconque des revendications précédentes, dans laquelle W est choisi parmi un groupement $CR_4$.

**9.** Utilisation selon l'une quelconque des revendications précédentes, dans laquelle $R_4$ est choisi parmi un atome d'hydrogène.

**10.** Utilisation selon l'une quelconque des revendications précédentes, dans laquelle Y est choisi parmi un atome d'oxygène.

**11.** Utilisation selon l'une quelconque des revendications précédentes, dans laquelle Z est choisi parmi un groupement $-C_pH_{2p}-$, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle.

**12.** Utilisation selon l'une quelconque des revendications précédentes, dans laquelle n est égal à 1 ou 2.

**13.** Utilisation selon l'une quelconque des revendications précédentes, dans laquelle B est un noyau benzénique, carbazolique ou indolique.

**14.** Utilisation selon l'une quelconque des revendications précédentes, dans laquelle B est non substitué ou substitué par un ou plusieurs groupements choisis parmi un radical halogéno, un radical alkyle en $C_1$-$C_6$, un radical alcoxy en $C_1$-$C_6$, un radical cyano, un radical trifluorométhyle, un radical alkylcarbonyle en $C_1$-$C_6$, un radical trifluorométhylsulfonyle, un radical trifluorométhylcarbonyle, un radical phénylsulfonyle, un radical phénylcarbonyle, un radical phényle non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un radical acylamino, un radical hydroxyle, un radical amino, un radical di(alkyl($C_1$-$C_6$))amino, un radical hydroxyalkyl($C_1$-$C_6$) amino, un radical di(hydroxyalkyl($C_1$-$C_6$))amino, un radical (aminoalkyl($C_1$-$C_6$))amino, un radical di(aminoalkyl ($C_1$-$C_6$))amino, un radical (alkyl($C_1$-$C_6$))(hydroxyalkyl($C_1$-$C_{-6}$))amino, un radical (aminoalkyl($C_1$-$C_6$))(alkyl($C_1$-$C_{-6}$)) amino, un radical (aminoalkyl($C_1$-$C_6$))(hydroxyalkyl($C_1$-$C_6$))amino, un radical hydroxyalkyle($C_1$-$C_6$), un radical aminoalkyle($C_1$-$C_6$), un radical alkyl($C_1$-$C_6$)aminoalkyle($C_1$-$C_6$), un radical di(alkyl($C_1$-$C_6$))aminoalkyle($C_1$-$C_6$), un radical (hydroxyalkyl($C_1$-$C_6$))aminoalkyle($C_1$-$C_6$), un radical di(hydroxyalkyl($C_1$-$C_6$))aminoalkyle($C_1$-$C_6$), un radical aminoalkyl($C_1$-$C_6$)aminoalkyle($C_1$-$C_6$), un radical di(aminoalkyl($C_1$-$C_6$))aminoalkyle($C_1$-$C_6$), un radical (alkyl ($C_1$-$C_6$))(hydroxyalkyl($C_1$-$C_6$))aminoalkyle($C_1$-$C_6$), un radical (alkyl($C_1$-$C_6$))(aminoalkyl($C_1$-$C_6$))amino, un radical (hydroxyalkyl($C_1$-$C_6$))(alkyl($C_1$-$C_6$))aminoalkyle($C_1$-$C_6$), un radical phénylalkyle($C_1$-$C_6$) non substitué ou substitué

par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un groupement cationique du type ammonium quaternaire, un radical alkyle($C_1$-$C_6$) substitué par un groupement cationique du type ammonium quaternaire, un radical carboxyle, un radical alkyle($C_1$-$C_6$) substitué par un radical carboxyle, un radical thio, un radical thioalkyle ($C_1$-$C_6$), un radical sulfonate, un radical alkyle($C_1$-$C_6$) substitué par un radical sulfonate, un radical alkyl($C_1$-$C_6$) carbonylalkyle($C_1$-$C_6$), un radical di(halogénoalkyl($C_1$-$C_6$))amino, un radical acétamido, un radical aryloxy, un radical aryloxyalkyle($C_1$-$C_6$), un radical éthényle, un radical éthénylcarbonyle, un groupement $NR_6R_7$, $R_6$ et $R_7$ pouvant former ensemble avec l'atome d'azote auquel ils sont attachés un cycle en $C_5$, $C_6$ ou $C_7$ non aromatique, éventuellement interrompu par un ou plusieurs hétéroatomes tels qu'un atome d'azote, d'oxygène ou de soufre, une chaîne alkylène pouvant contenir un atome d'oxygène ou de soufre et pouvant être terminée par un groupement cyano, alkylsulfonyle en $C_1$-$C_6$ ou alkylcarbonyle en $C_1$-$C_6$; deux groupements adjacents de B pouvant former un cycle aromatique ou héteroaromatique, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, ou un cycle de type -O-$C_rH_{2r}$-O- où r représente un entier égal à 1 ou 2.

**15.** Utilisation selon la revendication 14, dans laquelle B est non substitué ou substitué par un ou plusieurs groupements choisis parmi un radical hydroxyle ; un radical amino ; un radical di(alkyl($C_1$-$C_6$))amino ; un radical alkyle en $C_1$-$C_6$ ; un radical acétamido ; un groupement pyridinium ; un groupement trialkyl($C_1$-$C_6$)ammonium.

**16.** Utilisation selon l'une quelconque des revendications précédentes, dans laquelle le ou les composés de formule (I) sont choisis parmi l'acide 9a-[2-[4-(diméthylamino)phényl]-1,3-butadiényl]-2,3,9,9a-tétrahydro-9,9-diméthyl oxazolo[3,2-a]indole-7-carboxylique ; l'acide [9a-[2-[4-(diméthylamino)phényl]éthènyl]-2,3,9,9a-tétrahydro-9,9-diméthyloxazolo[3,2-a]indol-7-yl] phosphonique ; la 4-[2-(9,9-diéthyl-2,3-dihydro-7-méthoxyoxazolo[3,2-a]indol-9a (9H)-yl)éthènyl]-N,N-diéthyl benzenamine ; l'acide [3-[9a-[2-[4-(diméthylamino)phényl]éthènyl]-2,3,9,9a-tétrahydro-9,9-diméthyloxazolo[3,2-a]indol-7-yl]propyl] phosphonique ; la 4-[2-(2,3-dihydro-9,9-diméthyloxazolo[3,2-a] indol-9a(9H)-yl)éthènyl]-N-méthyl-N-phényl benzènamine ; la 4-[2-(2,3-dihydro-9,9-diméthyloxazolo[3,2-a]indol-9a (9H)-yl)éthènyl]-3-éthoxy-N,N-diéthyl benzènamine ; la 4-[2-(2,3-dihydro-9,9-diméthyloxazolo[3,2-a]indol-9a (9H)-yl)éthènyl]-N-éthyl-N-(2-méthylpropyl) benzènamine ; la 4-[2-(2,3-dihydrooxazolo[3,2-a]indol-9a(9H)-yl)éthènyl]-N,N-diméthyl benzènamine ; la 4-[4-(2,3-dihydro-7,9,9-triméthyloxazolo[3,2-a]indol-9a(9H)-yl)-1,3-butadiènyl]-N,N-diméthyl benzènamine ; la 4-[4-(2,3-dihydro-9,9-diméthyl-7-nitrooxazolo[3,2-a]indol-9a(9H)-yl)-1,3-butadiènyl]-N,N-diméthyl benzenamine ; le 9a-[4-[4-(diméthylamino)phényl]-1,3-butadiènyl]-2,3,9,9a-tétrahydro-9,9-diméthyl oxazolo[3,2-a]indole-7-carbonitrile ; le 9a-[2-[4-(diméthylamino)phényl]éthènyl]-2,3,9,9a-tétrahydro-9,9-diméthyl oxazolo[3,2-a]indole-7-carbonitrile ; la 4-[2-(2,3-dihydro-9,9-diméthyloxazolo[3,2-a]indol-9a(9H)-yl)éthènyl]-N,N-diméthyl benzenamine ; l'acide 9a-[2-[4-(diméthylamino)phényl]éthènyl]-2,3,9,9a-tétrahydro-9,9-diméthyl-méthylester oxazolo[3,2-a]indole-7-sulfonique ; la N,N-bis(2-chloroéthyl)-4-[2-(2,3-dihydro-9,9-diméthyloxazolo [3,2-a]indol-9a(9H)- yl)éthènyl] benzènamine ; la 9a-[2-[4-(diméthylamino)phényl]éthènyl]-9,9a-dihydro-9,9-diméthyl-7-(octylsulfonyl) oxazolo[3,2-a]indol-2(3H)-one ; la 9a-[2-[4-(diméthylamino)phényl]éthènyl]-9,9a-dihydro-9,9-diméthyl-7-(phénylsulfonyl) oxazolo[3,2-a]indol-2(3H)-one ; la 9a-[2-[4-(diméthylamino)phényl]-1-méthyléthènyl]-9,9a-dihydro-9,9-diméthyl-7-(méthylsulfonyl) oxazolo[3,2-a]indol-2(3H)-one ; la 9a-[2-[4-(diméthylamino)phényl] éthènyl]-9,9a-dihydro-9,9-diméthyl-7-(méthylsulfonyl) oxazolo[3,2-a]indol-2(3H)-one ; la 4-[2-[2,3-dihydro-9,9-diméthyl-7-(méthylsulfonyl)oxazolo[3,2-a]indol-9a(9H)-yl]éthènyl]-N,N-diméthyl benzènamine ; la 4-[2-[9-(éthoxyméthyl)-2,3-dihydro-9-méthyl-7-(méthylsulfonyl)oxazolo[3,2-a]indol-9a(9H)-yl]éthènyl]-N,N-diméthyl benzenamine ; la 4-[2-[2,3- dihydro- 2,9,9- triméthyl- 7-(méthylsulfonyl) oxazolo [3,2- a] indol- 9a (9H)-yl] éthènyl]-N, N- diméthyl benzènamine ; la 4-[2-[2,3-dihydro-9,9-diméthyl-7-(méthylsulfonyl)oxazolo[3,2-a]indol-9a(9H)-yl]-1-propényl]-N,N-diméthyl benzènamine ; la N,N-dibutyl-4-[2-[2,3-dihydro-9,9-diméthyl-7-(méthylsulfonyl)oxazolo[3,2-a]indol-9a (9H)-yl]éthènyl] benzènamine ; 4-[2-[2,3-dihydro-9,9-diméthyl-7-(phénylsulfonyl)oxazolo[3,2-a]indol-9a(9H)-yl] éthènyl]-N,N-diméthyle ; la 4-[2-[2, 3-dihydro-9,9-diméthyl-7-(octylsulfonyl)oxazolo[3,2-a]indol-9a(9H)-yl]éthènyl]-N,N-diméthyl benzènamine ; le N-[4-[2-[7-(butylsulfonyl)-2,3-dihydro-9,9-diméthyloxazolo[3,2-a]indol-9a (9H)-yl]éthènyl]phényl] acétamide ; la 4- [2-[7-(butylsulfonyl)-2,3-dihydro-9,9-diméthyloxazolo[3,2-a]indol-9a (9H)-yl]éthènyl]-N,N-diméthyl benzènamine ; la 4-[4-[2,3-dihydro-9,9-diméthyl-7-(méthylsulfonyl)oxazolo[3,2-a]indol-9a(9H)-yl]-1,3-butadiènyl]-N,N-diméthyl benzènamine ; le 9a-[2-[4-(diméthylamino)phényl]éthènyl]-2,3,9,9a-tétrahydro-9-méthyl oxazolo[3,2-a]indole-9-éthanol ; la 4-[2-(9,9-diéthyl-2,3-dihydrooxazolo[3,2-a]indol-9a(9H)-yl) éthènyl]-N,N-diméthyl benzènamine ; la 4-[2-[2,3-dihydro-9-méthyl-9-(2-phénoxyéthyl)oxazolo[3,2-a]indol-9a (9H)-yl]éthènyl]-N,N-diméthyl benzènamine ; la 4-[2-[9-(éthoxyméthyl)-2,3-dihydro-9-méthyloxazolo[3,2-a]indol-9a (9H)-yl]éthènyl]-N,N-diméthyl benzènamine ; la 4-[2-(11,11-diméthylbenz[e]oxazolo[3,2-a]indol-10a(11H)-yl)éthènyl]-N,N-diméthyl benzenamine ; la 4-[2-(7-méthoxy-9,9-diméthyloxazolo[3,2-a]indol-9a(9H)-yl)éthènyl]-N,N-diméthyl benzènamine ; la 4-[2-(9,9-diméthyloxazolo[3,2-a]indol-9a(9H)-yl)éthènyl]-N,N-diméthyl benzènamine ; la 9a-[2-[4-(diméthylamino)phényl]éthènyl]-3-éthyl-9,9a-dihydro-9,9-diméthyl oxazolo[3,2-a]indol-2(3H)-one; la 7-chloro-9a-[2-[4-(diméthylamino)phényl]éthènyl]-9,9a-dihydro-3,9,9-triméthyl oxazolo[3,2-a]indol-2(3H)-one ; la 9a-[2-[4-(dibutylamino)phényl]éthènyl]-9,9a-dihydro-9,9-diméthyl oxazolo[3,2-a]indol-2(3H)-one ; la 9a-[2-[4-(diméthylamino)phényl]éthènyl]-9,9a-dihydro-9-(2-hydroxyéthyl)-9-méthyl oxazolo[3,2-a]indol-2(3H)-one ; la 9a-[2-[4-(diméthylamino)phényl]-1-propényl]-9,9a-dihydro-9,9-diméthyl oxazolo[3,2-a]indol-2(3H)-one ; la 9,9a-dihydro-7,9,9-triméthyl-9a-[2-(4-nitrophényl)éthènyl] oxazolo[3,2-a]indol-2(3H)-one ; le N-[4-[2-(2,3-dihydro-9,9-diméthyl-2-oxooxazolo[3,2-a]indol-9a(9H)-yl)éthènyl]phényl] acetamide ; la 9a-[2-[4-(diméthylamino)phényl]éthènyl]-9,9a-dihydro-6-méthoxy-9,9-diméthyl oxazolo[3,2-a]indol-2(3H)-one ; l'acide 9a-[2-[4-(diméthylamino)phényl]éthènyl]-2,3,9,9a-tétrahydro-9,9-diméthyl-2-oxo-éthyl ester oxazolo[3,2-a]indole-7-carboxylique; la 9a-[2-[4-(diméthylamino)phényl]éthènyl]-9,9a-dihydro-7,9,9-triméthyl oxazolo[3,2-a]indol-2(3H)-one ; la 10a-[2-[4-(diméthylamino)phényl]éthènyl]-10a,11-dihydro-11,11-diméthyl benz[e]oxazolo[3,2-a]indol-9(8H)-one ; la 9,9a-dihydro-9,9-diméthyl-9a-[2-(4-nitrophényl)éthènyl] oxazolo[3,2-a]indol-2(3H)-one ; la 7-chloro-9a-[2-[4-(diméthylamino)phényl]éthènyl]-9,9a-dihydro-9,9-diméthyl oxazolo[3,2-a]indol-2(3H)-one ; la 9a-[2-[4-(diméthylamino)phényl]éthènyl]-9,9a-dihydro-9,9-diméthyl oxazolo[3,2-a]indol-2(3H)-one ; la 4-[2-(7-chloro-2,3-dihydro-9,9-diméthyloxazolo[3,2-a]indol-9a(9H)-yl)éthènyl]-N,N-diméthyl benzènamine ; la 4-[2-(2,3-dihydro-9,9-diméthyloxazolo[3,2-a]indol-9a(9H)-yl)éthènyl]-N,N-diméthyl benzènamine ; la 4-[2-(7-chloro-2,3-dihydro-2,9,9-triméthyloxazolo[3,2-a]indol-9a(9H)-yl)éthènyl]-N,N-diméthyl benzènamine ; la 4-[2-(2,3-dihydro-9,9-diméthyloxazolo[3,2-a]indol-9a(9H)-yl)-1-méthyléthènyl]-N,N-diméthyl benzènamine ; la 4-[2-(2,3-dihydro-9,9-diméthyloxazolo[3,2-a]indol-9a(9H)-yl)éthènyl]-N,N-diéthyl benzènamine ; le 2,3,9,9a-tétrahydro-7-méthoxy-9,9-diméthyl-9a-[2-(4-nitrophényl)éthènyl] oxazolo[3,2-a]indole ; la 4-[2-(2,3-dihydro-9,9-diméthyl-7-nitrooxazolo[3,2-a]indol-9a(9H)-yl)éthènyl]-N,N-diméthyl benzènamine ; la N,N-dibutyl-4-[2-(2,3-dihydro-9,9-diméthyloxazolo[3,2-a]indol-9a(9H)-yl)éthènyl] benzènamine ; le 2,3,9,9a-tétrahydro-9,9-diméthyl-7-nitro-9a-[2-(4-nitrophényl)éthènyl] oxazolo[3,2-a]indole ; le 7-chloro-2,3,9,9a-tétrahydro-9,9-diméthyl-9a-[2-(4-nitrophényl)éthènyl] oxazolo[3,2-a]indole ; la 4-[2-(2,3-dihydro-7-iodo-9,9-diméthyloxazolo[3,2-a]indol-9a(9H)-yl)éthènyl]-N,N-diméthyl benzènamine ; la 4-[2-(2,3-dihydro-5-méthoxy-9,9-diméthyloxazolo[3,2-a]indol-9a(9H)-yl)éthènyl]-N,N-diméthyl benzènamine ; la 4-[2-(2,3-dihydro-7-méthoxy-9,9-diméthyloxazolo[3,2-a]indol-9a(9H)-yl)éthènyl]-N,N-diméthyl benzènamine ; la 4-[4-(2,3-dihydro-9,9-diméthyloxazolo[3,2-a]indol-9a(9H)-yl)-1,3-butadiènyl]-N,N-diéthyl benzènamine ; la 4-[4-(7-chloro-2,3-dihydro-9,9-diméthyloxazolo[3,2-a]indol-9a(9H)-yl)-1,3-butadiènyl]-N,N-diméthyl benzènamine ; la 4-[4- (2,3-dihydro-9,9-diméthyloxazolo[3,2-a]indol-9a(9H)-yl)-1,3-butadiènyl]-N,N-diméthyl benzènamine ; la 4-[4-(2,3-dihydro-7-méthoxy-9,9-diméthyloxazolo[3,2-a]indol-9a(9H)-yl)-1,3-butadiènyl]-N,N-diméthyl benzènamine ; le 2,3,9,9a-tétrahydro-7,9,9-triméthyl-9a-[2-(4-nitrophényl)éthènyl] oxazolo[3,2-a]indole ; le N-[4-[2-(2,3-dihydro-9,9-diméthyloxazolo[3,2-a]indol-9a(9H)-yl)éthènyl]phényl] acétamide ; la 4-[2-(2,3-dihydro-6-méthoxy-9,9-diméthyloxazolo[3,2-a]indol-9a(9H)-yl)éthènyl]-N,N-diméthyl benzènamine ; la 4-[2-(8,9-dihydro-11,11-diméthylbenz[e]oxazolo[3,2-a]indol-10a(11H)-yl)éthènyl]-N,N-diméthyl benzènamine ; la 4-[2-(2,3-dihydro-7,9,9-triméthyloxazolo[3,2-a]indol-9a(9H)-yl)éthènyl]-N,N-diméthyl benzènamine ; le 2,3,9,9a-tétrahydro-9,9-diméthyl-9a-[2-(4-nitrophényl)éthènyl] oxazolo[3,2-a]indole ; la 9,9a-dihydro-9,9-diméthyl-9a-[2-(9-méthyl-9H-carbazol-3-yl)éthènyl]-7-(méthylsulfonyl) oxazolo[3,2-a]indol-2(3H)-one ; le 9a-[2-(9-hexyl-9H-carbazol-3-yl)éthènyl]-2,3,9,9a-tétrahydro-9,9-diméthyl-7-(phénylsulfonyl) oxazolo[3,2-a]indole ; le 2,3,9,9a-tétrahydro-9,9-diméthyl-7-(méthylsulfonyl)-9a-[2-(9-octyl-9H-carbazol-3-yl)éthènyl] oxazolo[3,2-a]indole ; l'acide 9a-[2-(9-butyl-6-méthoxy-9H-carbazol-3-yl)éthènyl]-2,3,9,9a-tétrahydro-9,9-diméthyl-, éthyl ester oxazolo[3,2-a]indole-7-carboxylique ; l'acide 9a-[2-(9-éthyl-9H-carbazol-3-yl)éthènyl]-2,3,9,9a-tétrahydro-9,9-diméthyl-, méthyl ester oxazolo[3,2-a]indole-7-sulfonique ; le 3-chloro-6-[2-[2,3-dihydro-9,9-diméthyl-7-(méthylsulfonyl)oxazolo[3,2-a]indol-9a(9H)-yl]éthènyl]-9-octyl 9H-carbazole ; le 3-[2-[2,3-dihydro-9,9-diméthyl-7-(méthylsulfonyl)oxazolo[3,2-a]indol-9a(9H)-yl]éthènyl]-9-méthyl 9H-carbazole; le 3-[2-[9-(2- éthoxyéthyl)-2,3-dihydro-9-méthyl-7-(méthylsulfonyl)oxazolo[3,2-a]indol-9a(9H)-yl]éthènyl] 9H-carbazole ; l'acide 9a-[2-(9-hexyl-9H-carbazol-3-yl)éthènyl]-2,3,9,9a-tétrahydro-9-(2-hydroxyéthyl)-9-méthyl-, éthyl ester oxazolo[3,2-a]indole-7-carboxylique ; l'acide 2,3,9,9a-tétrahydro-9,9-diméthyl-9a-[2-(9-octyl-9H-carbazol-3-yl)éthènyl]-, éthyl ester oxazolo[3,2-a]indole-7-carboxylique ; l'acide 9a-[2-(9-butyl-9H-carbazol-3-yl)éthènyl]-2,3,9,9a-tétrahydro-9,9-diméthyl-, éthyl ester oxazolo[3,2-a]indole-7-carboxylique ; l'acide 2,3,9,9a-tétrahydro-9,9-diméthyl-9a-[2-(9-méthyl-9H-carbazol-3-yl)éthènyl]-, éthyl ester oxazolo[3,2-a]indole-7-carboxylique ; la 9a-[2-(9-butyl-6-éthoxy-9H-carbazol-3-yl)éthènyl]-2,3,9,9a-tétrahydro-N,N,9,9-tétraméthyl oxazolo[3,2-a]indol-7-amine ; le 2,3,9,9a-tétrahydro-9-méthyl-9a-[2-(9-méthyl-9H-carbazol-3-yl)éthènyl] oxazolo[3,2-a]indole-9-éthanol ; la 9a-[2-(9-butyl-9H-carbazol-3-yl)éthènyl]-2,3,9,9a-tétrahydro-N,N,9,9-tétraméthyl oxazolo[3,2-a]indol-7-amine ; le 3-[2-(2,3-dihydro-6-méthoxy-9,9-diméthyloxazolo[3,2-a]indol-9a(9H)-yl)éthènyl]-9-méthyl 9H-carbazole ; le 3-[2-(2,3-dihydro-9,9-diméthyloxazolo[3,2-a]indol-9a(9H)-yl)-1-propényl]-9-méthyl 9H-carbazole; le 3-[2-(7-chloro-2,3-dihydro-9,9-diméthyloxazolo[3,2-a]indol-9a(9H)-yl)éthènyl]-9-méthyl 9H-carbazole ; le 3-bromo-6-[2-(2,3-dihydro-9,9-diméthyloxazolo[3,2-a]indol-9a(9H)-yl)éthènyl]-9-éthyl 9H-carbazole ; le 3-[2-(2,3-dihydro-9,9-diméthyloxazolo[3,2-a]indol-9a(9H)-yl)éthènyl]-9-méthyl 9H-carbazole.

**17.** Utilisation selon la revendication 16, dans laquelle le ou les composés de formule (I) sont choisis parmi les composés

présentés dans le tableau ci-dessous :

| | |
|---|---|
| 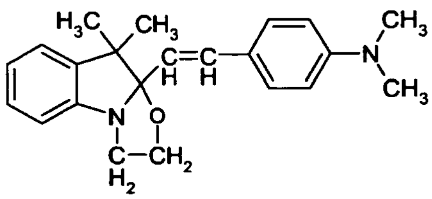 | Colorant 1 |
| 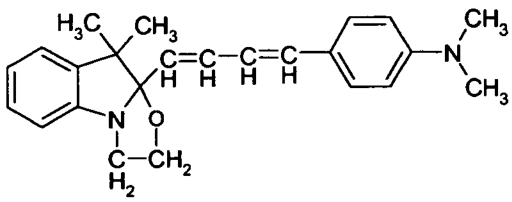 | Colorant 2 |
| 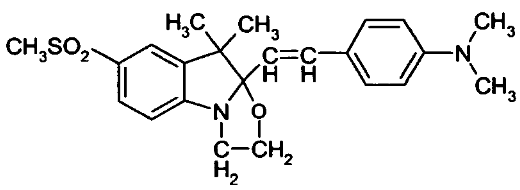 | Colorant 3 |
| 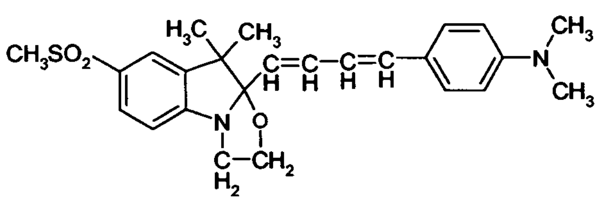 | Colorant 4 |
| 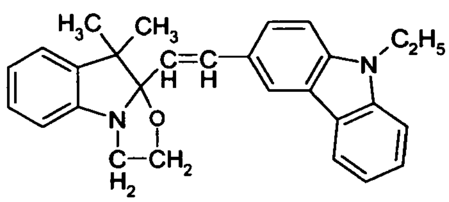 | Colorant 5 |
| 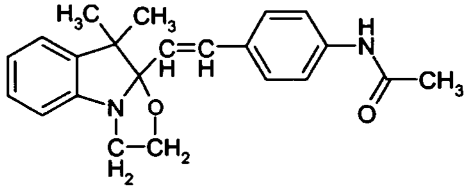 | Colorant 6 |

(suite)

| | |
|---|---|
| 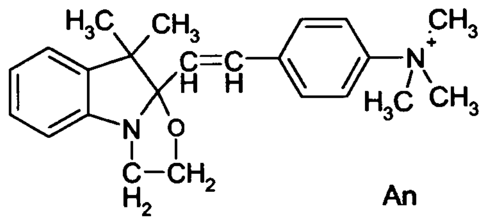 | Colorant 7 |
| 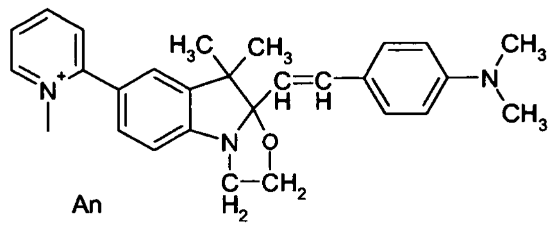 | Colorant 8 |
| 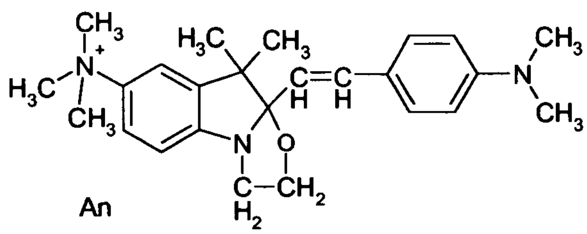 | Colorant 9 |
| 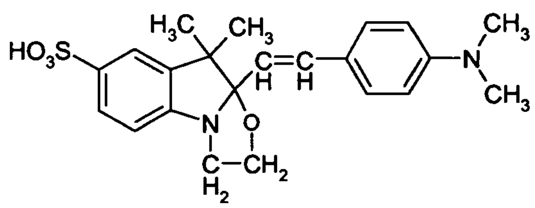 | Colorant 10 |
| 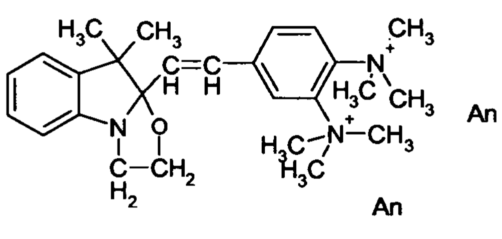 | Colorant 11 |

dans lesquels An, identiques ou différents, représentent un contre-ion négatif.

**18.** Utilisation selon l'une quelconque des revendications précédentes, dans laquelle le ou composés choisis parmi les composés de formule (I), les composés de formule (II) et leurs sels d'addition représentent de 0,0001 à 10 % en poids par rapport au poids total de la composition.

**19.** Utilisation selon l'une quelconque des revendications précédentes, dans laquelle la composition tinctoriale comprend

au moins un agent acidifiant et / ou au moins un agent alcalinisant.

**20.** Utilisation selon la revendication 19, dans laquelle le ou les agents acidifiants sont choisis parmi les acides minéraux et les acides organiques.

**21.** Utilisation selon la revendication 18 ou 19, dans laquelle le ou les agents alcalinisants sont choisis parmi :

- les acides aminés basiques ;
- les carbonates ou les bicarbonates de métaux alcalins ou alcalino-terreux ;
- les silicates ou les métasilicates ;
- les composés de formule (III) suivante :

$$X(OH)_n \qquad (III)$$

dans laquelle :

• X représente un ion potassium, lithium, sodium, ammonium $N^+R_8R_9R_{10}R_{11}$ avec $R_8$, $R_9$, $R_{10}$ et $R_{11}$, identiques ou différents, désignant un radical alkyle en $C_2$-$C_4$ lorsque n est égal à 1 ;
• X représente un atome de magnésium ou de calcium lorsque n est égal à 2 ;

- les composés de formule (IV) suivante :

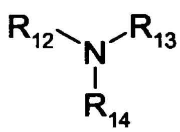

(IV)

dans laquelle :

• $R_{12}$ représente un atome d'hydrogène ; un radical alkyle en $C_1$-$C_6$ ; un radical monohydroxyalkyle en $C_1$-$C_6$ ; un radical polyhydroxyalkyle en $C_2$-$C_6$ ;
• $R_{13}$ et $R_{14}$, identiques ou différents, représentent un atome d'hydrogène ; un radical alkyle en $C_1$-$C_6$ ; un radical monohydroxyalkyle en $C_1$-$C_6$; un radical polyhydroxyalkyle en $C_2$-$C_6$ ;

- les composés de formule (V) suivante :

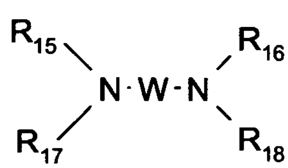

(V)

dans laquelle :

• W est un reste propylène éventuellement substitué par un groupement hydroxyle ou un radical alkyle en $C_1$-$C_4$ ;
• $R_{15}$, $R_{16}$, $R_{17}$ et $R_{18}$, identiques ou différents, représentent un atome d'hydrogène ; un radical alkyle en $C_1$-$C_4$ ou hydroxyalkyle en $C_1$-$C_4$.

**22.** Utilisation selon l'une quelconque des revendications précédentes, dans laquelle le milieu approprié pour la teinture est constitué par de l'eau ou par au moins un solvant organique ou par un mélange d'eau et d'au moins un solvant organique.

**23.** Utilisation selon la revendication 22, dans laquelle le ou les solvants sont choisis parmi les cétones ; les monoalcools ou les diols, linéaires ou ramifiés, comprenant 2 à 10 atomes de carbone ; les alcools aromatiques ; les polyols ou éthers de polyol ; ainsi que les alkyléthers de diéthylèneglycol.

**24.** Utilisation selon la revendication 22 ou 23, dans laquelle le milieu approprié pour la teinture est constitué par de l'eau ou par un mélange d'eau et d'au moins un solvant organique et le pH est compris entre 2 et 12.

**25.** Utilisation selon l'une quelconque des revendications précédentes, dans laquelle la composition tinctoriale comprend au moins un additif choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, les agents antioxydants, les agents de pénétration, les agents séquestrants, les parfums, les tampons, les agents dispersants, les agents de conditionnement, les agents conservateurs, les agents opacifiants.

**26.** Procédé de traitement des fibres kératiniques, dans lequel une composition tinctoriale telle que définie à l'une quelconque des revendications 1 à 25 est appliquée sur les fibres kératiniques pendant un temps de pose suffisant, cette application étant éventuellement suivie d'un rinçage.

**27.** Procédé selon la revendication 26, dans lequel l'application de la composition tinctoriale est suivie d'un pré- ou post-traitement des fibres kératiniques permettant la révélation de la coloration.

**28.** Procédé selon la revendication 27, dans lequel le pré- ou post-traitement consiste à traiter les fibres par action de la lumière, un courant électrique, la chaleur, un agent acidifiant tel que défini à la revendication 19 ou 20 et / ou un agent alcalinisant tel que défini à la revendication 19 ou 21 et / ou un solvant tel que défini à la revendication 22 ou 23, une radication électromagnétique.

**29.** Procédé selon l'une quelconque des revendications 26 à 28, dans lequel la composition tinctoriale est appliquée en présence d'au moins un agent oxydant.

**30.** Procédé selon la revendication 29, dans lequel l'agent oxydant est choisi parmi le peroxyde d'hydrogène, le peroxyde d'urée, les bromates de métaux alcalins, les persels, les peracides et les enzymes de type oxydase.

**31.** Dispositif à plusieurs compartiments contenant dans un premier compartiment une composition tinctoriale comprenant au moins un composé tel que défini à l'une quelconque des revendications 1 à 17 et dans un deuxième compartiment au moins un agent acidifiant tel que défini à la revendication 19 ou 20 et / ou au moins un agent alcalinisant tel que défini à la revendication 19 ou 21 et / ou au moins un solvant tel que défini à la revendication 22 ou 23.

**32.** Dispositif selon la revendication 31, contenant dans un troisième compartiment au moins un agent oxydant tel que défini à la revendication 29 ou 30.

**33.** Composés tels que définis à l'une quelconque des revendications 1 à 17, comportant au moins un groupement choisi parmi un groupement cationique du type ammonium quaternaire.

**34.** Composés selon la revendication 33 dans lesquels le ou les groupements cationiques du type ammonium quaternaire sont choisis parmi les groupements trialkyl($C_1$-$C_6$)ammonium, oxazolium, thiazolium, imidazolium, pyrazolium, pyridinium, pyrrolium, triazolium, isooxazolium, isothiazolium, pyrimidinium, pyrazinium, triazinium, pyridazinium, indolium, quinolinium ou isoquinolimiun, substitué ou non substitué.

**35.** Composition tinctoriale comprenant, dans un milieu approprié pour la teinture, au moins un composé tel que défini à la revendication 33 ou 34.

## Claims

**1.** Use, for dyeing keratin fibres, of a dye composition comprising, in a suitable dyeing medium, at least one compound chosen from the compounds of formula (I) and the compounds of formula (II), and the addition salts thereof:

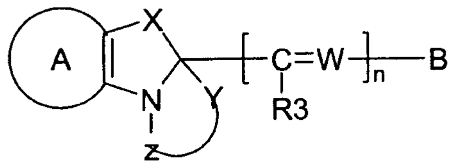

(I)

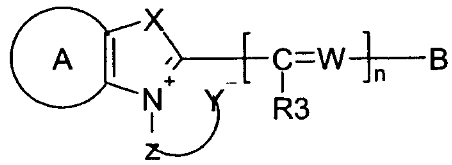

(II)

in which:

- A is a substituted or unsubstituted, 5- to 16- membered, fused or non-fused aromatic or heteroaromatic nucleus;
- X represents an oxygen atom, a sulfur atom or a group $CR_1R_2$;
- $R_1$ and $R_2$ represent, independently of each other, a hydrogen atom, a $C_1$-$C_6$ alkyl radical, a $C_1$-$C_6$ hydroxyalkyl radical, a $C_1$-$C_6$ alkoxyalkyl radical or an alkylene chain that may contain an oxygen or sulfur atom; $R_1$ and $R_2$ may together form an aromatic or non-aromatic 5- or 6-membered ring optionally containing one or more heteroatoms such as a nitrogen, oxygen or sulfur atom;
- $R_3$ represents a hydrogen atom, a halogen atom, a $C_1$-$C_6$ alkyl radical, a cyano radical, an aromatic group, a phenoxy group or a nitro radical;
- W represents a group $CR_4$ or a nitrogen atom;
- $R_4$ represents a hydrogen atom, a halogen atom, a $C_1$-$C_6$ alkyl radical, a cyano radical, an aromatic group, a phenoxy radical or a nitro radical.
- Y represents an oxygen atom, a sulfur atom or a group $NR_5$;
- $R_5$ represents a hydrogen atom or a $C_1$-$C_6$ alkyl radical;
- Z represents a group $-C_pH_{2p}-$, with p being an integer between 2 and 4, which may be unsubstituted or substituted with one or more substituents chosen from halo, hydroxyl, alkyl, haloalkyl, alkoxy, amino, mono- or dialkylamino, mono- or dihydroxyalkylamino and carboxyl radicals; a group $-C_qH_{2q}CO-$, with q being an integer between 1 and 3, which may be unsubstituted or substituted with one or more substituents chosen from halo, hydroxyl, alkyl, haloalkyl, alkoxy, amino, mono- or dialkylamino, mono- or dihydroxyalkylamino and carboxyl radicals;
- n represents an integer from 1 to 4;
- B represents a substituted or unsubstituted, 5-to 16-membered, fused or non-fused aromatic or heteroaromatic nucleus.

**2.** Use according to Claim 1, in which A is a benzene, anthracene, naphthalene or quinoline nucleus.

**3.** Use according to Claim 1 or 2, in which A is unsubstituted or substituted with one or more groups chosen from a halo radical, a $C_1$-$C_6$ alkyl radical, a $C_1$-$C_6$ alkoxy radical, a $C_1$-$C_6$ alkylsulfonyl radical ($-SO_2$-alkyl), a $C_1$-$C_6$ alkylsulfonate radical ($-SO_3$-alkyl), a cyano radical, a trifluoromethyl radical, a $C_1$-$C_6$ alkylcarbonyl radical, a trifluoromethylsulfonyl radical ($-SO_2-CF_3$), a trifluoromethylcarbonyl radical, a phenylsulfonyl radical ($-SO_2$-Ph), a phenylsulfonate radical ($-SO_3$-Ph), a phenylcarbonyl radical, a nitro radical, a $C_1$-$C_6$ alkoxycarbonyl radical, a phosphonyl radical ($-PO(OH)_2$), a phosphonyl($C_1$-$C_6$)alkyl radical (-alkyl-$PO(OH)_2$), a hydroxyl radical, an amino radical, a di($C_1$-$C_6$)alkylamino radical, a (hydroxy($C_1$-$C_6$) alkyl) amino radical, a di(hydroxy($C_1$-$C_6$)alkyl)amino radical, an (amino($C_1$-$C_6$)alkyl)amino radical, a di (amino ($C_1$-$C_6$) alkyl) amino radical, a (hydroxy ($C_1$-$C_6$) alkyl) (($C_1$-$C_6$) alkyl) amino radical, an (amino ($C_1$-$C_6$) alkyl) (($C_1$-$C_6$) alkyl) amino radical, an (amino ($C_1$-$C_6$) alkyl) (hydroxy ($C_1$-$C_6$) alkyl) amino radical, a hydroxy ($C_1$-$C_6$) alkyl radical, an amino ($C_1$-$C_6$) alkyl radical, a (($C_1$-$C_6$) alkyl) amino ($C_1$-$C_6$) alkyl radical, a di(($C_1$-$C_6$)-alkyl) amino ($C_1$-$C_6$) alkyl radical, a (hydroxy ($C_1$-$C_6$) - alkyl) amino ($C_1$-$C_6$) alkyl radical, a di (hydroxy ($C_1$-$C_6$) alkyl) - amino ($C_1$-$C_6$) alkyl radical, an (amino ($C_1$-$C_6$) alkyl) amino-($C_1$-$C_6$) alkyl radical, a di (amino ($C_1$-$C_6$) alkyl) amino ($C_1$-$C_6$) - alkyl radical, a (($C_1$-$C_6$) alkyl) (hydroxy ($C_1$-$C_6$) alkyl) amino-($C_1$-$C_6$) alkyl radical, an (amino

($C_1$-$C_6$) alkyl) (($C_1$-$C_6$) alkyl) - amino radical, a (hydroxy ($C_1$-$C_6$) alkyl) (($C_1$-$C_6$) alkyl) - amino ($C_1$-$C_6$) alkyl radical; a phenyl ($C_1$-$C_6$) alkyl radical optionally substituted with one or more substituents chosen from halo, hydroxyl, alkyl, haloalkyl, alkoxy, amino, mono- or dialkylamino, mono- or dihydroxyalkylamino and carboxyl radicals, a cationic group of the quaternary ammonium type, a $C_1$-$C_6$ alkyl radical substituted with a cationic group of the quaternary ammonium type, a carboxyl radical, a ($C_1$-$C_6$) alkyl radical substituted with a carboxyl radical, a thio radical, a thio ($C_1$-$C_6$)alkyl radical, a sulfonate radical ($-SO_3^-$) a ($C_1$-$C_6$) alkyl radical substituted with a sulfonate radical, a ($C_1$-$C_6$) alkylcarbonyl ($C_1$-$C_6$)alkyl radical, a di (halo ($C_1$-$C_6$)alkyl)amino radical, an acetamido radical, an aryloxy radical, an aryloxy ($C_1$-$C_6$) alkyl radical, an ethenyl radical ($-CH=CH_2$), an ethenylcarbonyl radical ($-CO-CH=CH_2$); two adjacent groups possibly forming an aromatic or heteroaromatic ring, which is unsubstituted or substituted with one or more substituents chosen from halo, hydroxyl, alkyl, haloalkyl, alkoxy, amino, mono- or dialkylamino, mono- or dihydroxyalkylamino and carboxyl radicals, or a ring of $-O-C_mH_{2m}-O-$ type where m is an integer equal to 1 or 2.

4. Use according to Claim 3, in which A is unsubstituted or substituted with one or more groups chosen from a ($C_1$-$C_6$) alkylsulfonyl radical; a pyridinium or imidazolium group which is unsubstituted or substituted with one or more substituents chosen from halo, hydroxyl, alkyl, haloalkyl, alkoxy, amino, mono- or dialkylamino, mono- or dihydroxyalkylamino and carboxyl radicals; a tri($C_1$-$C_6$)alkylammonium group; a sulfonate radical.

5. Use according to any one of the preceding claims, in which X is chosen from a group $CR_1R_2$.

6. Use according to any one of the preceding claims, in which $R_1$ and $R_2$ are chosen from a $C_1$-$C_6$ alkyl radical.

7. Use according to any one of the preceding claims, in which $R_3$ is chosen from a hydrogen atom.

8. Use according to any one of the preceding claims, in which W is chosen from a group $CR_4$.

9. Use according to any one of the preceding claims, in which $R_4$ is chosen from a hydrogen atom.

10. Use according to any one of the preceding claims, in which Y is chosen from an oxygen atom.

11. Use according to any one of the preceding claims, in which Z is chosen from a $-C_pH_{2p}-$ group, with p being an integer between 2 and 4, which is unsubstituted or substituted with one or more substituents chosen from halo, hydroxyl, alkyl, haloalkyl, alkoxy, amino, mono- or dialkylamino, mono- or dihydroxyalkylamino and carboxyl radicals.

12. Use according to any one of the preceding claims, in which n is equal to 1 or 2.

13. Use according to any one of the preceding claims, in which B is a benzene, carbazole or indole nucleus.

14. Use according to any one of the preceding claims, in which B is unsubstituted or substituted with one or more groups chosen from a halo radical, a $C_1$-$C_6$ alkyl radical, a $C_1$-$C_6$ alkoxy radical, a cyano radical, a trifluoromethyl radical, a $C_1$-$C_6$ alkylcarbonyl radical, a trifluoromethylsulfonyl radical, a trifluoromethylcarbonyl radical, a phenylsulfonyl radical, a phenylcarbonyl radical, a phenyl radical which is unsubstituted or substituted with one or more substituents chosen from halo, hydroxyl, alkyl, haloalkyl, alkoxy, amino, mono- or dialkylamino, mono- or dihydroxyalkylamino and carboxyl radicals, an acylamino radical, a hydroxyl radical, an amino radical, a di(($C_1$-$C_6$)alkyl)amino radical, a hydroxy-($C_1$-$C_6$)alkylamino radical, a di(hydroxy($C_1$-$C_6$)alkyl)amino radical, an (amino ($C_1$-$C_6$) alkyl) amino radical, a di (amino ($C_1$-$C_6$) alkyl) amino radical, a (($C_1$-$C_6$) alkyl) - (hydroxy ($C_1$-$C_6$) alkyl) amino radical, an (amino ($C_1$-$C_6$) - alkyl) (($C_1$-$C_6$) alkyl) amino radical, an (amino ($C_1$-$C_6$) - alkyl) (hydroxy ($C_1$-$C_6$) alkyl) amino radical, a hydroxy-($C_1$-$C_6$) alkyl radical, an amino ($C_1$-$C_6$) alkyl radical, a ($C_1$-$C_6$) alkylamino ($C_1$-$C_6$) alkyl radical, a di (($C_1$-$C_6$)-alkyl) amino ($C_1$-$C_6$) alkyl radical, a (hydroxy ($C_1$-$C_6$) - alkyl) amino ($C_1$-$C_6$) alkyl radical, a di (hydroxy ($C_1$-$C_6$) - alkyl) amino ($C_1$-$C_6$) alkyl radical, an amino ($C_1$-$C_6$) alkylamino ($C_1$-$C_6$) alkyl radical, a di(amino ($C_1$-$C_6$) alkyl)amino-($C_1$-$C_6$) alkyl radical, a (($C_1$-$C_6$) alkyl) (hydroxy ($C_1$-$C_6$) - alkyl) amino ($C_1$-$C_6$) alkyl radical, a (($C_1$-$C_6$) alkyl) (amino-($C_1$-$C_6$) alkyl) amino radical, a (hydroxy ($C_1$-$C_6$) alkyl) - (($C_1$-$C_6$) alkyl) amino ($C_1$-$C_6$) alkyl radical, a phenyl ($C_1$-$C_6$) - alkyl radical which is unsubstituted or substituted with one or more substituents chosen from halo, hydroxyl, alkyl, haloalkyl, alkoxy, amino, mono- or dialkylamino, mono- or dihydroxyalkylamino and carboxyl radicals, a cationic group of the quaternary ammonium type, a ($C_1$-$C_6$)alkyl radical substituted with a cationic group of the quaternary ammonium type, a carboxyl radical, a ($C_1$-$C_6$)alkyl radical substituted with a carboxyl radical, a thio radical, a thio($C_1$-$C_6$)alkyl radical, a sulfonate radical, a ($C_1$-$C_6$) alkyl radical substituted with a sulfonate radical, a ($C_1$-$C_6$)-alkylcarbonyl($C_1$-$C_6$) alkyl radical, a di(halo($C_1$-$C_6$)-alkyl)amino radical, an acetamido radical, an aryloxy radical, an aryloxy($C_1$-$C_6$)alkyl radical, an ethenyl radical, an ethenylcarbonyl radical, a group $NR_6R_7$, $R_6$ and $R_7$

possibly forming, together with the nitrogen atom to which they are attached, a non-aromatic $C_5$, $C_6$ or $C_7$ ring, optionally interrupted with one or more heteroatoms such as a nitrogen, oxygen or sulfur atom, an alkylene chain possibly containing an oxygen or sulfur atom and possibly ending with a cyano, $C_1$-$C_6$ alkylsulfonyl or $C_1$-$C_6$ alkylcarbonyl group; two adjacent groups of B possibly forming an aromatic or heteroaromatic ring, which is unsubstituted or substituted with one or more substituents chosen from halo, hydroxyl, alkyl, haloalkyl, alkoxy, amino, mono- or dialkylamino, mono- or dihydroxyalkylamino and carboxyl radicals, or a ring of -O-$C_rH_{2r}$-O- type in which r represents an integer equal to 1 or 2.

**15.** Use according to Claim 14, in which B is unsubstituted or substituted with one or more groups chosen from a hydroxyl radical; an amino radical; a di (($C_1$-$C_6$) alkyl) amino radical; a $C_1$-$C_6$ alkyl radical; an acetamido radical; a pyridinium group; a tri($C_1$-$C_6$) alkylammonium group.

**16.** Use according to any one of the preceding claims, in which the compound(s) of formula (I) is (are) chosen from 9a-[2-[4-(dimethylamino)phenyl]-1,3-butadienyl]-2,3,9,9a-tetrahydro-9,9-dimethyloxazolo[3,2-a]indole-7-carboxylic acid; [9a-[2-[4-(dimethylamino)phenyl]-ethenyl]-2,3,9,9a-tetrahydro-9,9-dimethyloxazolo-[3,2-a]indol-7-yl]phosphonic acid; 4-[2-(9,9-diethyl-2,3-dihydro-7-methoxyoxazolo[3,2-a]indol-9a(9H)-yl)-ethenyl]-N,N-diethylbenzenamine; [3-[9a-[2-[4-(dimethylamino)phenyl]ethenyl]-2,3,9,9a-tetrahydro-9,9-dimethyloxazolo[3,2-a]indol-7-yl]propyl]phosphonic acid; 4-[2-(2,3-dihydro-9,9-dimethyloxazolo[3,2-a]indol-9a(9H)-yl)ethenyl]-N-methyl-N-phenylbenzenamine; 4-[2-(2,3-dihydro-9,9-dimethyloxazolo[3,2-a]indol-9a(9H)-yl)ethenyl]-3-ethoxy-N,N-diethylbenzenamine; 4-[2-(2,3-dihydro-9,9-dimethyloxazolo[3,2-a]indol-9a(9H)-yl)-ethenyl]-N-ethyl-N-(2-methylpropyl)benzenamine; 4-[2-(2,3-dihydrooxazolo[3,2-a]indol-9a(9H)-yl)ethenyl]-N,N-dimethylbenzenamine; 4-[4-(2,3-dihydro-7,9,9-trimethyloxazolo[3,2-a]indol-9a(9H)-yl)-1,3-butadienyl]-N,N-dimethylbenzenamine; 4-[4-(2,3-dihydro-9,9-dimethyl-7-nitrooxazolo[3,2-a]indol-9a(9H)-yl)-1,3-butadienyl]-N,N-dimethylbenzenamine; 9a-[4-[4-(dimethylamino)-phenyl]-1,3-butadienyl]-2,3,9,9a-tetrahydro-9,9-dimethyloxazolo[3,2-a]indole-7-carbonitrile; 9a-[2-[4-(dimethylamino) phenyl]ethenyl]-2,3,9,9a-tetrahydro-9,9-dimethyloxazolo[3,2-a]indole-7-carbonitrile; 4-[2-(2,3-dihydro-9,9-dimethyloxazolo[3,2-a]indol-9a(9H)-yl)-ethenyl]-N,N-dimethylbenzenamine; 9a-[2-[4-(dimethylamino)phenyl]ethenyl]-2,3,9,9a-tetrahydro-9,9-dimethyloxazolo[3,2-a]indole-7-sulfonic acid methyl ester; N,N-bis(2-chloroethyl)-4-[2-(2,3-dihydro-9,9-dimethyloxazolo[3,2-a]indol-9a(9H)-yl)ethenyl]benzenamine; 9a-[2-[4-(dimethylamino)phenyl]ethenyl]-9,9a-dihydro-9,9-dimethyl-7-(octylsulfonyl)oxazolo[3,2-a]-indol-2(3H)-one; 9a-[2-[4-(dimethylamino)phenyl]-ethenyl]-9,9a-dihydro-9,9-dimethyl-7-(phenylsulfonyl)-oxazolo[3,2-a]indol-2(3H)-one; 9a-[2-[4-(dimethylamino) phenyl]- 1- methylethenyl]- 9,9a- dihydro- 9,9- dimethyl- 7-(methylsulfonyl) oxazolo [3,2- a] indol- 2 (3H)-one; 9a-[2-[4-(dimethylamino) phenyl] ethenyl]- 9,9a- dihydro- 9,9- dimethyl- 7-(methylsulfonyl) oxazolo [3,2- a] indol- 2 (3H)-one; 4-[2-[2,3-dihydro-9,9-dimethyl-7-(methylsulfonyl)oxazolo[3,2-a]indol-9a(9H)-yl]ethenyl]-N,N-dimethylbenzenamine; 4-[2-[9-(ethoxymethyl)-2,3-dihydro-9-methyl-7-(methylsulfonyl)oxazolo[3,2-a]indol-9a(9H)-yl]ethenyl]-N,N-dimethylbenzenamine; 4-[2-[2,3-dihydro-2,9,9-trimethyl-7-(methylsulfonyl)oxazolo-[3,2-a]indol-9a(9H)-yl] ethenyl]-N,N-dimethylbenzenamine; 4-[2-[2,3-dihydro-9,9-dimethyl-7-(methylsulfonyl)oxazolo[3,2-a]indol-9a (9H)-yl]-1-propenyl]- N,N-dimethylbenzenamine; N,N-dibutyl-4-[2-[2,3-dihydro-9,9-dimethyl-7-(methylsulfonyl)oxazolo[3,2-a]indol-9a(9H)-yl]ethenyl]benzenamine; 4-[2-[2,3-dihydro-9,9-dimethyl-7-(phenylsulfonyl)oxazolo[3,2-a] indol-9a(9H)-yl]ethenyl]-N,N-dimethyl; 4-[2-[2,3-dihydro-9,9-dimethyl-7-(octylsulfonyl)oxazolo[3,2-a]indol-9a (9H)-yl]ethenyl]-N,N-dimethylbenzenamine; N-[4-[2-[7-(butyl-sulfonyl)-2,3-dihydro-9,9-dimethyloxazolo[3,2-a]indol-9a(9H)-yl]ethenyl]phenyl]acetamide; 4-[2-[7-(butylsulfonyl)-2,3-dihydro-9,9-dimethyloxazolo[3,2-a]indol-9a (9H)-yl]ethenyl]-N,N-dimethylbenzenamine; 4-[4-[2,3-dihydro-9,9-dimethyl-7-(methylsulfonyl)oxazolo[3,2-a]-indol-9a(9H)-yl]-1,3-butadienyl]-N,N-dimethylbenzenamine; 9a-[2-[4-(dimethylamino)phenyl]ethenyl]-2,3,9,9a-tetrahydro-9-methyloxazolo[3,2-a]indole-9-ethanol; 4-[2-(9,9-diethyl-2,3-dihydrooxazolo[3,2-a]-indol-9a(9H)-yl)ethenyl]-N,N-dimethylbenzenamine; 4-[2-[2,3-dihydro-9-methyl-9-(2-phenoxyethyl)oxazolo[3,2-a]-indol-9a(9H)-yl]ethenyl]-N,N-dimethylbenzenamine; 4-[2-[9-(ethoxymethyl)-2,3-dihydro-9-methyloxazolo[3,2-a]-indol-9a(9H)-yl]ethenyl]-N,N-dimethylbenzenamine; 4-[2-(11,11-dimethylbenz[e]oxazolo[3,2-a]indol-10a(11H)-yl)-ethenyl]-N,N-dimethylbenzenamine; 4-[2-(7-methoxy-9,9-dimethyloxazolo[3,2-a]indol-9a(9H)-yl)ethenyl]-N,N-dimethylbenzenamine;4-[2-(9,9-dimethyloxazolo[3,2-a]-indol-9a(9H)-yl)ethenyl]-N,N-dimethylbenzenamine;9a-[2-[4-(dimethylamino)phenyl]ethenyl]-3-ethyl-9,9a-dihydro-9,9-dimethyloxazolo[3,2-a]indol-2(3H)-one;7-chloro-9a-[2-[4-(dimethylamino)phenyl]ethenyl]-9,9a-dihydro-3,9,9-trimethyloxazolo[3,2-a]indol-2(3H)-one; 9a-[2-[4-(dibutylamino)phenyl] ethenyl]-9,9a-dihydro-9,9-dimethyloxazolo[3,2-a]indol-2(3H)-one; 9a-[2-[4-(dimethylamino)phenyl]ethenyl]-9,9a-dihydro-9-(2-hydroxyethyl)-9-methyloxazolo[3,2-a]indol-2(3H)-one; 9a-[2-[4-(dimethylamino)phenyl]-1-propenyl]-9,9a-dihydro-9,9-dimethyloxazolo[3,2-a]indol-2(3H)-one; 9,9a-dihydro-7,9,9-trimethyl-9a-[2-(4-nitrophenyl)-ethenyl]oxazolo[3,2-a]indol-2(3H)-one; N-[4-[2-(2,3-dihydro-9,9-dimethyl-2-oxooxazolo[3,2-a]indol-9a(9H)-yl)ethenyl] phenyl]acetamide; 9a-[2-[4-(dimethylamino)-phenyl]ethenyl]-9,9a-dihydro-6-methoxy-9,9-dimethyloxazolo[3,2-a] indol-2(3H)-one; 9a-[2-[4-(dimethylamino)phenyl]ethenyl]-2,3,9,9a-tetrahydro-9,9-dimethyl-2-oxooxazolo[3,2-a]indole-7-carboxylic acid ethyl ester; 9a-[2-[4-(dimethylamino)phenyl]ethenyl]-9,9a-dihydro-7,9,9-trimethyloxazolo[3,2-a]indol-2(3H)-one; 10a-[2-[4-(dimethylamino)phenyl]ethenyl]-10a,11-dihydro-11,11-dimethylbenz[e]oxazolo [3,2-a]indol-9(8H)-one; 9,9a-dihydro-9,9-dimethyl-9a-[2-(4-nitrophenyl)-ethenyl]oxazolo[3,2-a]indol-2(3H)-one; 7-chloro- 9a-[2-[4-(dimethylamino) phenyl] ethenyl]- 9,9a- dihydro- 9,9- dimethyloxazolo [3,2- a] indol- 2 (3H)-one; 9a-[2-[4-(dimethylamino)phenyl]ethenyl]-9,9a-dihydro-9,9-dimethyloxazolo[3,2-a]indol-2(3H)-one; 4-[2-(7-chloro-2,3-dihydro-9,9-dimethyloxazolo[3,2-a]indol-9a(9H)-yl)ethenyl]-N,N-dimethylbenzenamine; 4-[2-(2,3-dihydro-9,9-dimethyloxazolo[3,2-a]indol-9a(9H)-yl)ethenyl]-N,N-dimethylbenzenamine; 4-[2-(7-chloro-2,3-dihydro-2,9,9-trimethyloxazolo[3,2-a]indol-9a(9H)- yl]ethenyl]-N,N-dimethylbenzenamine; 4-[2-(2,3-dihydro-9,9-dimethyloxazolo [3,2-a]indol-9a(9H)-yl)-1-methylethenyl]-N,N-dimethylbenzenamine; 4-[2-(2,3-dihydro-9,9-dimethyloxazolo[3,2-a] indol-9a(9H)-yl)ethenyl]-N,N-diethylbenzenamine; 2,3,9,9a-tetrahydro-7-methoxy-9,9-dimethyl-9a-[2-(4-nitrophenyl)ethenyl]oxazolo[3,2-a]-indole; 4-[2-(2,3-dihydro-9,9-dimethyl-7-nitrooxazolo-[3,2-a]indol-9a(9H)-yl)ethenyl]-N, N-dimethylbenzenamine; N,N-dibutyl-4-[2-(2,3-dihydro-9,9-dimethyloxazolo[3,2-a]indol-9a(9H)-yl)ethenyl]benzenamine; 2,3,9,9a-tetrahydro-9,9-dimethyl-7-nitro-9a-[2-(4-nitrophenyl)ethenyl]oxazolo[3,2-a]indole; 7-chloro-2,3,9,9a-tetrahydro-9,9-dimethyl-9a-[2-(4-nitrophenyl)-ethenyl]oxazolo[3,2-a]indole; 4-[2-(2,3-dihydro-7-iodo-9,9-dimethyloxazolo[3,2-a]indol-9a(9H)-yl)ethenyl]-N,N-dimethylbenzenamine; 4-[2-(2,3-dihydro-5-methoxy-9,9-dimethyloxazolo[3,2-a]indol-9a(9H)- yl)ethenyl]-N,N-dimethylbenzenamine; 4-[2-(2,3-dihydro-7-methoxy-9,9-dimethyloxazolo[3,2-a]indol-9a(9H)-yl)ethenyl]-N,N-dimethylbenzenamine; 4-[4-(2,3-dihydro-9,9-dimethyloxazolo [3,2-a]indol-9a(9H)-yl)-1,3-butadienyl]- N,N-diethylbenzenamine; 4-[4-(7-chloro-2,3-dihydro-9,9-dimethyloxazolo [3,2-a]indol-9a(9H)-yl)-1,3-butadienyl]-N,N-dimethylbenzenamine; 4-[4-(2,3-dihydro-9,9-dimethyloxazolo[3,2-a]indol-9a(9H)-yl)-1,3-butadienyl]-N,N-dimethylbenzenamine; 4-[4-(2,3-dihydro-7-methoxy-9,9-dimethyloxazolo[3,2-a]indol-9a(9H)-yl)-1,3-butadienyl]-N,N-dimethylbenzenamine; 2,3,9,9a-tetrahydro-7,9,9-trimethyl-9a-[2-(4-nitrophenyl)ethenyl]oxazolo[3,2-a]indole; N-[4 -[2-(2,3-dihydro-9,9-dimethyloxazolo[3,2-a]indol-9a(9H)-yl)ethenyl]phenyl]acetamide; 4-[2-(2,3-dihydro-6-methoxy-9,9-dimethyloxazolo[3,2-a]indol-9a(9H)-yl)ethenyl]-N,N-dimethylbenzenamine; 4-[2-(8,9-dihydro-11,11-dimethylbenz[e]oxazolo[3,2-a]indol-10a(11H)-yl)ethenyl]-N,N-dimethylbenzenamine; 4-[2-(2,3-dihydro-7,9,9-trimethyloxazolo[3,2-a]indol-9a(9H)-yl)ethenyl]-N,N-dimethylbenzenamine; 2,3,9,9a-tetrahydro-9,9-dimethyl-9a-[2-(4-nitrophenyl)ethenyl]oxazolo[3,2-a]indole; 9,9a-dihydro-9,9-dimethyl-9a-[2-(9-methyl-9H-carbazol-3-yl)ethenyl]-7- (methylsulfonyl)oxazolo[3,2-a]indol-2(3H)-one; 9a-[2-(9-hexyl-9H-carbazol-3-yl)ethenyl]-2,3,9,9a-tetrahydro-9,9-dimethyl-7-(phenylsulfonyl)oxazolo[3,2-a]indole; 2,3,9,9a-tetrahydro-9,9-dimethyl-7-(methylsulfonyl)-9a-[2-(9-octyl-9H-carbazol-3-yl)ethenyl]oxazolo[3,2-a]indole; 9a-[2-(9-butyl-6-methoxy-9H-carbazol-3-yl)ethenyl]-2,3,9,9a-tetrahydro-9,9-dimethyloxazolo[3,2-a]indole-7-carboxylic acid ethyl ester; 9a-[2-(9-ethyl-9H-carbazol-3-yl)ethenyl]-2,3,9,9a-tetrahydro-9,9-dimethyloxazolo[3,2-a]indole-7-sulfonic acid methyl ester; 3-chloro-6-[2-[2,3-dihydro-9,9-dimethyl-7-(methylsulfonyl)oxazolo[3,2-a]indol-9a(9H)-yl]ethenyl]-9-octyl-9H-carbazole; 3-[2-[2,3-dihydro-9,9-dimethyl-7-(methylsulfonyl)oxazolo[3,2-a]indol-9a(9H)-yl]ethenyl]-9-methyl-9H-carbazole; 3-[2-[9-(2-ethoxyethyl)-2,3-dihydro-9-methyl-7-(methylsulfonyl)oxazolo[3,2-a]indol-9a(9H)-yl] ethenyl]-9H-carbazole; 9a-[2-(9-hexyl-9H-carbazol-3-yl)ethenyl]-2,3,9,9a-tetrahydro-9-(2-hydroxyethyl)-9-methyloxazolo[3,2-a]indole-7-carboxylic acid ethyl ester; 2,3,9,9a-tetrahydro-9,9-dimethyl-9a-[2-(9-octyl-9H-carbazol-3-yl)ethenyl]oxazolo[3,2-a]indole-7-carboxylic acid ethyl ester; 9a-[2-(9-butyl-9H-carbazol-3-yl)ethenyl]-2,3,9,9a-tetrahydro-9,9-dimethyloxazolo[3,2-a]indole-7-carboxylic acid ethyl ester; 2,3,9,9a-tetrahydro-9,9-dimethyl-9a-[2-(9-methyl-9H-carbazol-3-yl)ethenyl]oxazolo[3,2-a]indole-7-carboxylic acid ethyl ester; 9a-[2-(9-butyl-6-ethoxy-9H-carbazol-3-yl)ethenyl]-2,3,9,9a-tetrahydro-N,N,9,9-tetramethyloxazolo[3,2-a]indol-7-amine; 2,3,9,9a-tetrahydro-9-methyl-9a-[2-(9-methyl-9H-carbazol-3-yl)ethenyl]oxazolo[3,2-a]indole-9-ethanol; 9a-[2-(9-butyl-9H-carbazol-3-yl) ethenyl]-2,3,9,9a-tetrahydro-N,N,9,9-tetramethyloxazolo[3,2-a]indol-7-amine; 3-[2-(2,3-dihydro-6-methoxy-9,9-dimethyloxazolo[3,2-a]indol-9a(9H)-yl)ethenyl]-9-methyl-9H-carbazole; 3-[2-(2,3-dihydro-9,9-dimethyloxazolo[3,2-a]indol-9a(9H)-yl)-1-propenyl]-9-methyl-9H-carbazole; 3-[2-(7-chloro-2,3-dihydro-9,9-dimethyloxazolo[3,2-a]indol-9a(9H)-yl)-ethenyl]-9-methyl-9H-carbazole; 3-bromo-6-[2-(2,3-dihydro-9,9-dimethyloxazolo[3,2-a]indol-9a(9H)-yl] ethenyl]-9-ethyl-9H-carbazole; 3-[2-(2,3-dihydro-9,9-dimethyloxazolo[3,2-a]indol-9a(9H)-yl)ethenyl]-9-methyl-9H-carbazole.

**17.** Use according to Claim 16, in which the compound(s) of formula (I) is (are) chosen from the compounds given in the table below:

| | |
|---|---|
| 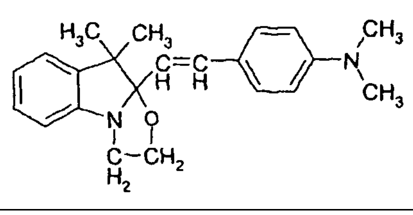 | Dye 1 |

(continued)

| | |
|---|---|
| 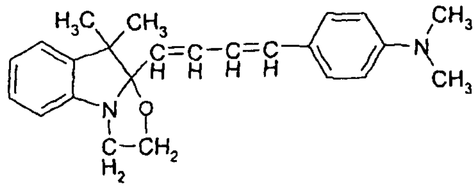 | Dye 2 |
| 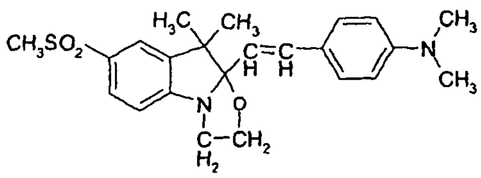 | Dye 3 |
| 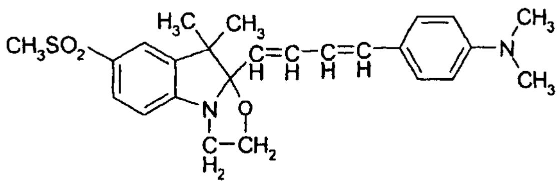 | Dye 4 |
| 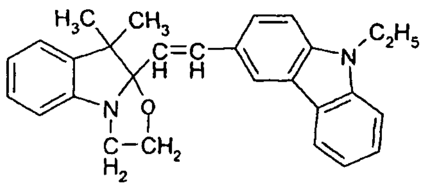 | Dye 5 |
| 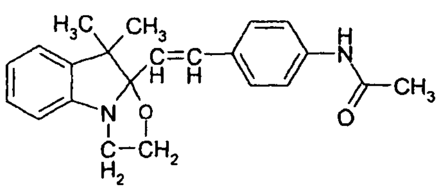 | Dye 6 |
| 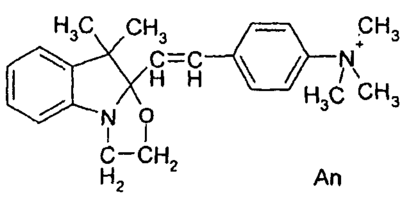 An | Dye 7 |

(continued)

| | |
|---|---|
| 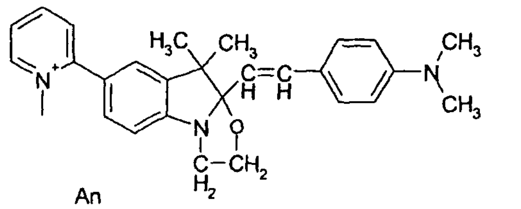 | Dye 8 |
| 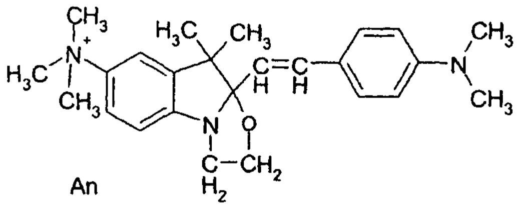 | Dye 9 |
| 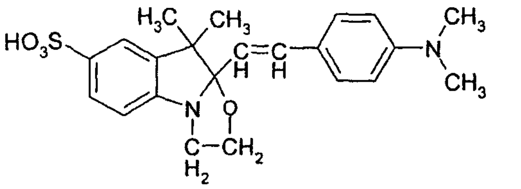 | Dye 10 |
| 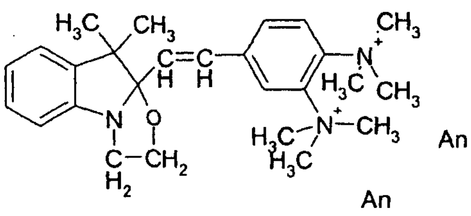 | Dye 11 |

in which An, which may be identical or different, represent a negative counterion.

**18.** Use according to any one of the preceding claims, in which the compound(s) chosen from the compounds of formula (I) and the compounds of formula (II), and the addition salts thereof, represent from 0.0001% to 10% by weight relative to the total weight of the composition.

**19.** Use according to any one of the preceding claims, in which the dye composition comprises at least one acidifying agent and/or at least one basifying agent.

**20.** Use according to Claim 19, in which the acidifying agents(s) is (are) chosen from mineral acids and organic acids.

**21.** Use according to Claim 18 or 19, in which the basifying agents are chosen from:

- basic amino acids;
- alkali metal or alkaline-earth metal carbonates or bicarbonates;
- silicates or metasilicates;
- the compounds of formula (III) below:

$$X(OH)_n \qquad (III)$$

in which:

• X represents a potassium, lithium, sodium or ammonium ion $N^+R_8R_9R_{10}R_{11}$ with $R_8$, $R_9$, $R_{10}$ and $R_{11}$, which may be identical or different, denoting a $C_2$-$C_4$ alkyl radical when n is equal to 1;
• X represents a magnesium or calcium atom when n is equal to 2;

- the compounds of formula (IV) below:

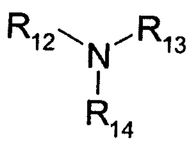

(IV)

in which:

• $R_{12}$ represents a hydrogen atom; a $C_1$-$C_6$ alkyl radical; a $C_1$-$C_6$ monohydroxyalkyl radical; a $C_2$-$C_6$ polyhydroxyalkyl radical;
• $R_{13}$ and $R_{14}$, which may be identical or different, represent a hydrogen atom; a $C_1$-$C_6$ alkyl radical; a $C_1$-$C_6$ monohydroxyalkyl radical; a $C_2$-$C_6$ polyhydroxyalkyl radical;

- the compounds of formula (V) below:

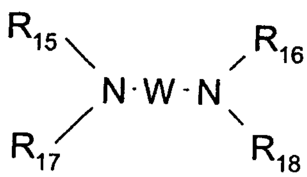

(V)

in which:

• W is a propylene residue optionally substituted with a hydroxyl group or a $C_1$-$C_4$ alkyl radical;
• $R_{15}$, $R_{16}$, $R_{17}$ and $R_{18}$, which may be identical or different, represent a hydrogen atom or a $C_1$-$C_4$ alkyl or $C_1$-$C_4$ hydroxyalkyl radical.

**22.** Use according to any one of the preceding claims, in which the suitable dyeing medium consists of water or of at least one organic solvent, or of a mixture of water and of at least one organic solvent.

**23.** Use according to Claim 22, in which the solvent(s) is (are) chosen from ketones; linear or branched monoalcohols or diols containing 2 to 10 carbon atoms; aromatic alcohols; polyols or polyol ethers; and also diethylene glycol alkyl ethers.

**24.** Use according to Claim 22 or 23, in which the suitable dyeing medium consists of water or of a mixture of water and of at least one organic solvent and the pH is between 2 and 12.

**25.** Use according to any one of the preceding claims, in which the dye composition comprises at least one additive chosen from anionic, cationic, nonionic, amphoteric and zwitterionic surfactants or mixtures thereof, anionic, cationic, nonionic, amphoteric or zwitterionic polymers or mixtures thereof, mineral or organic thickeners, antioxidants, penetrants, sequestrants, fragrances, buffers, dispersants, conditioning agents, preserving agents and opacifiers.

**26.** Keratin fibre treatment process, in which a dye composition as defined in any one of Claims 1 to 25 is applied to the keratin fibres for a sufficient leave-on time, this application being optionally followed by rinsing.

**27.** Process according to Claim 26, in which the application of the dye composition is followed by a pre- or post-treatment of the keratin fibres allowing development of the coloration.

**28.** Process according to Claim 27, in which the pre- or post-treatment consists in treating the fibres via the action of light, an electrical current, heat, an acidifying agent as defined in Claim 19 or 20 and/or a basifying agent as defined in Claim 19 or 21 and/or a solvent as defined in Claim 22 or 23, or electromagnetic radiation.

**29.** Process according to any one of Claims 26 to 28, in which the dye composition is applied in the presence of at least one oxidizing agent.

**30.** Process according to Claim 29, in which the oxidizing agent is chosen from hydrogen peroxide, urea peroxide, alkali metal bromates, persalts, peracids and enzymes of oxidase type.

**31.** Multi-compartment device containing, in a first compartment, a dye composition comprising at least one compound as defined in any one of Claims 1 to 17, and, in a second compartment, at least one acidifying agent as defined in Claim 19 or 20 and/or at least one basifying agent as defined in Claim 19 or 21 and/or at least one solvent as defined in Claim 22 or 23.

**32.** Device according to Claim 31, containing, in a third compartment, at least one oxidizing agent as defined in Claim 29 or 30.

**33.** Compounds as defined in any one of Claims 1 to 17, comprising at least one group chosen from a cationic group of the quaternary ammonium type.

**34.** Compounds according to Claim 33, in which the cationic groups of the quaternary ammonium type are chosen from tri ($C_1$-$C_6$) alkylammonium, oxazolium, thiazolium, imidazolium, pyrazolium, pyridinium, pyrrolium, triazolium, isoxazolium, isothiazolium, pyrimidinium, pyrazinium, triazinium, pyridazinium, indolium, quinolinium and isoquinolinium groups, which may be substituted or unsubstituted.

**35.** Dye composition comprising, in a suitable dyeing medium, at least one compound as defined in Claim 33 or 34.

## Patentansprüche

**1.** Verwendung einer Farbmittelzusammensetzung, die in einem zum Färben geeigneten Medium mindestens eine Verbindung enthält, die unter den Verbindungen der Formel (I), den Verbindungen der Formel (II) und ihren Additionssalzen ausgewählt ist, zum Färben von Keratinfasern:

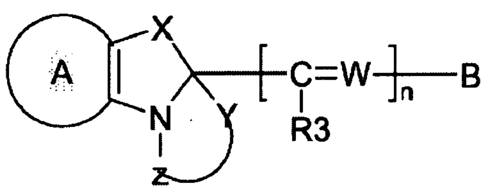

(I)

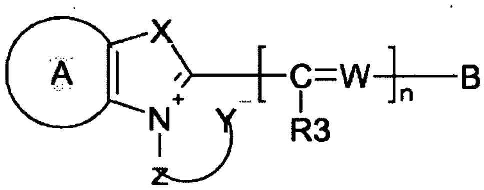

(II)

in den Formeln:

· A bedeutet einen 5- bis 16-gliedrigen, aromatischen oder heterocyclischen Kern, der kondensiert oder nicht kondensiert, substituiert oder unsubstituiert ist;
· X bedeutet ein Sauerstoffatom, ein Schwefelatom oder eine Gruppe $CR_1R_2$;
· $R_1$ und $R_2$ bedeuten unabhängig voneinander ein Wasserstoffatom, eine Alkyl($C_{1-6}$)gruppe, eine Hydroxyalkyl ($C_{1-6}$)-gruppe, eine Alkoxyalkyl(Ci-$_6$)gruppe, eine Alkylenkette, die ein Sauerstoffatom oder ein Schwefelatom enthalten kann; $R_1$ und $R_2$ können gemeinsam einen 5- oder 6-gliedrigen, aromatischen oder nichtaromatischen Ring bilden, der gegebenenfalls ein oder mehrere Heteroatome enthält, wie ein Stickstoffatom, ein Sauerstoffatom oder ein Schwefelatom;
· $R_3$ bedeutet ein Wasserstoffatom, ein Halogenatom, eine Alkyl($C_{1-6}$)gruppe, eine Cyanogruppe, eine aromatische Gruppe, eine Phenoxygruppe, eine Nitrogruppe;
· W bedeutet eine Gruppe $CR_4$ oder ein Stickstoffatom;
$R_4$ bedeutet ein Wasserstoffatom, ein Halogenatom, eine Alkyl($C_{1-6}$)gruppe, eine Cyanogruppe, eine aromatische Gruppe, eine Phenoxygruppe, eine Nitrogruppe;
· Y bedeutet ein Sauerstoffatom, ein Schwefelatom, eine Gruppe $NR_5$;
· $R_5$ bedeutet ein Wasserstoffatom, eine Alkyl($C_{1-6}$)gruppe;
· Z bedeutet eine Gruppe -$C_pH_{2p}$-, wobei p eine ganze Zahl von 2 bis 4 bedeutet, die unsubstituiert ist oder mit einem oder mehreren Substituenten substituiert ist, die unter den · Substituenten Halogen, Hydroxy, Alkyl, Halogenalkyl, Alkoxy, Amino, Mono- oder Dialkylamino, Mono- oder Dihydroxyalkylamino, Carboxy ausgewählt sind; eine Gruppe - $C_qH_{2q}$CO-, wobei q eine ganze Zahl von 1 bis 3 bedeutet, die unsubstituiert ist oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind;
· n bedeutet eine ganze Zahl von 1 bis 4;
· B bedeutet einen 5- bis 16-gliedrigen aromatischen oder heterocyclischen Kern, der kondensiert oder nicht kondensiert, substituiert oder unsubstituiert ist.

**2.** Verwendung nach Anspruch 1, wobei A ein Benzolring, ein Anthracenring, ein Naphthalinring oder ein Chinolinring ist.

**3.** Verwendung nach Anspruch 1 oder 2, wobei A unsubstituiert vorliegt oder mit einer oder mehreren Gruppen substituiert ist, die unter einer Halogengruppe, einer Alkyl($C_{1-6}$)gruppe, einer Alkoxy($C_{1-6}$)gruppe, einer Alkylsulfonyl ($C_{1-6}$)gruppe (-$SO_2$-alkyl), einer Alkylsulfonat($C_{1-6}$)gruppe (-$SO_3$-alkyl), einer Cyanogruppe, einer Trifluormethylgruppe, einer Alkyl($C_{1-6}$)carbonylgruppe, einer Trifluormethylsulfonylgruppe (-$SO_2$-$CF_3$), einer Trifluormethylcarbonylgruppe, einer Phenylsulfonylgruppe (-$SO_2$-Ph), einer Phenylsulfonatgruppe (-$SO_3$-Ph), einer Phenylcarbonylgruppe, einer Nitrogruppe, einer Alkoxy($C_1$-$_6$)carbonylgruppe, einer Phosphonylgruppe (-$PO(OH)_2$), einer Phosphonylalkyl($C_{1-6}$)gruppe (-alkyl-$PO(OH)_2$), einer Hydroxygruppe, einer Aminogruppe, einer Dialkyl($C_{1-6}$)aminogruppe, einer (Hydroxyalkyl($C_{1-6}$))aminogruppe, einer Di(hydroxyalkyl(C1-6))aminogruppe, einer (Aminoalkyl($C_{1-6}$))aminogruppe, einer Di(aminoalkyl($C_{1-6}$))aminogruppe, einer (Hydroxyalkyl($C_{1-6}$))-(alkyl($C_{1-6}$))aminogruppe, einer (Aminoalkyl($C_{1-6}$))(alkyl($C_{1-6}$))aminogruppe, einer (Aminoalkyl($C_{1-6}$))(hydroxyalkyl($C_{1-6}$))aminogruppe, einer Hydroxyalkyl($C_{1-6}$)gruppe, einer Aminoalkyl($C_{1-6}$)gruppe, einer (Alkyl($C_{1-6}$))aminoalkyl($C_{1-6}$)gruppe, einer Di(alkyl($C_{1-6}$))-aminoalkyl($C_{1-6}$)gruppe, einer (Hydroxyalkyl($C_{1-6}$))aminoalkyl($C_{1-6}$ )gruppe, einer Di(hydroxyalkyl($C_{1-6}$))aminoalkyl ($C_{1-6}$)gruppe, einer (Aminoalkyl($C_1$-$_6$))aminoalkyl($C_1$-$_6$)gruppe, einer Di(aminoalkyl($C_{1-6}$ ))aminoalkyl($C_{1-6}$)gruppe, einer (Alkyl($C_{1-6}$))(hydroxyalkyl($C_{1-6}$))-aminoalkyl($C_{1-6}$)gruppe, einer (Aminoalkyl($C_{1-6}$))(alkyl($C_{1-6}$ )aminogruppe, einer (Hydroxyalkyl($C_{1-6}$))(alkyl($C_{1-6}$))aminoalkyl($C_{1-6}$ )gruppe, einer Phenylalkyl($C_{1-6}$))gruppe, die gegebenenfalls mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind, einer kationischen Gruppe vom quartären Ammoniumtyp, einer Alkyl($C_{1-6}$)gruppe, die mit einer kationischen Gruppe vom quartären Ammoniumtyp substituiert ist, einer Carboxygruppe, einer Alkyl($C_{1-6}$)gruppe, die mit einer Carboxygruppe substituiert ist, einer Thiogruppe, einer Thioalkyl($C_{1-6}$)gruppe, einer Sulfonatgruppe (-$SO_3$-), einer Alkyl($C_{1-6}$) gruppe, die mit einer Sulfonatgruppe substituiert ist, einer Alkyl($C_{1-6}$)carbonylalkyl($C_{1-6}$ )gruppe, einer Di(halogenalkyl($C_{1-6}$))aminogruppe, einer Acetamidogruppe, einer Aryloxygruppe, einer Aryloxyalkyl($C_{1-6}$ )gruppe, einer Ethenylgruppe (-CH=$CH_2$), einer Ethenylcarbonylgruppe (-CO-CH=$CH_2$) ausgewählt sind; wobei zwei aneinander angrenzende Gruppen einen aromatischen oder heteroaromatischen Ring, der unsubstituiert vorliegt oder mit einem öder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind, oder einen Ring vom Typ -O-$C_mH_{2m}$-O-, worin m eine ganze Zahl 1 oder 2 bedeutet, bilden können.

**4.** Verwendung nach Anspruch 3, wobei A unsubstituiert vorliegt oder mit einer oder mehreren Gruppen substituiert ist, die unter einer Alkyl($C_{1-6}$)sulfonylgruppe; einer Pyridinium- oder Imidazoliumgruppe, die unsubstituiert ist oder

mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind; einer Trialkyl($C_{1-6}$ )ammoniumgruppe; einer Sulfonatgruppe ausgewählt sind.

**5.** Verwendung nach einem der vorhergehenden Ansprüche, wobei X unter den Gruppen $CR_1R_2$ ausgewählt ist.

**6.** Verwendung nach einem der vorhergehenden Ansprüche, wobei die Gruppen $R_1$ und $R_2$ unter den Alkyl($C_{1-6}$) gruppen ausgewählt sind.

**7.** Verwendung nach einem der vorhergehenden Ansprüche, wobei die Gruppe $R_3$ unter einem Wasserstoffatom ausgewählt ist.

**8.** Verwendung nach einem der vorhergehenden Ansprüche, wobei W unter den Gruppen $CR_4$ ausgewählt ist.

**9.** Verwendung nach einem der vorhergehenden Ansprüche, wobei die Gruppe $R_4$ unter einem Wasserstoffatom ausgewählt ist.

**10.** Verwendung nach einem der vorhergehenden Ansprüche, wobei Y unter einem Sauerstoffatom ausgewählt ist.

**11.** Verwendung nach einem der vorhergehenden Ansprüche, wobei Z unter einer Gruppe $-C_pH_{2p}-$ ausgewählt ist, wobei p eine ganze Zahl von 2 bis 4 bedeutet, die unsubstituiert ist oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind.

**12.** Verwendung nach einem der vorhergehenden Ansprüche, wobei n 1 oder 2 ist.

**13.** Verwendung nach einem der vorhergehenden Ansprüche, wobei B einen Benzolring, einen Carbazolring oder einen Indolring bedeutet.

**14.** Verwendung nach einem der vorhergehenden Ansprüche, wobei B unsubstituiert vorliegt oder mit einem oder mehreren Substituenten substituiert ist, die unter einem Halogen, einer Alkyl($C_{1-6}$)gruppe, einer Alkoxy($C_{1-6}$)gruppe, einer Cyanogruppe, einer Trifluormethylgruppe, einer Alkyl($C_{1-6}$)carbonylgruppe, einer Trifluormethylsulfonylgruppe, einer Trifluormethylcarbonylgruppe, einer Phenylsulfonylgruppe, einer Phenylcarbonylgruppe, einer Phenylgruppe, die unsubstituiert vorliegt oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind, einer Acylaminogruppe, einer Hydroxygruppe, einer Aminogruppe, einer Dialkyl($C_{1-6}$)aminogruppe, einer Hydroxyalkyl($C_{1-6}$)aminogruppe, einer Di(hydroxyalkyl($C_{1-6}$))aminogruppe, einer (Aminoalkyl($C_{1-6}$ ))aminogruppe, einer Di(aminoalkyl($C_{1-6}$))aminogruppe, einer (Alkyl($C_{1-6}$))(hydroxyalkyl($C_{1-6}$)) aminogruppe, einer (Aminoalkyl($C_{1-6}$))(alkyl($C_{1-6}$))aminogruppe, einer (Aminoalkyl($C_{1-6}$))(hydroxyalkyl($C_{1}$-$_{6}$))aminogruppe, einer Hydroxyalkyl($C_{1-6}$)gruppe, einer Aminoalkyl($C_{1-6}$)gruppe, einer Alkyl($C_{1-6}$)aminoalkyl($C_{1-6}$)gruppe, einer Di(alkyl($C_{1-6}$))aminoalkyl($C_{1-6}$)gruppe, einer (Hydroxyalkyl($C_{1-6}$))aminoalkyl($C_{1-6}$)gruppe, einer Di(hydroxyalkyl($C_{1-6}$))aminoalkyl($C_{1-6}$)gruppe, einer Aminoalkyl($C_{1-6}$)aminoalkyl($C_{1-6}$)gruppe, einer Di(aminoalkyl($C_{1-6}$))aminoalkyl($C_{1-6}$)gruppe, einer (Alkyl($C_{1-6}$))(hydroxyalkyl($C_{1-6}$))aminoalkyl($C_{1-6}$)gruppe, einer (Alkyl($C_{1-6}$))(aminoalkyl ($C_{1-6}$))aminogruppe, einer (Hydroxyalkyl($C_{1-6}$))(alkyl($C_{1-6}$))aminoalkyl($C_{1-6}$)gruppe, einer Phenylalkyl($C_{1-6}$))-gruppe, die unsubstituiert oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind, einer kationischen Gruppe vom quartären Ammoniumtyp, einer Alkyl($C_{1-6}$)gruppe, die mit einer kationischen Gruppe vom quartären Ammoniumtyp substituiert ist, einer Carboxygruppe, einer Alkyl($C_{1}$-$_{6}$) gruppe, die mit einer Carboxygruppe substituiert ist, einer Thiogruppe, einer Thioalkyl($C_{1-6}$)gruppe, einer Sulfonatgruppe, einer Alkyl($C_{1-6}$)gruppe, die mit einer Sulfonatgruppe substituiert ist, einer Alkyl($C_{1-6}$)carbonylalkyl($C_{1-6}$) gruppe, einer Di(halogenalkyl($C_{1-6}$))-aminogruppe, einer Acetamidogruppe, einer Aryloxygruppe, einer Aryloxyalkyl ($C_{1-6}$)gruppe, einer Ethenylgruppe, einer Ethenylcarbonylgruppe, einer Gruppe $NR_6R_7$, wobei $R_6$ und $R_7$ gemeinsam mit dem Stickstoffatom, an das sie gebunden sind, einen nichtaromatischen 5- oder 6- oder 7-gliedrigen Ring bilden können, der gegebenenfalls durch ein oder mehrere Heteroatome unterbrochen sein kann, wie Stickstoff, Sauerstoff oder Schwefel, einer Alkylenkette ausgewählt sind, die ein Sauerstoffatom oder Schwefelatom enthalten kann und durch eine Cyanogruppe, Alkyl($C_{1-6}$)sulfonylgruppe oder Alkyl($C_{1-6}$)carbonylgruppe abgeschlossen werden kann; wobei zwei aneinander angrenzende Gruppen B einen aromatischen oder heteroaromatischen Ring, der unsubstituiert vorliegt oder mit einem oder mehreren Substituenten substituiert ist, die unter den Substituenten Halogen,

Hydroxy, Alkyl, Halogenalkyl, Alkoxy, Amino, Mono- oder Dialkylamino, Mono- oder Dihydroxyalkylamino, Carboxy ausgewählt sind, oder einen Ring vom Typ -O-$C_rH_{2r}$-O-, worin r eine ganze Zahl 1 oder 2 bedeutet, bilden können.

**15.** Verwendung nach Anspruch 14, wobei B unsubstituiert vorliegt oder mit einem oder mehreren Substituenten substituiert ist, die unter einer Hydroxygruppe; einer Aminogruppe, einer Di(alkyl($C_{1-6}$))aminogruppe; einer Alkyl($C_{1-6}$) gruppe; einer Acetamidogruppe; einer Pyridiniumgruppe; einer Trialkyl($C_{1-6}$)ammoniumgruppe ausgewählt sind.

**16.** Verwendung nach einem der vorhergehenden Ansprüche, wobei die Verbindung(en) der Formel (I) unter den folgenden Verbindungen ausgewählt sind: 9a-[2-[4-(Dimethylamino)phenyl]-1, 3-butadienyl]-2, 3, 9, 9a-tetrahydro-9, 9-dimethyloxazolo [3, 2-a]indol-7-carbonsäure; [9a-[2-[4-(Dimethylamino)phenyl]ethenyl]-2,3,9,9a-tetrahydro-9,9-dimethyloxazolo[3,2-a]indol-7-yl]-phosphonsäure; 4-[2-(9,9-Diethyl-2,3-dihydro-7-methoxyoxazolo[3,2-a]indol-9a (9H)-yl)ethenyl]-N,N-diethyl-benzenamin; [3-[9a-[2-[4-(Dimethylamino)phenyl]ethenyl]-2,3,9,9a-tetrahydro-9,9-dimethyloxazolo[3,2-a]indol-7-yl]propyl]-phosphonsäure; 4-[2-(2,3-Dihydro-9,9-dimethyloxazolo[3,2-a]indol-9a(9H)-yl)ethenyl]-N-methyl-N-phenyl-benzenamin; 4-[2-(2,3-Dihydro-9,9-dimethyloxazolo[3,2-a]indol-9a(9H)-yl)ethenyl]-3-ethoxy-N,N-diethyl-benzenamin; 4-[2-(2,3-Dihydro-9,9-dimethyloxazolo[3,2-a]indol-9a(9H)-yl)ethenyl]-N-ethyl-N-(2-methylpropyl)-benzenamin; 4-[2-(2,3-Dihydrooxazolo[3,2-a]indol-9a(9H)-yl)ethenyl]-N,N-dimethyl benzenamin; 4-[4-(2,3-Dihydro-7,9,9-trimethyloxazolo[3,2-a]indol-9a(9H)-yl)-1,3-butadienyl]-N,N-dimethyl-benzenamin; 4-[4-(2,3-Dihydro-9,9-dimethyl-7-nitrooxazolo[3,2-a]indol-9a(9H)-yl)-1,3-butadienyl]-N,N-dimethyl-benzenamin; 9a-[4-[4-(Dimethylamino)phenyl]-1,3-butadienyl]-2,3,9,9a-tetrahydro-9,9-dimethyl-oxazolo[3,2-a]indol-7-carbonitril; 9a-[2-[4-(Dimethylamino)phenyl]ethenyl]-2,3,9,9a-tetrahydro-9,9-dimethyloxazolo[3,2-a]indol-7-carbonitril; 4-[2-(2,3-Dihydro-9,9-dimethyloxazolo[3,2-a]indol-9a(9H)-yl)ethenyl]-N,N-dimethyl-benzenamin;9a-[2-[4-(Dimethylamino)phenyl]ethenyl]-2,3,9,9a-tetrahydro-9,9-dimethyl- -oxazolo[3,2-a]indol-7-sulfonsäuremethylester; N,N-Bis (2-chlorethyl)-4-[2-(2,3-dihydro-9,9-dimethyloxazolo[3,2-a]indol-9a(9H)-yl)ethenyl]-benzenamin;9a-[2-[4-(Dimethylamino)phenyl]ethenyl]-9,9a-dihydro-9,9-dimethyl-7-(octylsulfonyl)-oxazolo[3,2-a]indol-2(3H)-on;9a-[2-[4-(Dimethylamino)phenyl]ethenyl]-9,9a-dihydro-9,9-dimethyl-7-(phenylsulfonyl)-oxazolo[3,2-a]indol-2(3H)-on; 9a-[2-[4-(Dimethylamino) phenyl]-1-methylethenyl]-9,9a-dihydro-9,9-dimethyl-7-(methylsulfonyl)-oxazolo [3,2-a] indol-2 (3H)-on; 9a-[2-[4-(Dimethylamino) phenyl] ethenyl]- 9,9a- dihydro- 9,9- dimethyl- 7-(methylsulfonyl)-oxazolo [3,2- a] indol- 2 (3H)-on; 4-[2-[2,3-Dihydro-9,9-dimethyl-7-(methylsulfonyl)oxazolo[3,2-a]indol-9a(9H)-yl]ethenyl]- N,N-dimethyl-benzenamin; 4-[2-[9-(EthoXymethyl)-2,3-dihydro-9-methyl-7-(methylsulfonyl)oxazolo[3,2-a]indol-9a(9H)-yl]ethenyl]-N,N-dimethyl benzenamin; 4-[2-[2,3-Dihydro-2,9,9-trimethyl-7-(methylsulfonyl)oxazolo[3,2-a]indol-9a(9H)-yl] ethenyl]-N,N-dimethyl-benzenamin; 4-[2-[2,3-Dihydro-9,9-dimethyl-7-(methylsulfonyl)oxazolo[3,2-a]indol-9a (9H)-yl]-1-propenyl]-N,N-dimethyl-benzenamin; N,N-Dibutyl-4-[2-[2,3-dihydro-9,9-dirizethyl-7-(methylsulfonyl)oxazolo[3,2-a]indol-9a(9H)-yl]ethenyl]-benzenamin; 4-[2-[2,3-Dihydro-9,9-dimethyl-7-(phenylsulfonyl)oxazolo[3,2-a] indol-9a(9H)-yl]ethenyl]-N,N-dimethyl; 4-[2-[2,3-Dihydro-9,9-dimethyl-7-(octylsulfonyl)-oxazolo[3,2-a]indol-9a (9H)-yl]ethenyl]-N,N-dimethyl-benzenamin; N-[4-[2-[7-(Butylsulfonyl)-2,3-dihydro-9,9-dimethyloxazolo[3,2-a]-indol-9a(9H)-yl]ethenyl]phenyl]-acetamid; 4-[2-[7-(Butylsulfonyl)-2,3-dihydro-9,9-dimethyloxazolo[3,2-a]indol-9a (9H)-yl]ethenyl]-N,N-dimethyl-benzenamin; 4-[4-[2,3-Dihydro-9,9-dimethyl-7-(methylsulfonyl)oxazolo[3,2-a]indol-9a(9H)-yl]-1 ,3- butadienyl]- N ,N-dimethyl-benzenamin; 9a-[2-[4-(Dimethylamino)phenyl]ethenyl]-2,3,9,9a-tetrahydro-9-methyl-oxazolo[3,2-a]indol-9-ethanol; 4-[2-(9,9-Diethyl-2,3-dihydrooxazolo[3,2-a]indol-9a(9H)-yl)ethenyl]-N, N-dimethyl-benzenamin; 4-[2-[2,3-Dihydro-9-methyl-9-(2-phenoxyethyl)oxazolo[3,2-a]indol-9a(9H)-yl]ethenyl]-N, N-dimethyl-benzenamin; 4-[2-[9-(Ethoxymethyl)-2,3-dihydro-9-methyl-oxazolo[3,2-a]indol-9a(9H)-yl]ethenyl]-N,N-dimethyl-benzenamin; 4-[2-(11,11-Dimethylbenz[e]oxazolo[3,2-a]indol-10a(11H)-yl)ethenyl]-N,N-dimethyl-benzenamin; 4-[2-(7-Methoxy-9,9-dimethyloxazolo[3,2-a]indol-9a(9H)-yl)ethenyl]-N,N-dimethyl-benzenamin; 4-[2-(9,9-Dimethyloxazolo[3,2-a]indol-9a(9H)-yl)ethenyl]-N,N-dimethyl-benzenamin; 9a-[2-.[4-(Dimethylamino)-phenyl] ethenyl]-3-ethyl-9,9a-dihydro-9,9-dimethyl-oxazolö[3,2-a]-indol-2(3H)-on; 7-Chlor-9a-[2-[4-(dimethylamino)phenyl] ethenyl]-9,9a-dihydro-3,9,9-trimethyl-oxazolo[3,2-a]indol-2(3H)-on; 9a-[2-[4-(Dibutylamino)phenyl]ethenyl]-9,9a-dihydro-9,9-dimethyl-oxazolo[3,2-a]indol-2(3H)-on; 9a-[2-[4-(Dimethylamino)-phenyl]ethenyl]-9,9a-dihydro-9-(2-hydroxyethyl)-9-methyl-oxazolo[3,2-a]indol-2(3H)-on; 9a-[2-[4-(Dimethylamino)phenyl]-1-propenyl]-9,9a-dihydro-9,9-dimethyloxazolo[3,2-a]indol-2(3H)-on; 9,9a-Dihydro-7,9,9-trimethyl-9a-[2-(4-nitrophenyl)ethenyl]-oxazolo[3,2-a]indol-2(3H)-on; N-[4-[2-(2,3-Dihydro-9,9-dimethyl-2-oxooxazolo[3,2-a]indol-9a(9H)-yl)ethenyl]phenyl]-acetamid; 9a-[2-[4-(Dimethylamino)phenyl]ethenyl]-9,9a-dihydro-6-methoxy-9,9-dimethyl oxazolo[3,2-a]indol-2(3H)-on; 9a-[2-[4-(Dimethylamino) phenyl] ethenyl]-2,3,9,9a-tetrahydro-9,9-dimethyl-2-oxo-oxazolo [3,2-a] indol-7-carbonsäureethylester; 9a-[2-[4-(Dimethylamino)phenyl]ethenyl]-9,9a-dihydro-7,9,9-trimethyl-oxazolo[3,2-a]indol-2 (3H)-on; 10a-[2-[4-(Dimethylamino)-phenyl]ethenyl]-10a,11-dihydro-11,11-dimethyl-benz[e]oxazolo-[3,2-a]indol-9 (8H)-on;9,9a-Dihydro-9,9-dimethyl-9a-[2-(4-nitrophenyl)ethenyl]-oxazolo[3,2-a]indol-2(3H)-on;7-Chlor-9a-[2-[4-(dimethylamino)phenyl]ethenyl]-9,9a-dihydro-9,9-dimethyl-oxazolo[3,2-a]indol-2(3H)-on; 9a-[2-[4-(Dimethylamino) phenyl]-ethenyl]-9,9a-dihydro-9,9-dimethyl-oxazolo[3,2-a]indol-2(3H)-on; 4-[2-(7-Chlor-2,3-dihydro-9,9-dimethyloxazolo[3,2-a]indol-9a(9H)-yl)ethenyl]-N,N-dimethyl-benzenamin; 4-[2-(2,3-Dihydro-9,9-dimethyloxazolo[3,2-a]indol-9a(9H)-yl)ethenyl]-N,N-dimethyl-benzenamin; 4-[2-(7-Chlor-2,3-dihydro-2,9,9-trimethyloxazolo[3,2-a]indol-9a(9H)-yl)ethenyl]-N,N-dimethyl-benzenamin; 4-[2-(2,3-Dihydro-9,9-dimethyloxazolo[3,2-a]indol-9a(9H)-yl)-1-methyl-ethenyl]-N,N-dimethyl-benzenamin; 4-[2-(2,3-Dihydro-9,9-dimethyloxazolo[3,2-a]indol-9a(9H)-yl)ethenyl]-N,N-diethyl-benzenamin; 2,3,9,9a-Tetrahydro-7-methoxy-9,9-dimethyl-9a-[2-(4-nitrophenyl)ethenyl]-oxazolo[3,2-a]indol; 4-[2-(2,3-Dihydro-9,9-dimethyl-7-nitrooxazolo[3,2-a]indol-9a(9H)-yl)ethenyl]-N,N-dimethyl-benzenamin; N,N-Dibutyl-4-[2-(2,3-dihydro-9,9-dimethyloxazolo[3,2-a]indol-9a(9H)-yl)ethenyl]-benzenamin; 2,3,9,9a-Tetrahydro-9,9-dimethyl-7-nitro-9a-[2-(4-nitrophenyl)-ethenyl]-oxazolo[3,2-a]indol; 7-Chlor-2,3,9,9a-tetrahydro-9,9-dimethyl-9a-[2-(4-nitrophenyl)ethenyl]-oxazolo[3,2-a]indol; 4-[2-(2,3-Dihydro-7-iod-9,9-dimethyloxazolo[3,2-a]indol-9a(9H)-yl)ethenyl]-N,N-dimethyl-benzenamin; 4-[2-(2,3-Dihydro-5-methoxy-9,9-dimethyloxazolo[3,2-a]indol-9a(9H)-yl)ethenyl]-N,N-dimethyl-benzenamin; 4-[2-(2,3-Dihydro-7-methoxy-9,9-dimethyloxazolo[3,2-a]indol-9a(9H)-yl)ethenyl]-N,N-dimethyl-benzenamin; 4-[4-(2,3-Dihydro-9,9-dimethyloxazolo[3,2-a]indol-9a(9H)-yl)-1,3-butadienyl]-N,N-diethyl-benzenamin; 4-[4-(7-Chlor-2,3-dihydro-9,9-dimethyloxazolo[3,2-a]indol-9a(9H)-yl)-1,3-butadienyl]-N,N-dimethyl-benzenamin; 4-[4-(2,3-Dihydro-9,9-dimethyloxazolo[3,2-a]indol-9a(9H)-yl)-1,3-butadienyl]-N,N-dimethyl-benzenamin; 4-[4-(2,3-Dihydro-7-methoxy-9,9-dimethyl-oxazolo[3,2-a]indol-9a(9H)-yl)-1,3-butadienyl]-N,N-dimethyl-benzenamin; 2,3,9,9a-Tetrahydro-7,9,9-trimethyl-9a-[2-(4-nitrophenyl)ethenyl]-oxazolo[3,2-a]indol; N-[4-[2-(2,3-Dihydro-9,9-dimethyloxazolo[3,2-a]indol-9a(9H)-yl)ethenyl]phenyl]-acetamid; 4-[2-(2,3-Dihydro-6-methoxy-9,9-dimethyloxazolo[3,2-a]indol-9a(9H)-yl)ethenyl]-N,N-dimethyl-benzenamin; 4-[2-(8,9-Dihydro-11,11-dimethylbenz[e]oxazolo[3,2-a]indol-10a(11 H)-yl)ethenyl]-N,N-dimethyl-benzenamin; 4-[2-(2,3-Dihydro-7,9,9-trimethyl-oxazolo[3,2-a]indol-9a(9H)-yl)ethenyl]-N,N-dimethyl-benzenamin; 2,3,9,9a-Tetrahydro-9,9-dimethyl-9a-[2-(4-nitrophenyl)ethenyl]-oxazolo[3,2-a]indol; 9,9a-Dihydro-9,9-dimethyl-9a-[2-(9-methyl-9H-carbazol-3-yl)ethenyl]-7-(methylsulfonyl)-oxazolo[3,2-a]indol-2(3H)-on; 9a-[2-(9-Hexyl-9H-carbazol-3-yl)ethenyl]-2,3,9,9a-tetrahydro-9,9-dimethyl-7-(phenylsulfonyl)-oxazolo[3,2-a]indol; 2,3,9,9a-Tetrahydro-9,9-dimethyl-7-(methylsulfonyl)-9a-[2-(9-octyl-9H-carbazol-3-yl)ethenyl]-oxazolo[3,2-a]indol; 9a-[2-(9-Butyl-6-methoxy-9H-carbazol-3-yl)ethenyl]-2,3,9,9a-tetrahydro-9,9-dimethyl-oxazolo[3,2-a]indol-7-carbonsäureethylester; 9a-[2-(9-Ethyl-9H-carbazol-3-yl)ethenyl]-2,3,9,9a-tetrahydro-9,9-dimethyl-oxazolo[3,2-a]indol-7-sulfonsäuremethylester; 3-Chlor-6-[2-[2,3-dihydro-9,9-dimethyl-7-(methylsulfonyl)oxazolo[3,2-a]indol-9a(9H)-yl]ethenyl]-9-octyl-9H-carbazol; 3-[2-[2,3-Dihydro-9,9-dimethyl-7-(methylsulfonyl)oxazolo[3,2-a]indol-9a(9H)-yl]ethenyl]-9-methyl-9H-carbazol; 3-[2-[9-(2-Ethoxyethyl)-2,3-dihydro-9-methyl-7-(methylsulfonyl)oxazolo[3,2-a]indol-9a(9H)-yl]ethenyl]-9H-carbazol; 9a-[2-(9-Hexyl-9H-carbazol-3-yl)ethenyl]-2,3,9,9a-tetrahydro-9-(2-hydroxyethyl)-9-methyl-oxazolo[3,2-a]indol-7-carbonsäureethylester; 2,3,9,9a-Tetrahydro-9,9-dimethyl-9a-[2-(9-octyl-9H-carbazol-3-yl)ethenyl]-oxazolo[3,2-a]indol-7-carbonsäureethylester; 9a-[2-(9-Butyl-9H-carbazol-3-yl)ethenyl]-2,3,9,9a-tetrahydro-9,9-dimethyl-oxazolo[3,2-a]indol-7-carbonsäureethylester; 2,3,9,9a-Tetrahydro-9,9-dimethyl-9a-[2-(9-methyl-9H-carbazol-3-yl)ethenyl]-oxazolo[3,2-a]indol-7-carbonsäureethylester; 9a-[2-(9-Butyl-6-ethoxy-9H-carbazol-3-yl)ethenyl]-2,3,9,9a-tetrahydro-N,N,9,9-tetramethyl-oxazolo[3,2-a]indol-7-amin; 2,3,9,9a-Tetrahydro-9-methyl-9a-[2-(9-methyl-9H-carbazol-3-yl)ethenyl]-oxazolo[3,2-a]indol-9-ethanol; 9a-[2-(9-Butyl-9H-carbazol-3-yl)ethenyl]-2,3,9,9a-tetrahydro-N,N,9,9-tetramethyl-oxazolo[3,2-a]indol-7-amin; 3-[2-(2,3-Dihydro-6-methoxy-9,9-dimethyloxazolo[3,2-a]indol-9a(9H)-yl)ethenyl]-9-methyl-9H-carbazol; 3-[2-(2,3-Dihydro-9,9-dimethyloxazolo[3,2-a]-indol-9a(9H)-yl)-1-propenyl]-9-methyl-9H-carbazol; 3-[2-(7-Chlor-2,3-dihydro-9,9-dimethyloxazolo[3,2-a]indol-9a(9H)-yl)ethenyl]-9-methyl-9H-carbazol; 3-Brom-6-[2-(2,3-dihydro-9,9-dimethyloxazolo[3,2-a]indol-9a(9H)-yl)ethenyl]-9-ethyl-9H-carbazol; 3-[2-(2,3-Dihydro-9,9-dimethyloxazolo[3,2-a]indol-9a(9H)-yl)ethenyl]-9-methyl-9H-carbazol.

**17.** Verwendung nach Anspruch 16, wobei die Verbindung(en) der Formel (I) unter den in der folgenden Tabelle dargestellten Verbindungen ausgewählt sind:

| | |
|---|---|
| 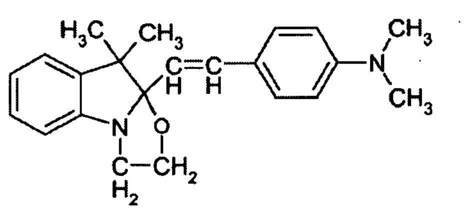 | Farbstoff 1 |

(fortgesetzt)

| | |
|---|---|
| 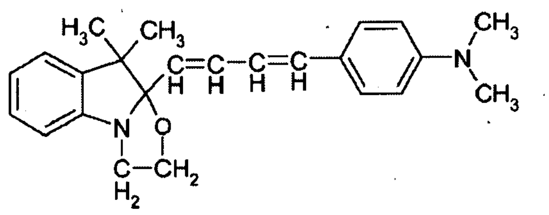 | Farbstoff 2 |
| 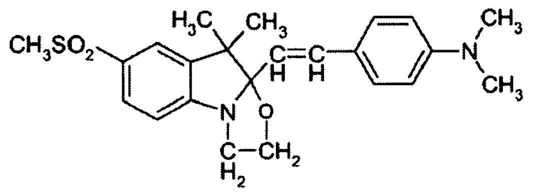 | Farbstoff 3 |
| 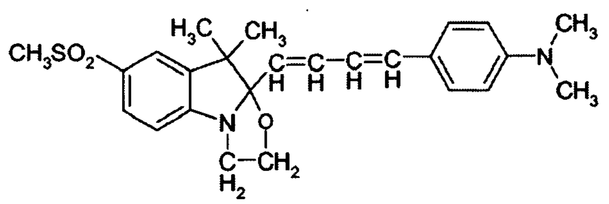 | Farbstoff 4 |
| 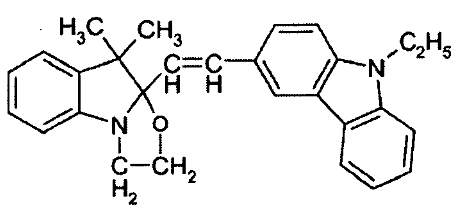 | Farbstoff 5 |
| 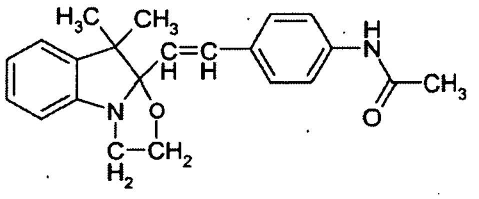 | Farbstoff 6 |

(fortgesetzt)

| | |
|---|---|
| 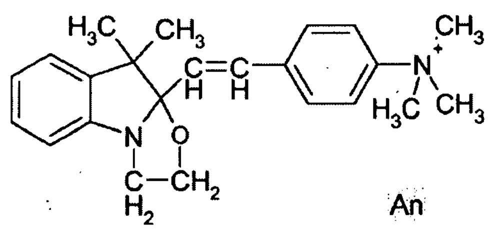 | Farbstoff 7 |
| 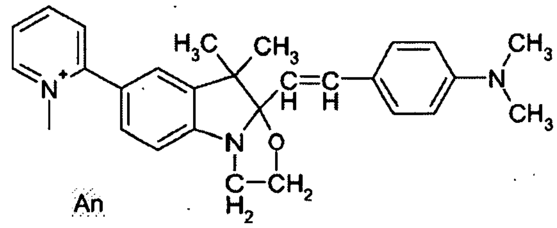 | Farbstoff 8 |
| 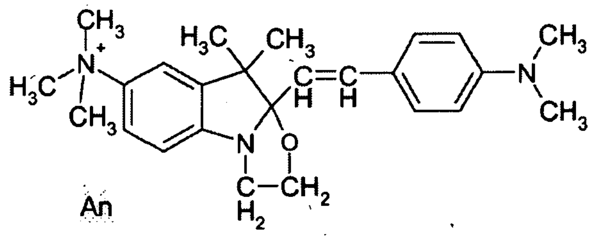 | Farbstoff 9 |
| 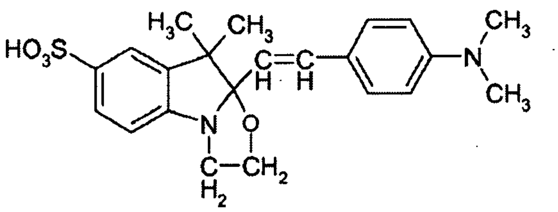 | Farbstoff 10 |
| 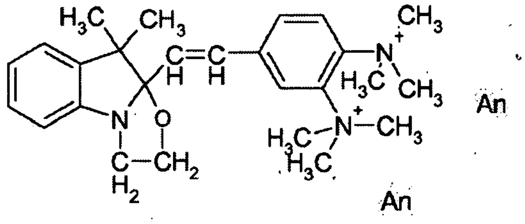 | Farbstoff 11 |

worin die Symbole An, die gleich oder verschieden sind, ein negatives Gegenion bedeuten.

**18.** Verwendung nach einem der vorhergehenden Ansprüche, wobei die Verbindung(en), die unter den Verbindungen der Formel (I), den Verbindungen der Formel (II) und ihren Additionssalzen ausgewählt sind, 0,0001 bis 10 Gew.-%, bezogen auf das Gesamtgewicht der Zusammensetzung, ausmachen.

**19.** Verwendung nach einem der vorhergehenden Ansprüche, wobei die Farbmittelzusammensetzung mindestens ein Ansäuerungsmittel und/oder mindestens ein Alkalisierungsmittel enthält.

**20.** Verwendung nach Anspruch 19, wobei das oder die Ansäuerungsmittel unter den anorganischen Säuren und organischen Säuren ausgewählt sind.

**21.** Verwendung nach Anspruch 18 oder 19, wobei das oder die das Alkalisierungsmittel ausgewählt ist unter:

- basischen Aminosäuren;
- Carbonaten oder Bicarbonaten von Alkalimetallen oder Erdalkalimetallen;
- Silicaten oder Metasilicaten;
- Verbindungen der folgenden Formel (III):

$$X(OH)_n \qquad (III)$$

worin bedeuten:

· X ein Kaliumion, Lithiumion, Natriumion oder Ammoniumion $N^+R_8R_9R_{10}R_{11}$, wobei die Gruppen $R_8$, $R_9$, $R_{10}$ und $R_{11}$, die gleich oder verschieden sind, eine $C_{2-4}$-Alkylgruppe bedeuten, wenn n 1 ist;
· X ein Magnesiumion oder Calciumion, wenn n 2 ist;

- Verbindungen der folgenden Formel (IV):

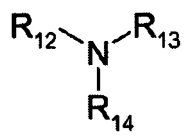

(IV)

worin bedeuten:

· $R_{12}$ ein Wasserstoffatom; eine Alkyl($C_{1-6}$)gruppe; eine Monohydroxyalkyl($C_{1-6}$)gruppe; eine Polyhydroxyalkyl($C_{2-6}$-gruppe, · $R_{13}$ und $R_{14}$, die gleich oder verschieden sind, ein Wasserstoffatom; eine Alkyl($C_{1-6}$) gruppe; eine Monohydroxyalkyl($C_{1-6}$)-gruppe; eine Polyhydroxyalkyl($C_{2-6}$)gruppe;

- Verbindungen der folgenden Formel (V):

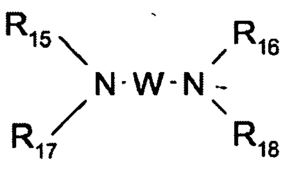

(V)

worin bedeuten:

· W einen Propylenrest, der gegebenenfalls mit einer Hydroxygruppe oder einer Alkyl($C_{1-4}$)gruppe substi-

tuiert ist;

· $R_{15}$, $R_{16}$, $R_{17}$ und $R_{18}$, die gleich oder voneinander verschieden sind, ein Wasserstoffatom; eine Alkyl ($C_{1-4}$)gruppe; eine Hydroxyalkyl($C_{1-4}$)gruppe.

**22.** Verwendung nach einem der vorhergehenden Ansprüche, wobei das zum Färben geeignete Medium aus Wasser oder mindestens einem organischen Lösungsmittel oder einem Gemisch aus Wasser und mindestens einem organischen Lösungsmittel besteht.

**23.** Verwendung nach Anspruch 22, wobei das oder die Lösungsmittel unter den Ketonen; linearen oder verzweigten, Monoalkoholen oder Diolen, die 2 bis 10 Kohlenstoffatomen enthalten; aromatischen Alkoholen; Polyolen oder Polyolethern; sowie Diethylenglycolalkylethern ausgewählt sind.

**24.** Verwendung nach Anspruch 22 oder 23, wobei das zum Färben geeignete Medium aus Wasser oder einem Gemisch aus Wasser und mindestens einem organischen Lösungsmittel besteht und der pH-Wert im Bereich von 2 bis 12 liegt.

**25.** Verwendung nach einem der vorhergehenden Ansprüche, wobei die Farbmittelzusammensetzung mindestens einen Zusatzstoff enthält, der unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln, Antioxidantien, Penetratiorismitteln, Maskierungsmitteln, Parfums, Puffern, Dispergiermitteln, Konditioniermitteln, Konservierungsmitteln und Trübungsmitteln ausgewählt ist.

**26.** Verfahren zur Behandlung von Keratinfasern, bei dem eine Farbmittelzusammensetzung, wie sie in einem der Ansprüche 1 bis 25 definiert ist, während einer ausreichenden Einwirkzeit auf die Keratinfasern aufgebracht wird, wobei nach der Anwendung gegebenenfalls gespült wird.

**27.** Verfahren nach Anspruch 26, wobei bei der Anwendung der Farbmittelzusammensetzung eine Vor- oder Nachbehandlung der Keratinfasern durchgeführt wird, die die Bildung der Färbung ermöglicht.

**28.** Verfahren nach Anspruch 27, wobei die Vor- oder Nachbehandlung darin besteht, die Fasern durch Einwirkung von Licht, mit elektrischem Strom, Wärme, einem Ansäuerungsmittel, wie es in Anspruch 19 oder 20 definiert ist, und/oder einem Alkalisierungsmittel, wie es in Anspruch 19 oder 21 definiert ist, und/oder einem Lösungsmittel, wie es in Anspruch 22 oder 23 definiert ist, elektromagnetischer Strahlung zu behandeln.

**29.** Verfahren nach einem der Ansprüche 26 bis 28, bei dem die Farbmittelzusaminensetzung in Gegenwart mindestens eines Oxidationsmittel.aufgebracht wird.

**30.** Verfahren nach Anspruch 29, wobei das Oxidationsmittel unter Wasserstoffperoxid, Harnstoffperoxid, Alkalimetallbromaten, Salzen von Persäuren, Persäuren und Oxidasen ausgewählt ist.

**31.** Vorrichtung mit mehreren Abteilungen, die in einer ersten Abteilung eine Farbmittelzusammensetzung, die mindestens eine Verbindung enthält, wie sie in einem der Ansprüche 1 bis 17 definiert ist, und in einer zweiten Abteilung mindestens ein Ansäuerungsmittel, wie es in Anspruch 19 oder 20 definiert ist, und/oder ein Alkalisierungsmittel, wie es in Anspruch 19 oder 21 definiert ist, und/oder ein Lösungsmittel, wie es in Anspruch 22 oder 23 definiert ist, enthält.

**32.** Vorrichtung nach Anspruch 31, die in einer dritten Abteilung mindestens ein Oxidationsmittel enthält, wie es in Anspruch 29 oder 30 definiert ist.

**33.** Verbindungen, wie sie in einem der Ansprüche 1 bis 17 definiert sind, die mindestens eine Gruppe aufweisen, die unter kationischen Gruppen vom quartären Ammoniumtyp ausgewählt sind.

**34.** Verbindungen nach Anspruch 33, bei denen die kationische(n) Gruppe(n) vom quartären Ammoniumtyp unter den Gruppen Trialkyl($C_{1-6}$)ammonium, Oxazolium, Thiazolium, Imidazolium, Pyrazolium, Pyridinium, Pyrrolium, Triazolium, Isooxazolium, Isothiazolium, Pyrimidinium, Pyrazinium, Triazinium, Pyridazinium, Indolium, Chinolinium oder Isochinolinium ausgewählt ist, die unsubstituiert vorliegen oder substituiert sind.

**35.** Farbmittelzusammensetzung, die in einem zum Färben geeigneten Medium mindestens eine Verbindung enthält,

wie sie in Anspruch 33 oder 34 definiert ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 56150006 A
- JP 56081522 A
- JP 56025106 A
- JP 55113710 A
- JP 55031057 A
- FR 2285439
- US 4380629 A


### Littérature non-brevet citée dans la description


- **J.H. WEIL.** *biochimie générale,* 1983, 5
- **LUBERT STRYER.** *BIOCHIMIE GENERALE,* 1995, 22
- **A. LEHNINGER.** *BIOCHIMIE GENERALE,* 1993, 115-116
- **DE BOECK-WESMAEL.** *BIOCHIMIE GENERALE,* 1994, 57-59